(19) **Europäisches Patentamt / European Patent Office / Office européen des brevets**

(11) **EP 2 918 222 A1**

(12) # EUROPEAN PATENT APPLICATION
published in accordance with Art. 153(4) EPC

(43) Date of publication:
**16.09.2015 Bulletin 2015/38**

(51) Int Cl.:
*A61B 5/00* (2006.01)   *A61B 19/00* (2006.01)
*G06F 19/00* (2011.01)

(21) Application number: **14862597.3**

(22) Date of filing: **18.07.2014**

(86) International application number:
**PCT/CN2014/082449**

(87) International publication number:
**WO 2015/070634 (21.05.2015 Gazette 2015/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.11.2013   CN 201310566688**

(71) Applicant: **Wu, Yibing**
**Haidian District, Beijing 100044 (CN)**

(72) Inventor: **Wu, Yibing**
**Haidian District, Beijing 100044 (CN)**

(74) Representative: **Tischner, Oliver**
**Lavoix**
**Bayerstrasse 83**
**80335 München (DE)**

(54) **LIFE MAINTENANCE MODE, BRAIN INHIBITION METHOD AND PERSONAL HEALTH INFORMATION PLATFORM**

(57)   The present invention provides a novel life maintenance mode named as the third type of life maintenance mode, comprising the steps of decomposing inspection items of clinical diagnosis which is usually carried out after a disease has occurred into a series of individual automatically measured and computed indicators of life condition data, which is popularized into people's daily life and work to be practiced continuously so as to produce continuous diagnosis and inspection analysis report and trend; performing active control of the change of the life condition data by means of senior independent consciousness of cerebral cortex with the automatically measured and computed life condition data as a tool to serve as a quantitative traction means for treatment, rehabilitation, and health and longevity; predict and prevent the occurrence and development of diseases on the basis of the produced inspection analysis report and trend; automatically sending messages to remind a patient at home to check the treatment behaviors on the basis of doctor's advices and prescriptions in the track record of medical treatment, and providing an automatic no-response alarming service.

**FIG. 1**

## Description

### Technical Field

The present invention relates to data analysis and health management in the medical field, in particular, relates to a life maintenance mode, a brain inhibition therapy and a personal health information platform.

### Background of the Invention

A human body comprises multiple organs, and the functioning conditions of these organs are closely related to the physical conditions of a person. The brain is an important organ of a human body, the senior consciousness of cerebral cortex has the ability of learning, and meanwhile also has the ability of forgetting and the tendency of being lazy. The emotional function of senior consciousness of cerebral cortex is a distinction between humans and animals, and is acquired through long term evolution. The functioning of cerebral cortex consumes life energy, and is a factor which causes the decrease of human longevity. A correct way of life and using brain properly are of vital importance to human health and longevity. If the senior consciousness of cerebral cortex can be changed into having positive promotion effect on health and longevity, human longevity may then be increased to a natural life period of 100-150 years. Blood stream distribution and breath are factors determining oxygen and energy obtained by a life entity, and they are all influenced or even indirectly controlled by the senior consciousness of cerebral cortex. Some diseases in life process, such as sleep, are directly controlled by the brain. All human beings are able to sleep after birth, especially during childhood when a human being is able to sleep whenever one feels sleepy. The reason for insomnia and symptoms of anxiety and depression is mainly due to stimulation from the outside world where a human being is required to sacrifice sleep in order to complete some cognitive tasks, and may be accompanied by positive or negative emotional processes. If accompanied by negative emotion, the brains of some people forget how to sleep, which can be a very short transition process of several days to finally form the consequence of insomnia. Drug addiction is another example. It is a process of basic instinct for the brain to secrete enkephalin, but due to intake of opium substance from outside, the brain forgets its ability of secreting enkephalin and at the same time pleasant emotion is induced, which causes the brain to learn the habit of requiring constant supply of such substance from outside, so as to give rise to drug addiction. The drug rehabilitation process is a reverse process, through painful process of negative emotion, to inhibit the brain's pleasant memories of substance from outside, so as to allow the brain to once again learn how to secrete enkephalin. Thus it can be seen that it is possible to let the brain once again learn how to sleep, or to learn some already-forgotten ability with the pleasant emotion, or even learn the new ability of keeping healthy and prolonging life.

It can be seen that, the brain condition plays a vital role in the health condition of a human body, therefore how to acquire information of the brain condition is of vital importance to subsequent brain research and medical healthcare. Chinese Patent Application Publication CN102783936A discloses a system for determining brain condition, including an input device for providing data describing the brain structure, and a processor communicably connected to the input device. The processor is configured for measuring at least one geometric characteristic of the brain structure by reference to the data, determining a brain index value by reference to at least one geometric characteristic, and comparing the brain index value against at least one predetermined index value whereby, based on a result of the comparison, a brain condition corresponding to the brain index value is able to be determined. In this technical solution, the indicators representing a brain index is too simple to accurately describe a brain condition, and thus is incapable of providing basis for health evaluation and medical service.

Furthermore, besides the brain, some condition information of other parts of a human body may also represent health condition of the human body, and if this information can be quantitatively acquired, the pertinence of health evaluation and medical service can then be greatly enhanced.

### Summary of the Invention

An objective of the present invention is to solve the problem that indicators of methods for inspecting human body condition in prior art are too simple to provide basis for health evaluation and medical service, thus the present invention provides a method for inspecting human body signal which combines multiple indicators to represent brain condition and body condition.

In order to solve the above-mentioned technical problem, the present invention provides a life maintenance mode, comprising the steps of

automatically measuring and computing life condition data, and classifying inspection items into quantitatively measured life condition data converted from individual tables;

performing active control of the change of the life condition data by means of senior independent consciousness of cerebral cortex in combination with brain inhibition therapy to serve as a quantitative traction means for treatment, rehabilitation, and health and longevity;

composing a report of inspection analysis and health trend prediction by analyzing and computing the life condition data, so as to predict and prevent the occurrence and development of diseases; automatically sending messages to remind a patient at home to check the treatment behaviors, based on doctor's advices and prescriptions in the track record of medical treatment, and providing an automatic no-response alarming service.

Preferably, the process of automatically measuring and computing life condition data comprises the following steps of

converting signal of vital signs and physiological data into digital signal,

transmitting the signal to the filtering module, noise control module and input amplification module of the monitoring circuit, and after being computed and processed by corresponding monitoring modules, the signal is transmitted to an analog-digital convertor circuit of the central computation and control management circuitry through an analog signal input port;

sending the processed signal to a communication interface of the central computation and control management circuitry through an RS232 interface circuit;

encrypting and compressing the digitalized signal data received by the central computation and control management circuitry to obtain a processed data stream, which is then fed into a dynamic data link cache queue;

the dynamic data link cache queue is a varying data storage and output configuration;

data configuration in a storage region is determined by network condition;

after an interruption event is triggered due to that the central computation and control management circuitry is notified of the change of network condition, the central computation and control management circuitry controls various permutations and combinations of the obtained data;

the data is then written into a data buffer of the storage queue upon a write instruction;

the data in the queue is outputted to a wireless internet access control circuit through a data port upon a read instruction of the central computation and control management circuitry;

the internet access control circuit carries out auto-dial to the network, network condition recognition, TCP/IP mode signal modulation, sub-package and outputting, so as to allow the data to be uploaded to the data storage server and data computation server.

Preferably, the process of automatically measuring and computing life condition data further comprises the following steps of

acquiring living condition data and subjective feeling data through a subjective feeling information collector which includes a computerized equipment, such as a cell phone, a mobile tablet, a stationary computer, with a browser window of B/S configuration or an operation window of APP software as the input interface, logging the data by manual input, and selecting data items related to different feelings or parameters by click-choice on the input interface;

uploading the data to the data storage and computation server of the personal health bank account directly through wireless or wired internet access of the computerized equipment.

Preferably, in the process of automatically measuring and computing life condition data, the acquired signal includes:

(1) vital signs and physiological data, including:

waveform signal: electroencephalographic wave, cerebral and peripheral blood perfusion wave, penis blood perfusion wave, electrocardiographic wave, pulse oximetry wave, mouth-nose-chest-abdominal respiration wave, electromyographic wave, eye movement wave, breath-end carbon-dioxide concentration wave;

numerical signal: pulse rate, non-invasive blood pressure, blood glucose, pulse oxygen saturation, breath-end carbon-dioxide concentration, inhaled oxygen concentration, body temperature, respiratory rate, penis cross-section diameter, penis hardness, penis temperature, penis arterial oxygen saturation, urine volume, relative exercise amount of limbs, body weight, exercise sweat amount;

(2) acquired living condition data: medicine dosage, food intake, water intake, defecation amount;

(3) acquired subjective feeling data: pain rating scale, emesis rating scale, dizziness rating scale, living quality rating scale, chest distress, urination frequency, urine color, hemorrhage, diarrhea, constipation, excrement color, palpitation, shortness of breath, impotence, vertigo, asthma, pyrexia, dysphoria, anger, anxiety rating scale, depression rating scale, difficulty of falling asleep, dysphylaxia, dreaminess.

Furthermore, there is provided a brain inhibition therapy, comprising the steps of

acquiring a real-time blood perfusion index of the supraorbital artery as a feedback parameter, performing automatic feedback control over infusion of venous sedative with an infusion pump under sedative dosage,

letting the oxygen metabolic requirement of senior cognition function of cerebral cortex decrease to the lowest level,

controlling inhibition of the senior cognition function of the cerebral cortex.

Preferably, the step of performing automatic feedback control over the infusion of venous sedative with an infusion pump under sedative dosage further comprises the following sub-steps of

performing closed-loop feedback control over the infusion of the venous sedative with the infusion pump by using characteristic indicators of the brain blood perfusion index, wherein, the feedback control mechanism is a steady-state regulation system which controls the brain blood perfusion index to reach the minimum;

performing closed-loop feedback control over the infusion of the venous sedative with the infusion pump by using the characteristic indicator of brain blood perfusion index of the supraorbital artery as a feedback parameter, to achieve the control target that the brain blood perfusion index of the supraorbital artery reaches the minimum;

acquiring the brain blood perfusion signal of the supraorbital artery by using signal acquisition and computation technology of blood perfusion of the supraorbital artery, through a wireless mobile terminal for acquisition and transmission of vital signs, transmitting the signal to a processing computer through WIFI, allowing the computer to automatically compute and process the signal to obtain quantitative data of the real-time blood perfusion index, and then sending a control instruction to a computer interface of the infusion pump through a standard RS232 communication interface, so as to control the automatic infusion of sedative with the infusion pump, thereby achieving the purpose of inhibition of cerebral cortex cognition function.

Preferably, Target Controlled Infusion (TCI) is adopted as the control mechanism, with the blood drug concentration as a controlled object and the blood perfusion index as a feedback regulation parameter, the blood perfusion of the supraorbital artery is adjusted to the minimum by increasing or reducing the blood drug concentration, so as to quantitatively control the automatic sleep sedation therapeutic treatment.

Furthermore, there is provided an encephalic signal acquisition method for the brain inhibition therapy, comprising the steps of

acquiring electroencephalographic wave on both sides of the forehead, supraorbital artery blood perfusion wave on both sides of the forehead, and peripheral blood perfusion wave;

letting the electroencephalographic wave, the supraorbital artery blood perfusion wave and the peripheral blood perfusion wave be transmitted to a front electrical signal amplifying circuit through a transducer, and then transmitted to an analog-digital convertor circuit to be converted into a group of discretized computer data set after being integrated, filtered and amplified by an analog circuit;

computing a brain blood perfusion index based on the supraorbital artery blood perfusion wave on both sides of the forehead;

computing a peripheral blood perfusion index based on the peripheral blood perfusion wave; processing the electroencephalographic wave to obtain characteristic indicators of the brain condition.

Preferably, the encephalic signal acquisition method further comprising the step of computing vascular elasticity based on electrocardiographic wave and the blood perfusion signal and also computing a brain power spectrum based on the electroencephalographic wave and the characteristic indicators of the brain condition.

Preferably, the characteristic indicators of the brain condition includes one or more of brain relaxing index, brain concentrating index, brain activity index, brain thinking index, brain restraining index, left-right brain lateralization index, brain stability index and brain sedation index.

Preferably, the encephalic signal acquisition method further comprising the step of establishing a brain condition scatter point diagram based on the characteristic indicators of the brain condition, so as to obtain the change of the brain condition.

Preferably, supraorbital artery blood perfusion wave on both sides of the forehead is acquired with a pressure sensor, a laser sensor and an electroencephalogram sensor on the forehead. Furthermore, there is provided an device with a signal transducer of electroencephalographic wave and forehead blood perfusion wave and an independent transducer of peripheral blood perfusion wave integrated therein, comprising

an integrated signal transducer of double-conduction electroencephalographic wave and double-conduction supraorbital artery blood perfusion wave, and an independent four-conduction transducer unit of peripheral blood perfusion wave, wherein, due to change of erythrocytes carried by blood stream of a blood vessel, the blood stream exerts a force onto the blood vessel wall so as to form a pulse;

a near-infrared laser emitter of the sensor illuminates the blood vessel to produce a reflective wave, and the reflective wave signal is received by a laser receiver on the lateral side and used as a first output signal; meanwhile, the pulse wave of the blood vessel wall is detected by a pressure transducer at the blood vessel and used as a second output signal; the first output signal and the second output signal are processed by a control computer chip to obtain a blood perfusion wave;

dry electrode metal pieces are used in the electroencephalogram sensor as medium to be affixed onto the forehead, so as to conduct cerebral electrical signal as a third output signal.

Preferably, the integrated transducer is fixed on a flexible silica gel, the size of which is within $12 \times 1.4 \times 0.4$cm and which is elastic, strip shaped, and able to be formed into an annular shape and affixed by adhesive tape of a medical band-aid; the peripheral blood perfusion transducers detect blood perfusion signal and are fixed on a flexible silica gel, the size of which is within $3 \times 1 \times 0.3$cm.

Furthermore, there is provided an operation method of the above-mentioned device, wherein,

the output signal of the transducer is converted into recognizable analog electrical signal by an electrical signal conversion and amplification unit which comprises an electroencephalographic signal acquisition and amplification module and a blood vessel blood perfusion wave signal acquisition and amplification module, the tiny signal acquired by the transducer is amplified and filtered to eliminate interference and void parts, transmitted to an analog-digital convertor, discretized and converted into digital signal, and then allocated, computed, encrypted and compressed by the control computer chip; the processed data stream is fed into a dynamic data link cache queue, the dynamic data link cache queue is a varying data storage and output configuration, and the data configuration in a storage region is determined by network condition; after an interruption event is triggered due to that the central computation and control management circuitry is notified of the change of network condition, the central computation and control management circuitry controls various permutations and combinations of the obtained data;

the data is then written into the storage queue upon a write instruction; the data in the queue is outputted to a wireless internet access circuit through a data port upon a read instruction of the control computer chip; the internet access circuit carries out auto-dial to the network, network condition recognition, TCP mode signal modulation, sub-package and outputting.

Furthermore, there is provided a sleep analysis method, comprising the steps of

extracting physiological signal indicating change of sleep quality which includes a brain blood perfusion index and a peripheral blood perfusion index, by using nerve electrophysiological measuring method in conjunction with brain blood perfusion measuring method, so as to establish a vital sign expressing means and characteristic indicators for quantitatively expressing and indicating condition change of sleep process, sleep structure and sleep quality;

objectively describing sleep quality by using the brain blood perfusion index and the peripheral blood perfusion index as a major physiological signal basis for sleep measurement, assisted by electroencephalographic signal, so as to reveal the relative change of brain blood perfusion and peripheral blood perfusion during sleep, and indicate the quantitative physiological change process of sleep influencing disease treatment and health and longevity;

automatically extracting electroencephalographic characteristic indicators which quantitatively describe change of sleep structure and brain blood and peripheral blood perfusion distribution characteristic indicators which quantitatively reflect changes of sleep quality and health condition, by real-time computation of the acquired multi-conduction electroencephalographic wave and multi-conduction blood perfusion wave;

acquiring the multi-conduction electroencephalographic waves and multi-conduction blood perfusion waves of a human in various conditions by using transducers for acquiring the signal of electroencephalographic wave, brain blood and peripheral blood perfusion wave, and computing and processing the data at real-time through a computer system which receives the same, so as to obtain real-time data of quantitative characteristic indicators;

performing real-time data computation with data pre-processing algorithms including waveform recognition algorithm, fuzzy control algorithm, spectrum analysis algorithm, wavelet analysis algorithm, multiple regression algorithm, and calculus algorithm;

computing frequency domain power and phase change of the electroencephalographic wave signal under a specific time domain window, and computing multi-variant components of multi-scale wavelet decomposition scale-space under a specific generating function with spectrum analysis algorithm and wavelet analysis algorithm of the electroencephalographic wave data with time window weighting, obtaining converted and inverse-converted cluster of data with inverse transformation algorithm of time domain component reconstruction, and obtaining a cluster of wavelet discrete values after processing the converted and inverse-converted cluster of data with fuzzy algorithm and threshold value extraction algorithm;

obtaining a series of quantitative electroencephalographic characteristic indicators representing various sleep conditions, after multiple regression algorithm incorporating sleep condition and weighting indexation;

obtaining the waveform change-point of multiple-cluster time domain blood perfusion waveforms of the multi-conduction blood perfusion wave with multi-scale wavelet analysis algorithm of the blood perfusion wave data under a specific generating function, and then obtaining the dynamic time domain power and relative speed of the blood perfusion wave with calculus algorithm and fitting computation, so as to produce quantitative characteristic indicators describing brain blood and peripheral blood perfusion signal for various sleep quality and sleep structure during sleep.

Furthermore, there is provided a personal health information platform, comprising:

obtaining vital signs of a human body, and acquiring medical health data;

decomposing medical clinical inspection items into individual standardized quantitative measurements for life condition, and self-organizing, analyzing and computing life condition information, so as to compose various inspection reports of disease physiological and vital sign data and health prediction reports;

establishing a system for real-time quantitative multi-index data extraction for brain condition under the mode of Internet of Things, so as to establish an integrated extracting and expressing method of quantitative characteristic indicators of nerve electrophysiological and blood perfusion signal related to human natural sleep structure and quality;

defining a brain blood perfusion unit and a peripheral blood perfusion unit through measurement of blood perfusion distribution, and establishing a training routine and method to respectively reduce metabolic requirement of the two units upon the relationship between blood flow and metabolism, so as to allow the organs to be able to withstand low blood perfusion level under disease condition;

establishing a new life inspection and maintenance mode as a third life maintenance mode for disease treatment, disease rehabilitation, disease prevention and health keeping and life prolonging, so as to control the change of vital sign data upon independent consciousness, in order to achieve the goal of keeping healthy and prolonging life;

introducing continuous quantitative precise measuring and computing of vital sign data and medical health data in the processes of disease treatment, disease rehabilitation, disease prevention and health keeping and life prolonging, changing negative emotion of a person under disease condition into positive emotion, and continuously perceiving the real-time change of outputted quantitative characteristic indicators of vital signs and physiological data, so as to enhance the disease treatment effect and disease rehabilitation effect and achieve the goal of disease prevention and life prolonging, wherein, the data and technique produced during the processes of disease treatment, rehabilitation, disease prevention and health keeping and life prolonging are used as a tool, and based on wireless network communication, the system of aiding or directly driving the processes of disease treatment, rehabilitation, disease prevention and health keeping and life prolonging for people is established.

Preferably, the personal health information platform further comprising establishing a media broadcasting system for demonstration pioneers of health keeping, life prolonging, disease treatment and disease rehabilitation, broadcasting treatment track record and vital sign data of the pioneers at regular time, so as to provide a demonstration example for quantitative health data comparison, learning and imitation of the processes of disease treatment, disease rehabilitation and health keeping and life prolonging for people through real-time media transmission.

Preferably, the platform comprises a system software platform and hardware devices including a wireless mobile terminal for vital sign data acquisition and transmission, a data computation service center server and a data storage center server, a mobile computer terminal or a stationary computer, and a mobile communication terminal.

Preferably, the platform adopts an internet communication platform as a communication transmission media, and the users are allowed to register their own application accounts with the health bank website and bind the accounts with their personal identification information. Preferably, on the platform, a wireless mobile terminal for vital sign data acquisition and transmission is used by each user in living and working environment, each terminal is bound with the application account of the user, informations of vital signs, subjective feeling and medicine intake events of the user are automatically acquired in daily usage through the wireless mobile terminal for vital sign data acquisition and transmission and automatically transmitted to a data computation center server and an account storage center server on the internet through wireless means;

the transmitted data chain is bound with the user's personal identification information and automatically stored into a database corresponding to the application account which is established by the user for permanent storage and timely presentation;

after receipt of the data, the data computation center server performs automatic real-time computation and analysis of massive data with cloud computing, various individual life condition characteristic indicators are computed respectively upon the principle of elaborating various indicators in an inspection and analysis report required for disease diagnosis, the characteristic indicators are used to form an inspection and analysis report which meets the corresponding disease diagnosis standards of a medical institution, the report is transmitted to the account storage server and stored in the user's application account for timely presentation.

Preferably, the personal health information platform further comprising the extraction process of multiple characteristic indicators of brain condition, wherein, the original data of multiple characteristic indicators of brain condition is derived from real-time electroencephalographic wave data of the wireless mobile terminal for vital sign data acquisition and transmission, and automatically mathematically processed and computed by the computation service center to obtain multiple quantitative numeric indexes representing various brain conditions, and an effective quantitative cerebrate habit is able to be established by using objective quantitative real-time characteristic indexes of brain condition, so as to learn the method and skill of quantitatively controlling the brain condition.

Preferably, the personal health information platform further comprising the extraction of characteristic indicators of blood perfusion distribution, wherein, the original data of characteristic indicators of blood perfusion distribution is derived from real-time blood perfusion wave of the wireless mobile terminal for vital sign data acquisition and transmission, and automatically continuously mathematically processed and computed by the computation service center to obtain quantitative numeric indexes representing blood perfusion distribution at various positions, the blood perfusion is required by human body metabolism, and reducing of local metabolic requirement is able to be learned by training of reducing the blood perfusion and withstanding of the reduced blood perfusion, so as to establish a basic environment for human body organ protection and low perfusion withstanding in disease condition, rehabilitation and life prolonging. Compared with the prior art, the aforementioned technical solution of the present invention has the following advantages:

(1) The present invention provides a method for inspecting human body signal, comprising the steps of acquiring electroencephalographic wave on both sides of the forehead, supraorbital artery blood perfusion wave on both sides of the forehead and peripheral blood perfusion wave, and processing the acquired wave signal to compute a brain blood perfusion index, a peripheral blood perfusion index and characteristic indicators of the brain condition. As lots of information about human body conditions, such as sleep condition, emotion condition and even functioning condition of other parts of the human body, are reflected in the human brain and can be directly or indirectly obtained through brain information, it is applicable to obtain brain functioning condition by analyzing brain condition information so as to provide basis for human body condition evaluation and medical service.

(2) The method for inspecting human body signal as mentioned in the present invention also comprises inspection of other characteristics of human body, such as obtaining vascular elasticity, analyzing respiratory wave and etc., so that the functioning conditions of human body are reflected by multiple characteristics which provide various basis for health evaluation, human body condition inspection and medical service.

**Brief Description of the Drawings**

To make the contents of the present invention more easily understood clearly, further description of the present invention is presented below, in conjunction with specific embodiments and appended drawings, wherein:

Figure 1 is a schematic diagram of the brain inhibition therapy with closed-loop feedback;
Figure 2 is a structural diagram of the central computation and control management circuitry;
Figure 3 is a structural schematic diagram of the transducer;
Figure 4 is a schematic diagram of the signal flow;
Figure 5 is a circuit structural diagram of the amplifier for blood perfusion signal;
Figure 6 is a circuit diagram of the amplifier for penis hardness signal;
Figure 7 is a circuit diagram of the amplifier for penis cross-section diameter signal;
Figure 8 is a circuit diagram of the amplifier for penis blood perfusion signal;
Figure 9 is a circuit diagram of the amplifier for electromyographic signal;
Figure 10 is a circuit diagram of the amplifier for mouth-nose respiration signal;
Figure 11 is a circuit diagram of the amplifier for electroencephalographic signal;
Figure 12 is a circuit diagram of the amplifier for electrocardiographic signal;
Figure 13 is a circuit diagram of the amplifier for chest-abdominal respiration signal;
Figure 14 is a circuit diagram of the amplifier for eye movement signal;
Figure 15 is a circuit diagram of the amplifier for relative exercise amount signal;
Figure 16 is a circuit diagram of the amplifier for measuring body weight and urine volume;
Figure 17 is a scatter point diagram for quantitative measuring and computing brain condition;
Figure 18 shows the predetermined threshold values of brain condition indexes;
Figure 19 shows the system architecture of cloud computing;
Figure 20 a schematic diagram of the robot competition;
Figure 21 is a circuit diagram of the control circuit for mind traction control;
Figure 22 is a circuit diagram of the signal acquisition circuit of robot movement signal;
Figure 23 shows the information structure of a personal health bank account;
Figure 24 is a feedback architecture diagram of disease treatment using life condition data.

**Detailed Description of Embodiments**

**Embodiment 1:**

A life maintenance mode, comprising the steps of

(1) automatically measuring and computing life condition data, and classifying inspection items into quantitatively measured life condition data converted from individual tables, which mainly comprises:

converting signal of vital signs and physiological data into digital signal,
transmitting the signal to the filtering module, noise control module and input amplification module of the monitoring circuit, and after being computed and processed by corresponding monitoring modules, the signal is transmitted to an analog-digital convertor circuit of the central computation and control management circuitry through an analog signal input port;

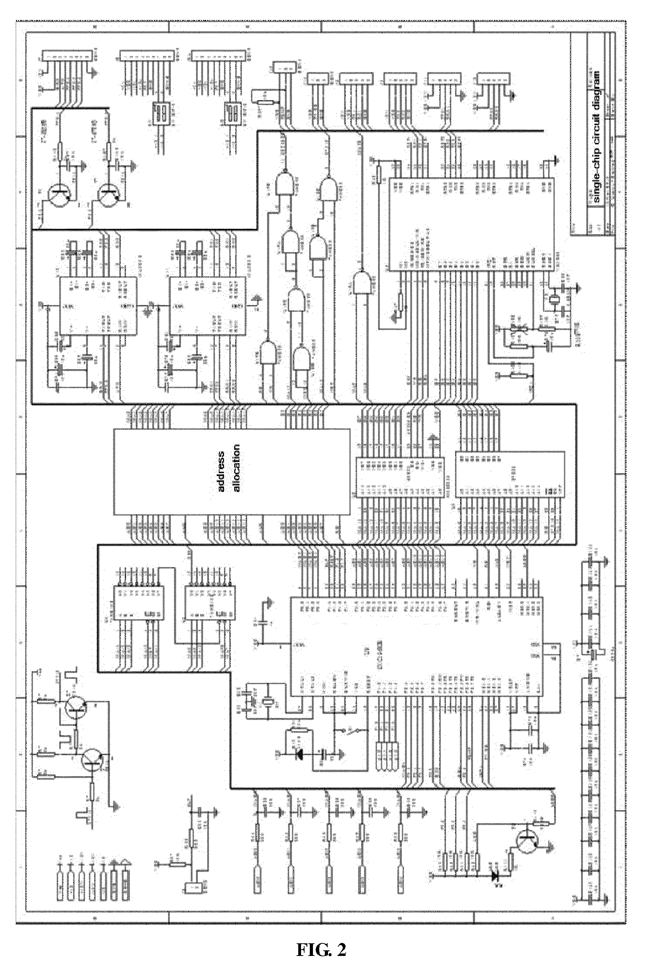

sending the processed signal to a communication interface of the central computation and control management circuitry through an RS232 interface circuit; the central computation and control management circuitry is shown in Figure 2,

encrypting and compressing the digitalized signal data received by the central computation and control management circuitry to obtain a processed data stream, which is then fed into a dynamic data link cache queue; the dynamic data link cache queue is a varying data storage and output configuration;

data configuration in a storage region is determined by network condition;

after an interruption event is triggered due to that the central computation and control management circuitry is notified of the change of network condition, the central computation and control management circuitry controls various permutations and combinations of the obtained data;

the data is then written into a data buffer of the storage queue upon a write instruction;

the data in the queue is outputted to a wireless internet access control circuit through a data port upon a read instruction of the central computation and control management circuitry;

the internet access control circuit carries out auto-dial to the network, network condition recognition, TCP/IP mode signal modulation, sub-package and outputting, so as to allow the data to be uploaded to the data storage server and data computation server.

Furthermore, it is required to acquire living condition data and subjective feeling data, which is achieved by using a subjective feeling information collector which includes a computerized equipment, such as a cell phone, a mobile tablet, a stationary computer, with a browser window of B/S configuration or an operation window of APP software as the input interface, logging the data by manual input, and selecting data items related to different feelings or parameters by click-choice on the input interface; uploading the data to the data storage and computation server of the personal health bank account directly through wireless or wired internet access of the computerized equipment.

Wherein, among the acquired signal, vital signs and physiological data further include: waveform signal: electroencephalographic wave, cerebral and peripheral blood perfusion wave, penis blood perfusion wave, electrocardiographic wave, pulse oximetry wave, mouth-nose-chest-abdominal respiration wave, electromyographic wave, eye movement wave, breath-end carbon-dioxide concentration wave;

numerical signal: pulse rate, non-invasive blood pressure, blood glucose, pulse oxygen saturation, breath-end carbon-dioxide concentration, inhaled oxygen concentration, body temperature, respiratory rate, penis cross-section diameter, penis hardness, penis temperature, penis arterial oxygen saturation, urine volume, relative exercise amount of limbs, body weight, exercise sweat amount.

Wherein, the acquired living condition data includes: medicine dosage, food intake, water intake, defecation amount.

Furthermore, the acquired subjective feeling data includes: pain rating scale, emesis rating scale, dizziness rating scale, living quality rating scale, chest distress, urination frequency, urine color, hemorrhage, diarrhea, constipation, excrement color, palpitation, shortness of breath, impotence, vertigo, asthma, pyrexia, dysphoria, anger, anxiety rating scale, depression rating scale, difficulty of falling asleep, dysphylaxia, dreaminess.

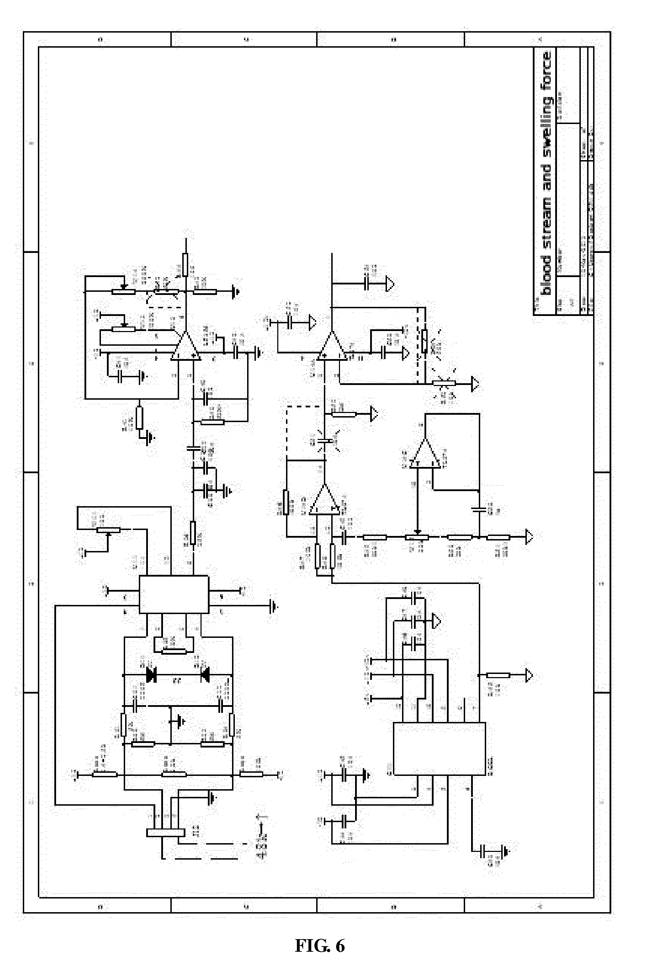

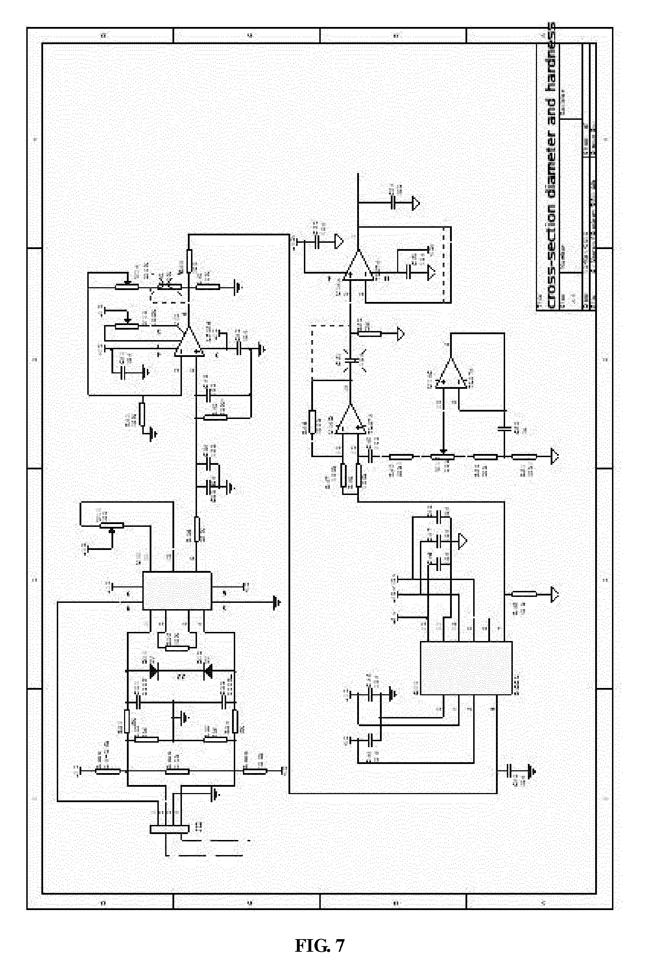

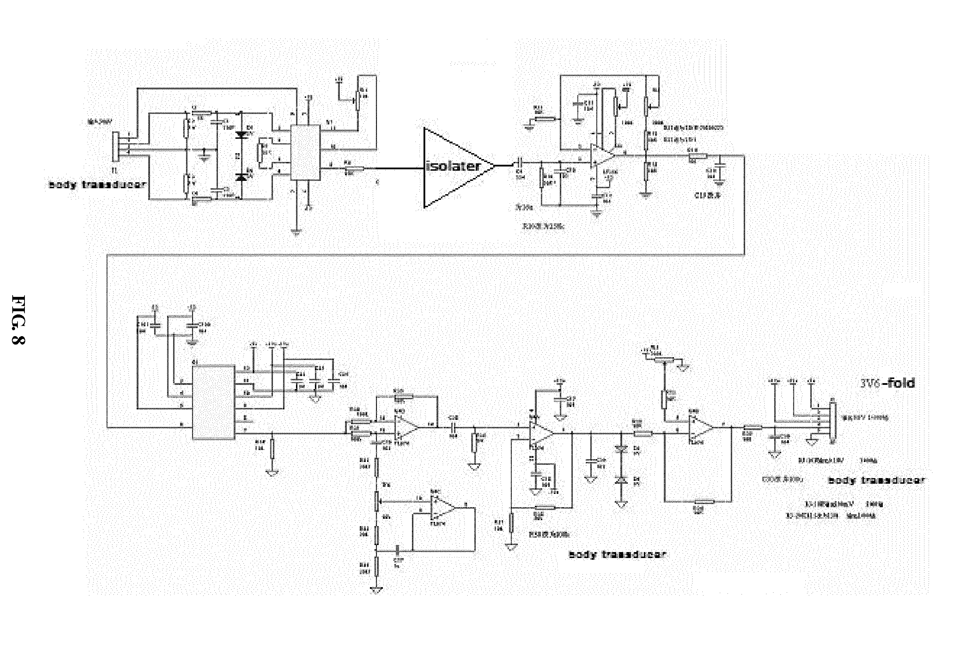

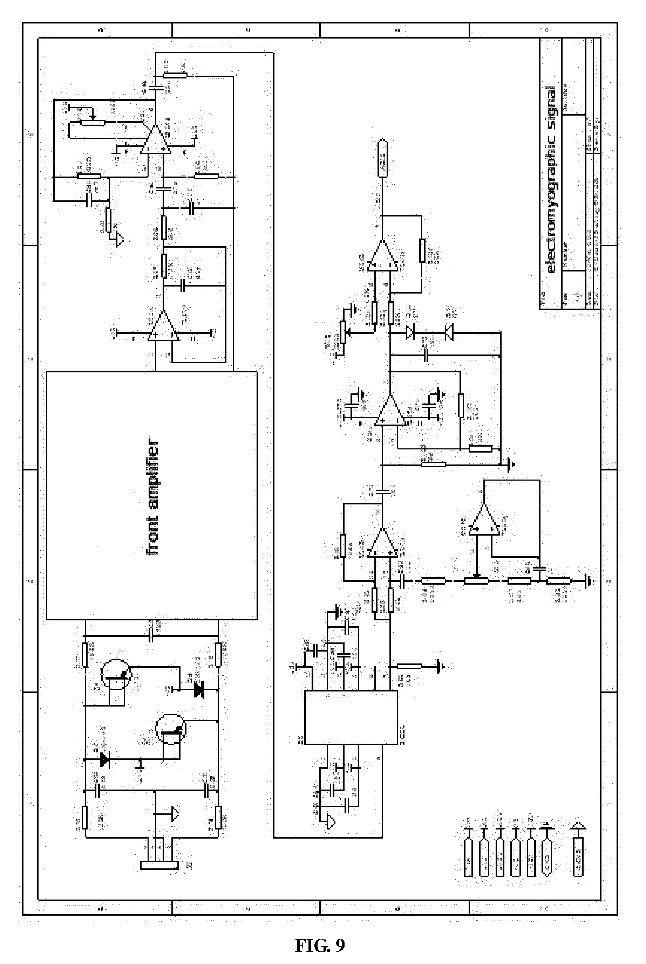

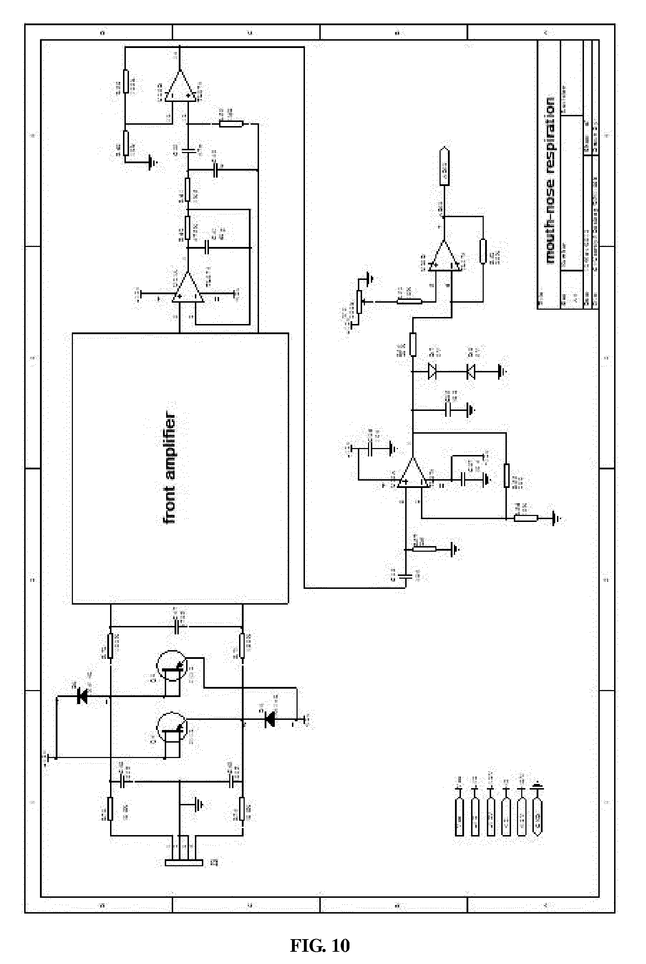

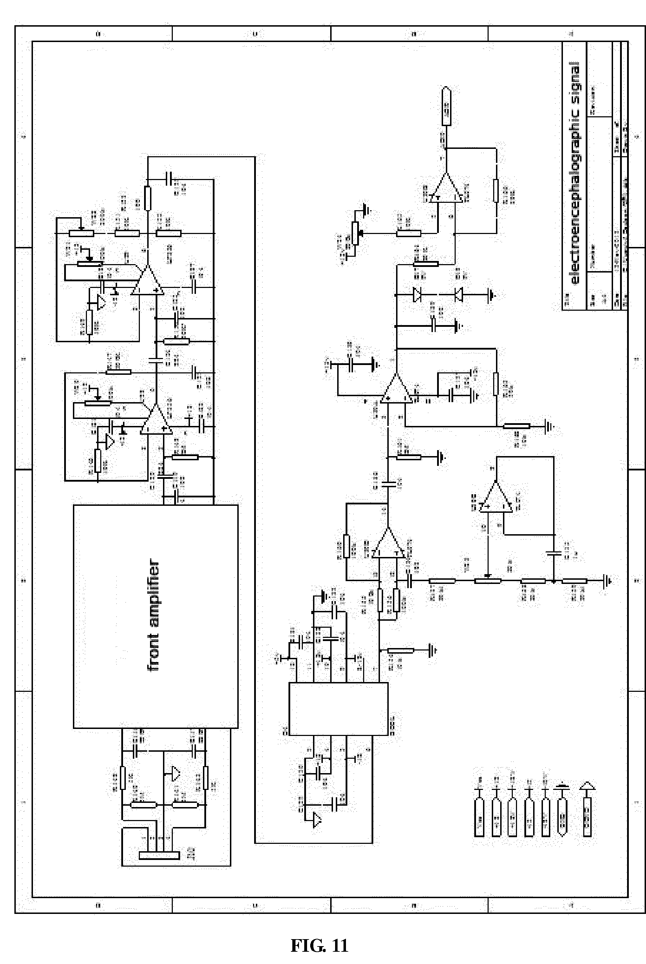

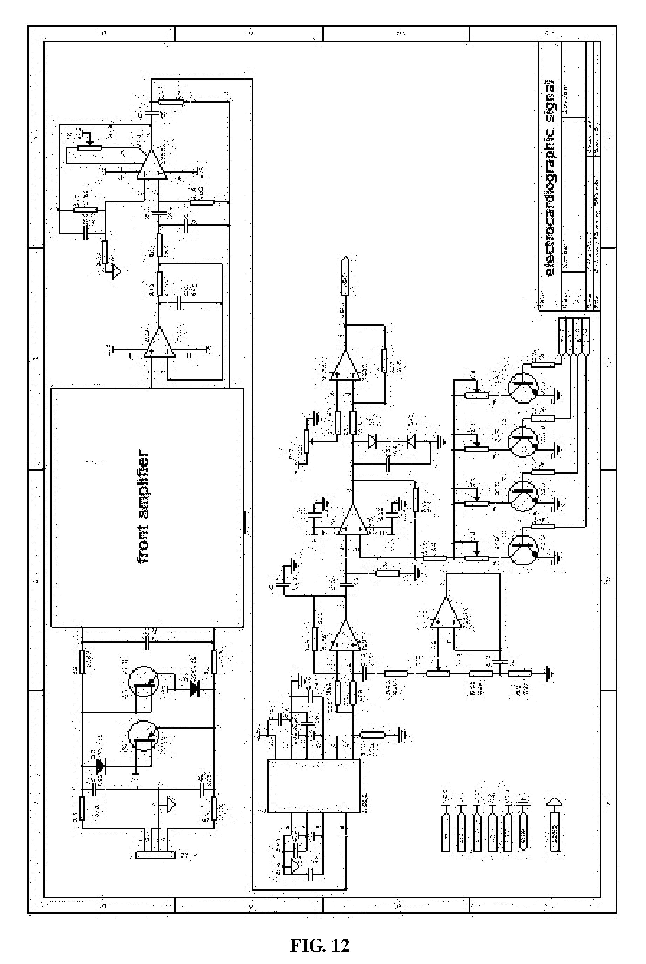

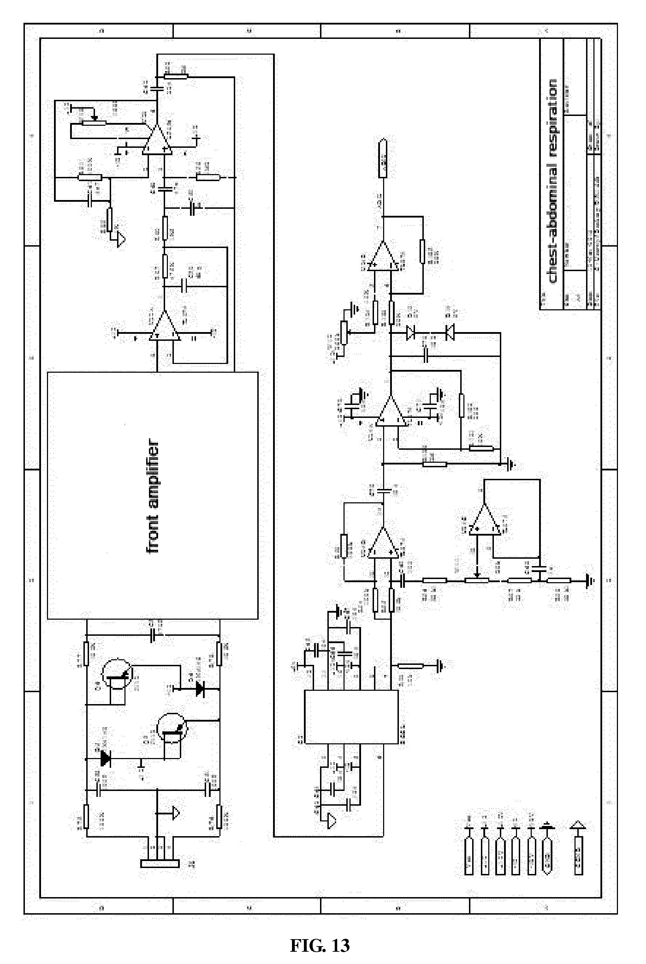

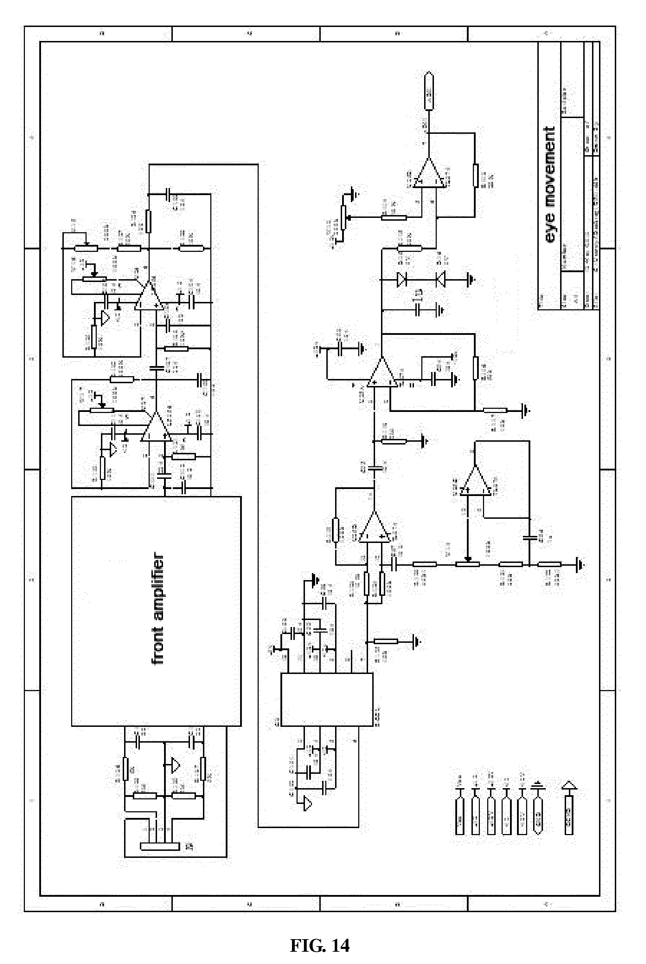

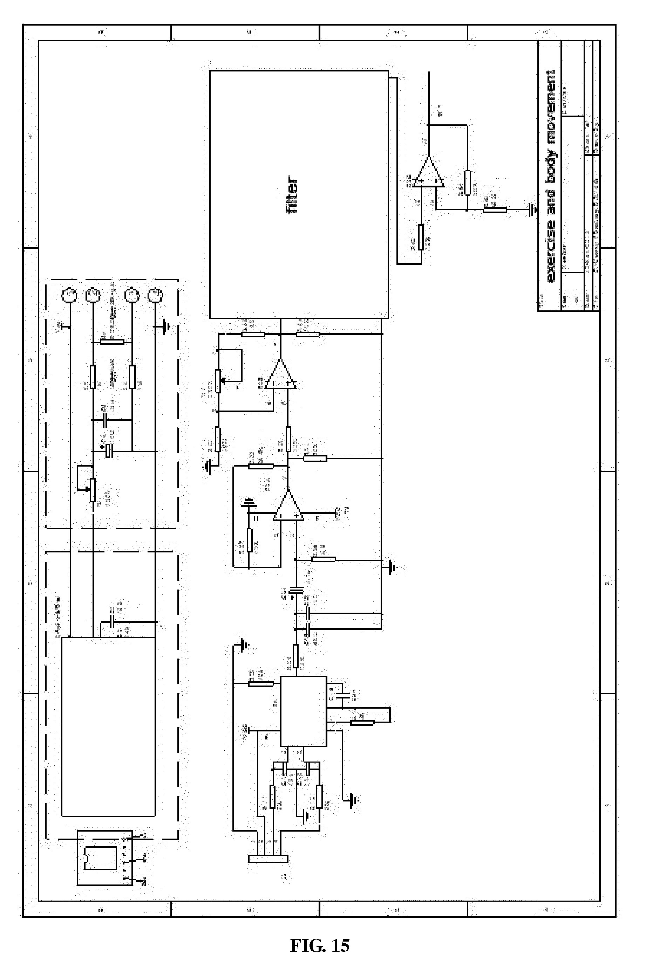

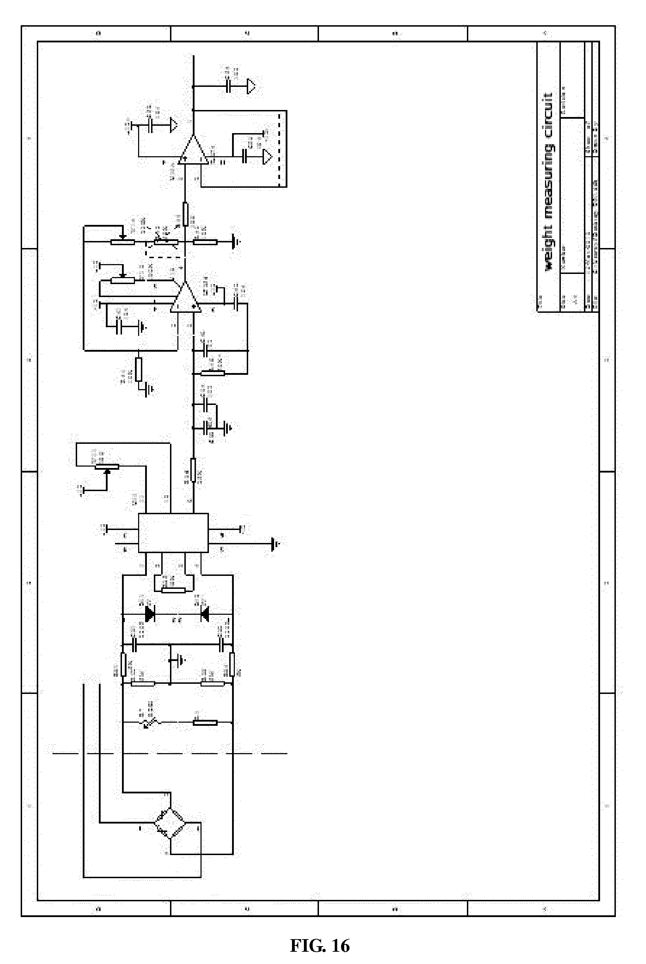

Wherein, Figure 6 is a circuit diagram of the amplifier for penis hardness signal; Figure 7 is a circuit diagram of the amplifier for penis cross-section diameter signal; Figure 8 is a circuit diagram of the amplifier for penis blood perfusion signal; Figure 9 is a circuit diagram of the amplifier for electromyographic signal; Figure 10 is a circuit diagram of the amplifier for mouth-nose respiration signal; Figure 11 is a circuit diagram of the amplifier for electroencephalographic signal; Figure 12 is a circuit diagram of the amplifier for electrocardiographic signal; Figure 13 is a circuit diagram of the amplifier for chest-abdominal respiration signal; Figure 14 is a circuit diagram of the amplifier for eye movement signal; Figure 15 is a circuit diagram of the amplifier for relative exercise amount signal; Figure 16 is a circuit diagram of the amplifier for measuring body weight and urine volume.

The wireless mobile terminal for acquisition and transmission of life condition information has three types of automatic life condition data collection capabilities including vital signs data collection, physiological data collection and subjective feeling data collection, with the ability to automatically transmit real-time data to wireless internet. The data transmission is bound with the personal identification account of the data owner, the machine number of the mobile terminal for information collection, and the data encryption algorithm. The life condition data collection includes acquisition of electroencephalographic wave, electrocardiographic wave, pulse rate, non-invasive blood pressure, blood glucose, pulse oxygen saturation, breath-end carbon-dioxide concentration, inhaled oxygen concentration, body temperature, mouth-nose respiration wave, chest-abdominal respiration wave, respiratory rate, facial electromyographic wave, eye movement wave, peripheral blood perfusion wave, cerebral blood perfusion wave, penis blood perfusion wave, penis cross-section diameter, penis hardness, penis temperature, penis arterial oxygen saturation, relative exercise amount of limbs (four limbs including two upper limbs and two lower limbs), body weight, exercise sweat amount, urine volume, subjective feeling (pain, emesis, dizziness, chest distress, urination frequency, hemorrhage, diarrhea, palpitation, shortness of breath, impotence, vertigo, asthma, pyrexia, dysphoria, dreaminess, psychological state), medicine dosage, food intake, water intake, defecation amount. The part for life condition data acquisition of the terminal device comprises a monitoring circuit, a computation and control circuit, a data processing circuit, a dynamic data link cache circuit, a wireless internet access control circuit, a power source circuit, etc.; the connection relation of the circuits is as follows: converting vital signs signal of the patient into electronic signal by using electrocardiographic, electroencephalographic, blood pressure and oximetric sensor probes on the patient's body, transmitting the electronic signal to the filtering module, noise control module and input amplification module of the monitoring circuit, and after being computed and processed respectively by corresponding monitoring modules (HXD_I), the signal is transmitted to an analog-digital convertor circuit and an RS232 interface circuit of the central computation and control unit through an analog signal input port and an RS232 communication port; encrypting and compressing the above-mentioned digitalized signal data received by the central computation and control unit to obtain a processed data stream which is then fed into a dynamic data link cache queue; the dynamic data link cache queue is a varying data storage and output configuration, and data configuration in a storage region thereof is determined by network condition; after an interruption event is triggered due to that the computation and control circuit is notified of the change of network condition, the computation and control circuit controls various permutations and combinations of the obtained data; the data is then written into a data buffer of the storage queue upon a write instruction; the data in the queue is outputted to a wireless internet access control circuit through a data port upon a read instruction of the computation and control circuit; the internet access control circuit carries out auto-dial to the network, network condition recognition, TCP mode signal modulation, sub-package and outputting; subjective feeling and voluntary event information is logged by click-choice on an internet browser mode interface of a configured mobile communication terminal device, such as a cell phone, a Pad, etc., and automatically transmitted by communication function.

(2) performing active control of the change of the life condition data by means of senior independent consciousness of cerebral cortex in combination with brain inhibition therapy to serve as a quantitative traction means for treatment, rehabilitation, and health and longevity. The brain inhibition therapy will be described in details in the subsequent embodiments. The brain inhibition therapy comprises the steps of acquiring a real-time blood perfusion index of the supraorbital artery as a feedback parameter, performing automatic feedback control over infusion of venous sedative with an infusion pump under sedative dosage, performing automatic feedback control over infusion of venous sedative with an infusion pump under sedative dosage, letting the oxygen metabolic requirement of senior cognition function of cerebral cortex decrease to the lowest level, and controlling inhibition of the senior cognition function of the cerebral cortex.

(3) composing a report of inspection analysis and health trend prediction by analyzing and computing the life condition data, so as to predict and prevent the occurrence and development of diseases.

(4) automatically sending messages to remind a patient at home to check the treatment behaviors, based on doctor's advices and prescriptions in the track record of medical treatment, and providing an automatic no-response alarming service.

In the present embodiment, there is established a novel life maintenance mode named as the third type of life maintenance mode, which comprises the steps of decomposing inspection items of clinical diagnosis which is usually carried out after a disease has occurred into a series of individual automatically measured and computed indicators of life condition data, which is popularized into people's daily life and work to be practiced continuously so as to produce continuous diagnosis and inspection analysis report and trend; performing active control of the change of the life condition data by means of senior independent consciousness of cerebral cortex with the automatically measured and computed life condition data as a tool (the data is changing during usage) to serve as a quantitative traction means for treatment, rehabilitation, and health and longevity; predict and prevent the occurrence and development of diseases on the basis of the produced inspection analysis report and trend; automatically sending messages to remind a patient at home to check the treatment behaviors on the basis of doctor's advices and prescriptions in the track record of medical treatment, and providing an automatic no-response alarming service. There is established a mode of intelligent Internet-of-Things application information service mode for life condition expression, adjustment and maintenance by man-machine interaction under all-time-space environment including acquisition, wireless transmission, storage, computation, review of human body life condition data and medical health data, inspection and analysis report clinical diagnosis, medical treatment track record, an Internet-of-Things macro-zone man-machine interaction system platform of real-time interaction, macro closed-loop voluntary treatment, automatic alarming, automatic reminding and automatic prediction and prevention of diseases, and establishment of personal digital health bank accounts. As used at home, the mode is carried out by decomposing medical clinical inspection items into individual standardized quantitative measurements for life condition, and self-organizing, analyzing and computing life condition information, so as to compose various inspection reports of disease physiological and vital sign data and health prediction reports; establishing a system for real-time quantitative multi-index data extraction for brain condition under the mode of Internet of Things, so as to establish an integrated extracting and expressing method of quantitative characteristic indicators of nerve electrophysiological and blood perfusion signal related to human natural sleep structure and quality; defining a brain blood perfusion unit and a peripheral blood perfusion unit through measurement of blood perfusion distribution, and establishing a training routine and method to respectively reduce metabolic requirement of the two units upon the relationship between blood flow and metabolism, so as to allow the organs to be able to withstand low blood perfusion level under disease condition; establishing a new life inspection and maintenance mode as a third life maintenance mode for disease treatment, disease rehabilitation, disease prevention and health keeping and life prolonging, so as to control the change of vital sign data upon independent consciousness, in order to achieve the goal of keeping healthy and prolonging life; introducing continuous quantitative precise measuring and computing of vital sign data and medical health data in the processes of disease treatment, disease prevention, disease rehabilitation and health keeping and life prolonging, changing negative emotion of a person under disease condition into positive emotion, and continuously perceiving the real-time change of outputted quantitative characteristic indicators of vital signs and physiological data, so as to enhance the disease treatment effect and disease rehabilitation effect and achieve the goal of disease prevention and life prolonging, wherein, the data and technique produced during the processes of disease treatment, rehabilitation, disease prevention and health keeping and life prolonging are used as a tool, and based on wireless network communication, the system of aiding or directly driving the processes of disease treatment, rehabilitation, disease prevention and health keeping and life prolonging for people is established; introducing games and competitions into the processes of disease treatment, rehabilitation, disease prevention and health keeping and life prolonging with these data and technique as a tool, so as to continuously promote health levels and at the same time bring about the objective effect of redistribution of social wealth; establishing a media broadcasting system for demonstration pioneers of health keeping, life prolonging, disease treatment and disease rehabilitation, broadcasting treatment track record and vital signs data of the pioneers at regular time, so as to provide a demonstration example for quantitative health data comparison, learning and imitation of the processes of disease treatment, disease rehabilitation and health keeping and life prolonging for people through real-time media transmission. The system platform comprises a system software platform and hardware devices including a wireless mobile terminal for vital signs data acquisition and transmission, a data computation service center server and a data storage center server, a mobile computer terminal or a stationary computer, and a mobile communication terminal. An internet communication platform is used as a communication transmission media, wherein, the users are allowed to register their own application accounts with the health bank website and bind the accounts with their personal identification information. Each user uses a wireless mobile terminal for vital signs data acquisition and transmission in living and working environment, with each terminal temporarily bound (for rent) or fixedly bound (for purchase) with the application account of the user, so that information of vital signs, subjective feeling and medicine intake events of the user are automatically acquired in daily usage through the wireless mobile terminal for vital signs data acquisition and transmission and automatically transmitted to a data computation center server and an account storage center server on the internet through wireless means. The transmitted data chain is bound with the user's personal identification information and automatically stored into a database corresponding to the application account which is established by the user for permanent storage and timely presentation. After receipt of the data, the data computation center server performs automatic real-time computation and analysis of massive data with cloud computing, various individual life condition characteristic indicators are computed respectively upon the principle of elaborating various indicators in an inspection and analysis report required for disease diagnosis, the characteristic indicators are used to form an inspection and analysis report which meets the corresponding disease diagnosis standards of a medical institution, the report is transmitted to the account storage server and stored in the user's application account for timely presentation. By controlling the change of life condition data with independent consciousness, there is established a precise, normative, scientific and quantitative standard procedure and method for large-scale popularized disease treatment, disease prevention, disease rehabilitation and health keeping and life prolonging based on wireless internet platform, as a third type of human body health maintenance and disease treatment mode which is parallel to Chinese traditional treatment and Western medicine treatment. The computed results of individual characteristic indicators, when synchronized and stored, are at the same time automatically and at real-time sent to a stationary computer or a handheld mobile terminal of the user, or sent to a movement control and driving unit of an actual object so as to drive a game process or the movement of the actual object, thereby transforming the change of characteristic indicators of life condition into a game progress or a competition of controlling the movement of an actual object. By means of perceiving and learning to control the game progress or the movement of the actual object, the ability to control characteristic indicators of life condition is learned at the same time, and thereby the method to treat disease and to improve and keep health is learned. The kernel of this method is utilizing the human basic instinct of gaming and competition adventure as well as the desire to earn money on the basis of the brain physiological feature of internal reward mechanism, thereby driving a continuous process of disease treatment, disease prevention, disease rehabilitation and health keeping and life prolonging, and building a good psychological environment of a focused and pleasant progress of disease treatment and health keeping. In the extraction process of multiple characteristic indicators of brain condition, the original data is derived from real-time electroencephalographic wave data of the wireless mobile terminal for vital sign data acquisition and transmission, and automatically mathematically processed and computed by the computation service center to obtain multiple quantitative numeric indexes representing various brain conditions, so that an effective quantitative cerebrate habit is able to be established by using objective quantitative real-time characteristic indexes of brain condition so as to learn the method and skill of quantitatively controlling the brain condition. In the extraction process of characteristic indicators of blood perfusion distribution, the original data is derived from real-time blood perfusion wave of the wireless mobile terminal for vital sign data acquisition and transmission, and automatically continuously mathematically processed and computed by the computation service center to obtain quantitative numeric indexes representing blood perfusion distribution at various positions, as the blood perfusion is required by human body metabolism, reducing of local metabolic requirement is able to be learned by training of reducing the blood perfusion and withstanding of the reduced blood perfusion, so as to establish a basic environment for human body organ protection and low perfusion withstanding in disease condition, rehabilitation and life prolonging. The storage of personal health data on the server is similar to the storage of fund in the bank, and is by means of establishing a personal life condition account. The life health information under each account is stored in a digitalized manner to form an electronic medical health core data record which is privately owned and has an attribute of privacy, with data architecture in accordance with the standard of medical clinical information architecture, and the information can be accessed, displayed, interacted, processed and controlled in an identification recognition manner on an controlled computer terminal. The data stored in an account is incorporated with real-time processing function, so as to automatically extract disease characteristic indicators at real-time for composing of a diagnosis technical report and obtaining of disease treatment raw data. It is possible to automatically extract treatment information and measurement inspection information (medicine dosage or blood pressure, blood glucose, body temperature measurements) at real-time, to send the information to a personal mobile terminal (a cell phone, a mobile computer, a stationary computer) at real-time, and to notify the user to carry out the corresponding treatment process by voice reminder, short message, screen display, and if no confirming reply message is received within a predetermined duration, the account will automatically send a message to a predetermined mobile communication device or stationary computer so as to submit a request for caring, or even an alarm request for first-aid, towards another person or an institution. The account automatically carries out preliminary health prediction upon medical health information in a period of time, and sends the prediction result to a personal mobile terminal or a computer of a predetermined health service provider for display and reminding at regular time. The account owner uses a web browser as an application window for human-machine interaction, operates and achieves control of timely access and display of medical health data, establishes a remote interaction communication window for real-time interaction, selects information to be transmitted to a receiver who is allowed to read the information. A daily popularized information flow of disease treatment, disease rehabilitation, disease prevention and health keeping and life prolonging is established on mass media, and used as a tool for people's daily health consumption. A pioneer leading mode of multiple disease treatment, disease rehabilitation, disease prevention and life prolonging based on all-time-space data is established by means of establishing a broadcasting platform for health information of pioneers. The correct rehabilitation processes of gradually improving or fully recovered patient of certain diseases, as well as the life condition data of individuals with longevity, are automatically disclosed and broadcasted at real-time on multiple media, thereby providing a daily objective quantitative leading information and data base for corresponding groups of people to imitate, compare and predict their own progress of disease and development of health and longevity. Thus a self-adapted and quantitatively compared health management mode in accordance with market-oriented economic behavior is established so as to provide contents for mass media to promote positive energy.

**Embodiment 2:**

In the present embodiment, there is provided a brain inhibition therapy, as shown in the schematic diagram of the brain inhibition therapy with closed-loop feedback, which mainly comprises the steps of

(1) acquiring a real-time blood perfusion index of the supraorbital artery as a feedback parameter.
(2) performing automatic feedback control over infusion of venous sedative with an infusion pump under sedative dosage, which comprises the following sub-steps of
performing closed-loop feedback control over the infusion of the venous sedative with the infusion pump by using characteristic indicators of the brain blood perfusion index, wherein, the feedback control mechanism is a steady-state regulation system which controls the brain blood perfusion index to reach the minimum;
performing closed-loop feedback control over the infusion of the venous sedative with the infusion pump by using the characteristic indicator of brain blood perfusion index of the supraorbital artery as a feedback parameter, to achieve the control target that the brain blood perfusion index of the supraorbital artery reaches the minimum;
acquiring the brain blood perfusion signal of the supraorbital artery by using signal acquisition and computation technology of blood perfusion of the supraorbital artery, through a wireless mobile terminal for acquisition and transmission of vital signs, transmitting the signal to a processing computer through WIFI, allowing the computer to automatically compute and process the signal to obtain quantitative data of the real-time blood perfusion index, and then sending a control instruction to a computer interface of the infusion pump through a standard RS232 communication interface, so as to control the automatic infusion of sedative with the infusion pump, thereby achieving the purpose of inhibition of cerebral cortex cognition function.

(3) letting the oxygen metabolic requirement of senior cognition function of cerebral cortex decrease to the lowest level

(4) controlling inhibition of the senior cognition function of the cerebral cortex.

Target Controlled Infusion (TCI) is adopted as the control mechanism, with the blood drug concentration as a controlled object and the blood perfusion index as a feedback regulation parameter, the blood perfusion of the supraorbital artery is adjusted to the minimum by increasing or reducing the blood drug concentration, so as to quantitatively control the automatic sleep sedation therapeutic treatment.

Blood perfusion has clear influence on disease treatment, especially treatment of serious disease. Active control of the change of brain blood perfusion and periphery blood perfusion (except for brain diseases) of the human body is able to enhance the treatment effect. Senior cognition function of cerebral cortex expends energy and consumes oxygen carried in blood stream, therefore increases blood perfusion towards the brain. Since this kind of energy consumption has certain harmful effect on disease treatment, the method of controlling background environment of disease treatment which is named as brain inhibition therapy, by controlling the energy requirement of senior cognition function of the brain and temporarily inhibiting the cognition function of the brain into dormancy so as to carry out disease treatment under such background environment, is able to cause blood perfusion transported towards the brain to decrease and blood perfusion at the nidus to increase, so that more of immunocytes, antibodies and oxygen carried in blood stream will arrive at the nidus to produce beneficial effect for disease treatment. This is achieved by performing closed-loop feedback control over the infusion of the venous sedative with the infusion pump by using the characteristic indicator of brain blood perfusion index of the supraorbital artery as a feedback parameter, with the feedback control mechanism being a steady-state regulation system, to achieve the control target that the brain blood perfusion index of the supraorbital artery reaches the minimum; as well as acquiring the brain blood perfusion signal of the supraorbital artery by using signal acquisition and computation technology of blood perfusion of the supraorbital artery, through a wireless mobile terminal for acquisition and transmission of vital signs, transmitting the signal to a processing computer through WIFI, allowing the computer to automatically compute and process the signal to obtain quantitative data of the real-time blood perfusion index, and then sending a control instruction to a computer interface of an infusion pump through a standard RS232 communication interface, so as to control the infusion pump (e.g., a Graseby 3400 infusion pump) to automatically infuse the sedative, with Target Controlled Infusion (TCI) as the control mechanism, thereby automatically controlling the blood perfusion of the supraorbital artery to reach the minimum.

**Embodiment 3:**

In the present embodiment, there is provided an encephalic signal acquisition method for the brain inhibition therapy, and this method is used for acquiring a real-time blood perfusion index of the supraorbital artery as a feedback parameter as described in Embodiment 2 and comprises the steps of

acquiring electroencephalographic wave on both sides of the forehead, supraorbital artery blood perfusion wave on both sides of the forehead, and peripheral blood perfusion wave;

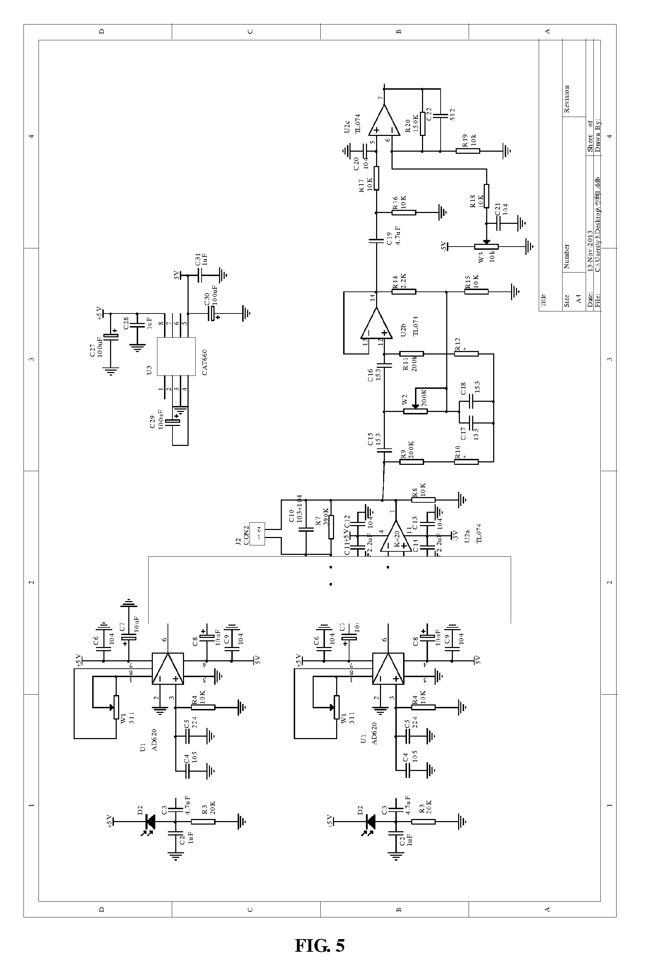

letting the electroencephalographic wave, the supraorbital artery blood perfusion wave and the peripheral blood perfusion wave be transmitted to a front electrical signal amplifying circuit through a transducer, and then transmitted to an analog-digital convertor circuit to be converted into a group of discretized computer data set after being integrated, filtered and amplified by an analog circuit; the circuit structural diagram of the amplifier for blood perfusion signal is shown in Figure 5;

computing a brain blood perfusion index based on the supraorbital artery blood perfusion wave on both sides of the forehead;

computing a peripheral blood perfusion index based on the peripheral blood perfusion wave;

processing the electroencephalographic wave to obtain characteristic indicators of the brain condition. The characteristic indicators of the brain condition includes one or more of brain relaxing index, brain concentrating index, brain activity index, brain thinking index, brain restraining index, left-right brain lateralization index, brain stability index and brain sedation index.

Furthermore, the method also comprises the step of computing vascular elasticity based on the electrocardiographic wave and the blood perfusion signal, as well as computing a brain power spectrum based on the electroencephalographic wave and the characteristic indicators of the brain condition.

The method further comprises the step of establishing a brain condition scatter point diagram based on the characteristic indicators of the brain condition, so as to obtain the change of the brain condition.

The supraorbital artery blood perfusion wave on both sides of the forehead is acquired with a pressure sensor, a laser sensor and an electroencephalogram sensor on the forehead.

In the present embodiment, a system for real-time quantitative multi-index data extraction for brain condition under the mode of Internet of Things is established, with data extraction of characteristic indicators of electroencephalographic wave and change of brain blood perfusion related to brain condition, wherein, acquiring physiological signal of double-conduction electroencephalographic wave and double-conduction supraorbital artery blood perfusion wave to obtain multi-sequence discrete electroencephalographic and blood perfusion data; performing a combination of wavelet algorithm, spectrum analysis algorithm, waveform recognition algorithm and fuzzy algorithm on the electroencephalographic data, with the computing objects including first-level data, result data produced by first-level calculation, as well as multi-level calculation and multiple regression iteration, which is fitted with weighting factor sequence of basic change characteristic data of electroencephalographic wave under operative anesthesia, so as to finally obtain a group of quantitative real-time characteristic indicators describing various conditions of the brain in sober state, including concentrating index **i_22**, relaxing index **i_35**, thinking intensity index **i_48**, sense organ sensitivity index **i_52,** readiness index **i_20**, memory processing index **i_60,** left-right brain excitement ratio index **i_50,** sedation level index **WLi** (wavelet index); performing wavelet analysis algorithm and calculus algorithm on the supraorbital artery blood perfusion data, so as to obtain a group of quantitative real-time characteristic indicators including mind dispersion index **g_10,** inhibition index **g_12,** forehead myographic index **g_15.** Multiple regression weighting algorithm is performed on the dataset of the aforementioned quantitative characteristic indicators, so as to obtain a group of condition indicators of the brain in tranquillization state, including a series of quantitative data analysis results of left-right brain lateralization index, inner concentrating index, outer concentrating index, response speed index, brain restraining index, brain relaxing index, brain activity index, brain fatigue index, sleepy index, memory processing index, brain energy consumption index, brain stability index, thinking intensity index, brain tranquillization time ratio, brain distraction times, closed-eye time ratio, open-eye time ratio, blink times, eye muscle tremor time ratio, left brain excitement time ratio, right brain excitement time ratio, outer concentrating time ratio, inner concentrating time ratio, etc. The real-time data resolution time window is 1.2 seconds, the brain condition result time window is 6 to 12 minutes, and the indexes are non-dimensional indexation numeric values. The signal of electroencephalographic wave and blood perfusion wave is transmitted to a front electronic signal amplifying circuit through a transducer, and then transmitted to an analog-digital convertor circuit to be converted into a group of discretized computer dataset after being integrated, filtered and amplified by an analog circuit, and the packed data is transmitted to a wireless communication control circuit after data stream control, encryption computation and data identification-address binding integration by the CPU chip, and then transmitted to the wireless internet platform through data cache after automatic internet access recognition, internet linking and TCP/IP data packing by the communication control circuit. A computer terminal receives the real-time data through internet, decodes and computes the data at real-time, so as to complete a quantitative analysis computation of brain condition, under the guidance of real-time voice, thereby obtaining a group of quantitative, pervasive, comparable characteristic indicators of brain condition.

1. **i_35**: brain relaxing index
2. **i_22:** brain concentrating index
3. **i_60:** brain activity index
4. **i_48**: brain thinking intensity index
5. **i_52**: brain restraining index
6. **i_66**: left-right brain lateralization index (left-right brain harmony index)
7. **i_45**: brain stability index
8. **WLi:** brain sedation level index
9. brain energy consumption: level of brain excitement
10. brain concentrating power: average concentrating ability
divided into: inner concentrating - focusing on self concentrating
outer concentrating - focusing on exterior communication
11. brain restraining (sense shutting): ability of shutting self input of hearing, seeing and somatosensory
12. brain relaxing: average ability of self relaxing and meditation
13. memory processing: the brain is under the state of memory processing means that the brain is drifting in and out of sleep
14. brain fatigue: fatigue level of the brain
15. brain restraining: ability of shutting sense organ input
16. brain stability: ability of maintaining at one constant brain condition
17. brain tranquillization: empty and quiet mentality of the brain, free imagination state
18. sleepy: sleepy level of the brain
19. response intensity: transformation speed between different brain conditions
20. current condition of the brain: a total evaluation score of brain power and brain health

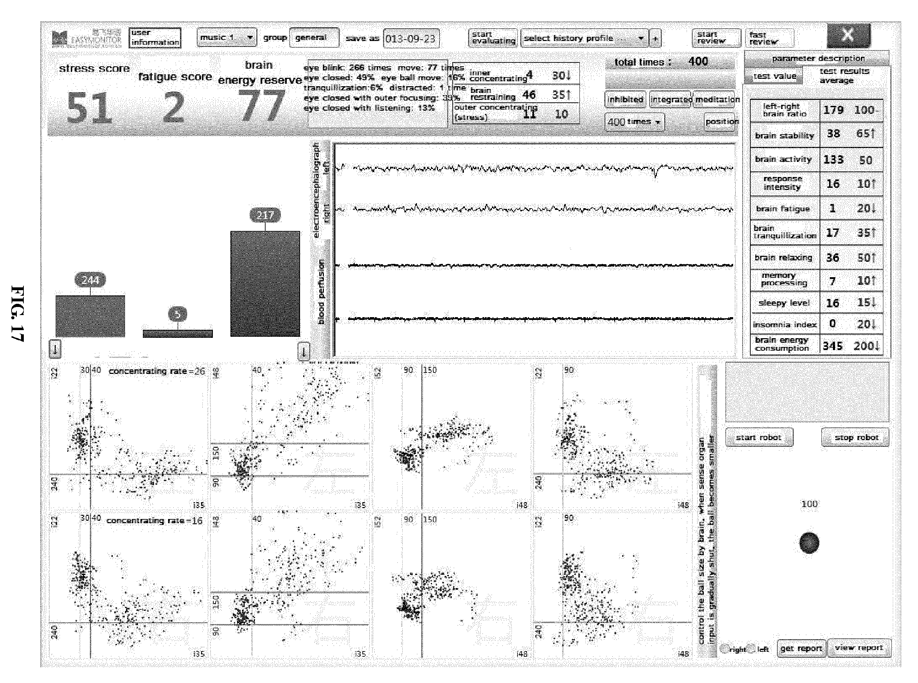

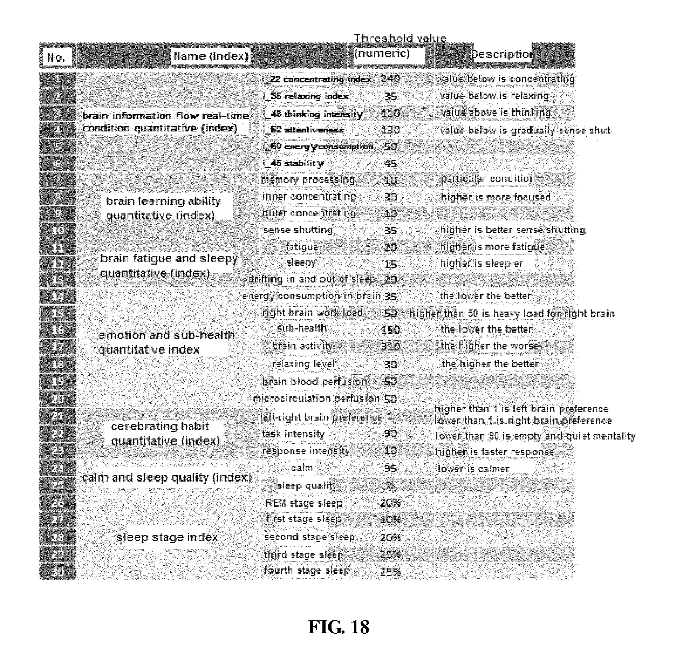

Plotting a scatter point diagram based on the acquired indexes of left-right brain real-time concentrating index and relaxing index **(i_22**, **i_35)**, real-time thinking intensity index and relaxing index **(i_48, i_35)**, real-time sense organ shutting index and thinking intensity index **(i_52, i_48)**, so as to express the morphological change of left-right brain condition. Setting **i_22**, **i_48**, **i_52** as vertical coordinates and **i_35**, **i_48** as horizontal coordinates, and respectively drawing a horizontal line at the scale point 240 of the vertical coordinate **i_22**, a vertical line at the scale point 40 of the horizontal coordinate **i_35** and a vertical line at the scale point 90 of the horizontal coordinate **i_48**, so as to divide the coordinate area into 4 quadrants. Each data point of **i_22**, **i_35**, **i_48**, **i_52** is shown as one point on the scatter point diagram. Various changes of brain condition are expressed by the density and distribution of the scatter points. By means of the left-right brain scatter point diagram, the brain condition is demonstrated in the images, and information such as convergence and divergence rate, cluster groups can be quantitatively extracted from the distribution of the scatter points. Figure 17 shows a scatter point diagram for quantitative measuring and computing brain condition; Figure 18 shows the predetermined threshold values of brain condition indexes.

**Embodiment 4:**

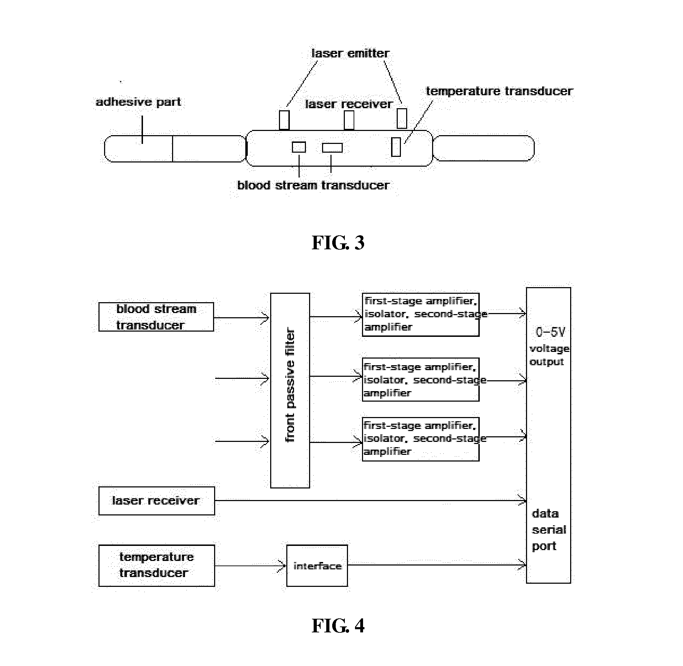

In the present embodiment, there is provided an device with a signal transducer of electroencephalographic wave and forehead blood perfusion wave and an independent transducer of peripheral blood perfusion wave integrated therein, comprising an integrated signal transducer of double-conduction electroencephalographic wave and double-conduction supraorbital artery blood perfusion wave, and an independent four-conduction transducer unit of peripheral blood perfusion wave, wherein, due to change of erythrocytes carried by blood stream of a blood vessel, the blood stream exerts a force onto the blood vessel wall so as to form a pulse;
a near-infrared laser emitter of the sensor illuminates the blood vessel to produce a reflective wave, and the reflective wave signal is received by a laser receiver on the lateral side and used as a first output signal; meanwhile, the pulse wave of the blood vessel wall is detected by a pressure transducer at the blood vessel and used as a second output signal; the first output signal and the second output signal are processed by a control computer chip to obtain a blood perfusion wave;
dry electrode metal pieces are used in the electroencephalogram sensor as medium to be affixed onto the forehead, so as to conduct cerebral electrical signal as a third output signal.
The integrated transducer is fixed on a flexible silica gel, the size of which is within $12 \times 1.4 \times 0.4$cm and which is elastic, strip shaped, and able to be formed into an annular shape and affixed by adhesive tape of a medical band-aid; the peripheral blood perfusion transducers detect blood perfusion signal and are fixed on a flexible silica gel, the size of which is within $3 \times 1 \times 0.3$cm. This is shown in Figure 3.
The operation method of the above-mentioned device is as follows:

the output signal of the transducer is converted into recognizable analog electrical signal by an electrical signal conversion and amplification unit which comprises an electroencephalographic signal acquisition and amplification module and a blood vessel blood perfusion wave signal acquisition and amplification module, the tiny signal acquired by the transducer is amplified and filtered to eliminate interference and void parts, transmitted to an analog-digital convertor, discretized and converted into digital signal, and then allocated, computed, encrypted and compressed by the control computer chip;
the processed data stream is fed into a dynamic data link cache queue, the dynamic data link cache queue is a varying data storage and output configuration, and the data configuration in a storage region thereof is determined by network condition;
after an interruption event is triggered due to that the central computation and control management circuitry is notified of the change of network condition, the central computation and control circuitry controls various permutations and combinations of the obtained data;
the data is then written into the storage queue upon a write instruction; the data in the queue is outputted to a wireless internet access circuit through a data port upon a read instruction of the control computer chip; the internet access circuit carries out auto-dial to the network, network condition recognition, TCP mode signal modulation, sub-package and outputting. The schematic diagram of the signal flow is shown in Figure 4.

In the present embodiment, there is provided a device with a signal transducer of electroencephalographic wave and forehead blood perfusion wave and an independent transducer of peripheral blood perfusion wave integrated therein, comprising an integrated signal transducer of double-conduction electroencephalographic wave and double-conduction supraorbital artery blood perfusion wave, and an independent four-conduction transducer unit of peripheral blood perfusion wave. Due to change of erythrocytes carried by blood stream of a blood vessel, the blood stream exerts a force onto the blood vessel wall so as to form a pulse. A near-infrared laser emitter of the sensor illuminates the blood vessel to produce a reflective wave, and the reflective wave signal is received by a laser receiver on the lateral side and used as a first output signal. Meanwhile, the pulse wave of the blood vessel wall is detected by a pressure transducer at the blood vessel and used as a second output signal. The first output signal and the second output signal are processed by a control computer chip to obtain a blood perfusion wave. Dry electrode metal pieces are used in the electroencephalogram sensor as medium to be affixed onto the forehead, so as to conduct cerebral electrical signal as a third output signal. The integrated transducer is fixed on a flexible silica gel, the size of which is within $12\times1.4\times0.4$cm and which is elastic, strip shaped, and able to be formed into an annular shape and affixed by adhesive tape of a medical band-aid. The peripheral blood perfusion transducers which detect blood perfusion signal are respectively fixed on a flexible silica gel, the size of which is within $3\times1\times0.3$cm. The peripheral blood perfusion transducers are respectively affixed on limb ends by adhesive tape of a medical band-aid. The output signal of the transducer is converted into recognizable analog electrical signal by an electrical signal conversion and amplification unit which comprises an electroencephalographic signal acquisition and amplification module and a blood vessel blood perfusion wave signal acquisition and amplification module, the tiny signal acquired by the transducer is amplified and filtered to eliminate interference and void parts, transmitted to an analog-digital convertor, discretized and converted into digital signal, and then allocated, computed, encrypted and compressed by the control computer chip; the processed data stream is fed into a dynamic data link cache queue, the dynamic data link cache queue is a varying data storage and output configuration, and the data configuration in a storage region thereof is determined by network condition; after an interruption event is triggered due to that the central computation and control management circuitry is notified of the change of network condition, the central computation and control circuitry controls various permutations and combinations of the obtained data; the data is then written into the storage queue upon a write instruction; the data in the queue is outputted to a wireless internet access circuit through a data port upon a read instruction of the control computer chip; the internet access circuit carries out auto-dial to the network, network condition recognition, TCP mode signal modulation, sub-package and outputting.

**Embodiment 5:**

In the present embodiment, there is provided a sleep analysis method, with the sleep analysis based on the above-mentioned brain condition signal, comprising the steps of

extracting physiological signal indicating change of sleep quality which includes a brain blood perfusion index and a peripheral blood perfusion index, by using nerve electrophysiological measuring method in conjunction with brain blood perfusion measuring method, so as to establish a vital sign expressing means and characteristic indicators for quantitatively expressing and indicating condition change of sleep process, sleep structure and sleep quality;

objectively describing sleep quality by using the brain blood perfusion index and the peripheral blood perfusion index as a major physiological signal basis for sleep measurement, assisted by electroencephalographic signal, so as to reveal the relative change of brain blood perfusion and peripheral blood perfusion during sleep, and indicate the quantitative physiological change process of sleep influencing disease treatment and health and longevity;

automatically extracting electroencephalographic characteristic indicators which quantitatively describe change of sleep structure and brain blood and peripheral blood perfusion distribution characteristic indicators which quantitatively reflect changes of sleep quality and health condition, by real-time computation of the acquired multi-conduction electroencephalographic wave and multi-conduction blood perfusion wave;

acquiring the multi-conduction electroencephalographic waves and multi-conduction blood perfusion waves of a human in various conditions by using transducers for acquiring the signal of electroencephalographic wave, brain blood and peripheral blood perfusion wave, and computing and processing the data at real-time through a computer system which receives the same, so as to obtain real-time data of quantitative characteristic indicators;

performing real-time data computation with data pre-processing algorithms including waveform recognition algorithm, fuzzy control algorithm, spectrum analysis algorithm, wavelet analysis algorithm, multiple regression algorithm, and calculus algorithm;

computing frequency domain power and phase change of the electroencephalographic wave signal under a specific time domain window, and computing multi-variant components of multi-scale wavelet decomposition scale-space under a specific generating function with spectrum analysis algorithm and wavelet analysis algorithm of the electroencephalographic wave data with time window weighting, obtaining converted and inverse-converted cluster of data with inverse transformation algorithm of time domain component reconstruction, and obtaining a cluster of wavelet discrete values after processing the converted and inverse-converted cluster of data with fuzzy algorithm and threshold value extraction algorithm;

obtaining a series of quantitative electroencephalographic characteristic indicators representing various sleep conditions, after multiple regression algorithm incorporating sleep condition and weighting indexation;

obtaining the waveform change-point of multiple-cluster time domain blood perfusion waveforms of the multi-conduction blood perfusion wave with multi-scale wavelet analysis algorithm of the blood perfusion wave data under a specific generating function, and then obtaining the dynamic time domain power and relative speed of the blood perfusion wave with calculus algorithm and fitting computation, so as to produce quantitative characteristic indicators describing brain blood and peripheral blood perfusion signal for various sleep quality and sleep structure during sleep.

In the present embodiment, there is provided a data exchange method for automatically obtaining, from a cluster of decomposed characteristic indicators directing to various change and development of diseases, those characteristic indicators of brain and peripheral blood perfusion distribution relating to sleep structure, sleep quality and heath condition. Wherein, extracting physiological signal indicating change of sleep quality which includes a brain blood perfusion index and a peripheral blood perfusion index, by using nerve electrophysiological measuring method in conjunction with brain blood perfusion measuring method, so as to establish a vital sign expressing means and characteristic indicators for quantitatively expressing and indicating condition change of sleep process, sleep structure and sleep quality; objectively describing sleep quality by using the brain blood perfusion index and the peripheral blood perfusion index as a major physiological signal basis for sleep measurement, assisted by electroencephalographic signal, so as to reveal the relative change of brain blood perfusion and peripheral blood perfusion during sleep, and indicate the quantitative physiological change process of sleep influencing disease treatment and health and longevity; automatically extracting electroencephalographic characteristic indicators which quantitatively describe change of sleep structure and brain blood and peripheral blood perfusion distribution characteristic indicators which quantitatively reflect changes of sleep quality and health condition, by real-time computation of the acquired multi-conduction electroencephalographic wave and multi-conduction blood perfusion wave; the extracting and computing of indicators are real-time, so as to quantitatively describe brain sleep condition and thus reflect physiological and pathological changes of health condition; acquiring the multi-conduction electroencephalographic waves and multi-conduction blood perfusion waves of a human in various conditions by using transducers for acquiring the signal of electroencephalographic wave, brain blood and peripheral blood perfusion wave, and computing and processing the data at real-time through a computer system which receives the same, so as to obtain real-time data of quantitative characteristic indicators; performing real-time data computation with data pre-processing algorithms including waveform recognition algorithm, fuzzy control algorithm, spectrum analysis algorithm, wavelet analysis algorithm, multiple regression algorithm, and calculus algorithm; computing frequency domain power and phase change of the electroencephalographic wave signal under a specific time domain window, and computing multi-variant components of multi-scale wavelet decomposition scale-space under a specific generating function with spectrum analysis algorithm and wavelet analysis algorithm of the electroencephalographic wave data with time window weighting, obtaining converted and inverse-converted cluster of data with inverse transformation algorithm of time domain component reconstruction, and obtaining a cluster of wavelet discrete values after processing the converted and inverse-converted cluster of data with fuzzy algorithm and threshold value extraction algorithm; obtaining a series of quantitative electroencephalographic characteristic indicators representing various sleep conditions, after multiple regression algorithm incorporating sleep condition and weighting indexation; obtaining the waveform change-point of multiple-cluster time domain blood perfusion waveforms of the multi-conduction blood perfusion wave with multi-scale wavelet analysis algorithm of the blood perfusion wave data under a specific generating function, and then obtaining the dynamic time domain power and relative speed of the blood perfusion wave with calculus algorithm and fitting computation, so as to produce quantitative characteristic indicators describing brain blood and peripheral blood perfusion signal for various sleep quality and sleep structure during sleep.

## **Embodiment 6:**

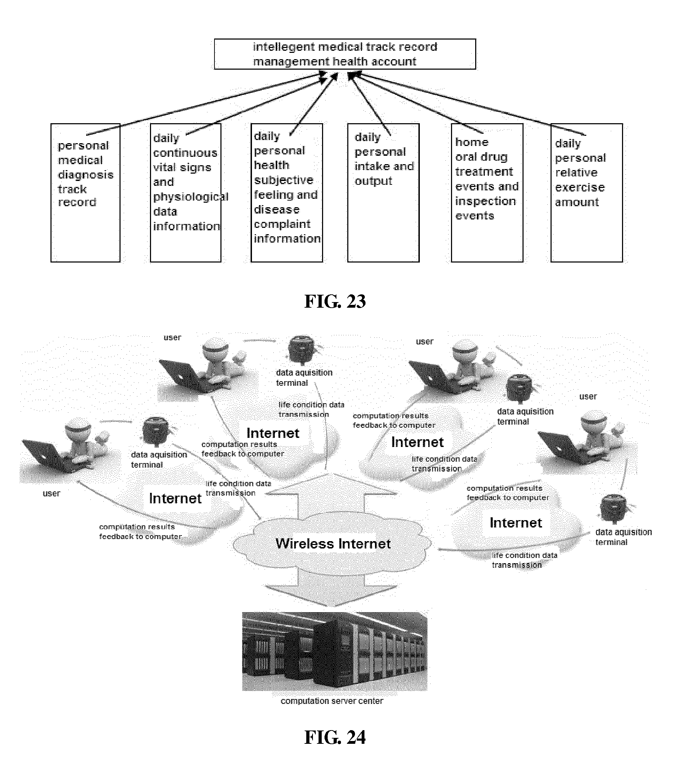

In the present embodiment, there is provided a personal health information platform based on the above-mentioned brain inhibition therapy and the third type of life maintenance mode, with the information structure of personal health bank account as shown in Figure 23, comprising:

obtaining vital signs of a human body, and acquiring medical health data;
decomposing medical clinical inspection items into individual standardized quantitative measurements for life condition, and self-organizing, analyzing and computing life condition information, so as to compose various inspection reports of disease physiological and vital sign data as well as health prediction reports;
establishing a system for real-time quantitative multi-index data extraction for brain condition under the mode of Internet of Things, so as to establish an integrated extracting and expressing method of quantitative characteristic indicators of nerve electrophysiological and blood perfusion signal related to human natural sleep structure and quality;
defining a brain blood perfusion unit and a peripheral blood perfusion unit through measurement of blood perfusion distribution, and establishing a training routine and method to respectively reduce metabolic requirement of the two units upon the relationship between blood flow and metabolism, so as to allow the organs to be able to withstand low blood perfusion level under disease condition;
establishing a new life inspection and maintenance mode as the third type of life maintenance mode for disease treatment, disease rehabilitation, disease prevention and health keeping and life prolonging, so as to control the change of vital sign data upon independent consciousness, in order to achieve the goal of keeping healthy and prolonging life;
introducing continuous quantitative precise measuring and computing of vital sign data and medical health data into the processes of disease treatment, disease rehabilitation, disease prevention and health keeping and life prolonging, changing negative emotion of a person under disease condition into positive emotion, and continuously perceiving the real-time change of outputted quantitative characteristic indicators of vital signs and physiological data, so as to enhance the disease treatment effect and disease rehabilitation effect and achieve the goal of disease prevention and life prolonging, wherein, the data and technique produced during the processes of disease treatment, rehabilitation, disease prevention and health keeping and life prolonging are used as a tool, and based on wireless network communication, the system of aiding or directly driving the processes of disease treatment, rehabilitation, disease prevention and health keeping and life prolonging for people is established.

Furthermore, establishing a media broadcasting system for demonstration pioneers of health keeping, life prolonging, disease treatment and disease rehabilitation, broadcasting treatment track record and vital sign data of the pioneers at regular time, so as to provide a demonstration example for quantitative health data comparison, learning and imitation of the processes of disease treatment, disease rehabilitation and health keeping and life prolonging for people through real-time media transmission. The platform comprises a system software platform and hardware devices including a wireless mobile terminal for vital sign data acquisition and transmission, a data computation service center server and a data storage center server, a mobile computer terminal or a stationary computer, and a mobile communication terminal. The platform adopts an internet communication platform as a communication transmission media, and the users are allowed to register their own application accounts with the health bank website and bind the accounts with their personal identification information; a wireless mobile terminal for vital sign data acquisition and transmission is used by each user in living and working environment, each terminal is bound with the application account of the user, informations of vital signs, subjective feeling and medicine intake events of the user are automatically acquired in daily usage through the wireless mobile terminal for vital sign data acquisition and transmission and automatically transmitted to a data computation center server and an account storage center server on the internet through wireless means; the transmitted data chain is bound with the user's personal identification information and automatically stored into a database corresponding to the application account which is established by the user for permanent storage and timely presentation; after receipt of the data, the data computation center server performs automatic real-time computation and analysis of massive data with cloud computing, various individual life condition characteristic indicators are computed respectively upon the principle of elaborating various indicators in an inspection and analysis report required for disease diagnosis, the characteristic indicators are used to form an inspection and analysis report which meets the corresponding disease diagnosis standards of a medical institution, the report is transmitted to the account storage server and stored in the user's application account for timely presentation. The aforementioned platform further comprises the extraction process of multiple characteristic indicators of brain condition, wherein, the original data of multiple characteristic indicators of brain condition is derived from real-time electroencephalographic wave data of the wireless mobile terminal for vital sign data acquisition and transmission, and automatically mathematically processed and computed by the computation service center to obtain multiple quantitative numeric indexes representing various brain conditions, and an effective quantitative cerebrate habit is able to be established by using objective quantitative real-time characteristic indexes of brain condition, so as to learn the method and skill of quantitatively controlling the brain condition. This platform further comprises the extraction of characteristic indicators of blood perfusion distribution, wherein, the original data of characteristic indicators of blood perfusion distribution is derived from real-time blood perfusion wave of the wireless mobile terminal for vital sign data acquisition and transmission, and automatically continuously mathematically processed and computed by the computation service center to obtain quantitative numeric indexes representing blood perfusion distribution at various positions, the blood perfusion is required by human body metabolism, and reducing of local metabolic requirement is able to be learned by training of reducing the blood perfusion and withstanding of the reduced blood perfusion, so as to establish a basic environment for human body organ protection and low perfusion withstanding in disease condition, as well as rehabilitation and life prolonging.

There is provided a personal medical health electronic data storage platform which combines personal medical diagnosis information, daily continuous vital signs and physiological data information, daily personal health subjective feeling and disease complaint information, intake and output, and personal home oral drug treatment information. The storage and usage of data is man-machine interactive by means of operation interface of internet browser, and medical health bank accounts similar to financial bank accounts and having personal security control are established for storage and access of medical health data. The medical diagnosis information is logged into a personal medical health account manually, the daily continuous vital signs and physiological data is collected by a wireless mobile terminal for vital sign data acquisition and transmission, and transmitted at real-time to the personal account through a wireless internet communication platform. The informations of personal subjective feeling and disease complaint, medicine treatment, food intake, water intake, defecation amount, urine amount, etc. are inputted through an APP application program on a mobile communication terminal and uploaded into the personal account. The medical health information in the account is based on the medical Clinical Information System (CIS) architecture, and comprises disease records, prescriptions, treatments, inspections, images, doctor's advices, and vital signs information. The data of the personal account is presented in forms of numeric value, image, trend curve, text report and real-time wave, and has various data display means such as flexible selection of time window, data combination and data mining. The acquired life condition data is computed and stored after being itemized, so as to compose inspection reports according to the formality and content requirements of a disease diagnosis inspection analysis report, for storage and access. The information of treatment and doctor's advice in the account is automatically sent to a personal communication terminal (a cell phone, a mobile computer, a stationary computer), by means of the connected message communication platform and according to requirement of time, thereby notifying the user to carry out the corresponding treatment process by voice reminder, short message, screen display, etc., and if no confirming reply message is received within a predetermined duration, the account will automatically send a message to a predetermined mobile communication device or stationary computer so as to submit a request for caring, or even an alarm message, towards another person or an institution. The account automatically carries out preliminary health prediction upon medical health information in a period of time, and sends the prediction result to a personal mobile terminal or a computer of a predetermined health service provider for display and reminding at regular time. There is a real-time interaction window configured in the account, so as to facilitate real-time communication and real-time diagnosis between doctor and patient, or between patient and patient, by means of video, text, waveform, numeric value, trend curve, etc. The account is bound with a computer and an intelligent mobile communication device, for accessing and displaying the data content on the two types of devices by means of website browsing.

In the present technical solution, there is established a daily popularized information flow of disease treatment, disease rehabilitation, disease prevention and health keeping and life prolonging which is used as a tool for people's daily health consumption. A pioneer leading mode of multiple disease treatment, disease rehabilitation, disease prevention and life prolonging based on all-time-space data is established by establishing a broadcasting platform for health information of pioneers, and a mode of quantitative elaborate popularized education and self training of medical health knowledge is established. This is characterized in that: based on personal health bank accounts, the complete medical track records and life condition data of certain natural persons are broadcasted at regular time everyday on the mass media (internet, TV, billboard), these persons are selected by medical committee screening and life condition data competition, and their medical track records and life condition data are completely disclosed in a compensated manner. These persons represent the correct treatment processes and correct rehabilitation processes of certain diseases, and include cancer rehabilitees, outstanding chronic disease controllers, outstanding women who have undergone a healthy pregnancy period, as well as elders with longevity over 100 years. They represent correct disease treatment methods, correct disease rehabilitation processes, correct chronic disease management methods, correct living tracks and diets, correct life attitude and emotion change, correct physical exercise and labor tracks, as well as correct disease diagnosis methods and elder care. The real-time life condition data and medical diagnosis data are acquired by means of a wireless mobile terminal for acquisition and transmission of vital signs as well as personal health accounts, and then automatically uploaded to an exhibition window of the mass media so as to be broadcasted to people of the whole country at regular time. These data comprises medical diagnosis information, treatment information, subjective feeling information, exercise amount information, diet information, excretion information, vital signs information, physiological parameter information, and simple home biochemical measurement information. These data is used as a reference content for people to imitate, compare and predict their own disease development and process of health and longevity, so as to establish a living track target of achieving scientific and quantitative correct healthy keeping and life prolonging in natural living and work environment, thereby obtaining and self training the knowledge of disease and health by means of quantitative comparison of vital signs and living data. Thus a self-adapted health management mode in accordance with market-oriented economic behavior is established so as to provide contents for mass media to promote positive energy. The influence modes in fashion business of creating star, pursuing star, Big-V, talent show, etc. are blended into the processes of health keeping and life prolonging as well as disease treatment and rehabilitation. These health demonstration pioneers, recognized as health stars and heath talents, lead people through a scientific and rational process of health and longevity based on quantitative data. Meanwhile, these pioneers also bring about reasonable incomes of fame and fortune for themselves.

**Embodiment 7:**

The embodiment provides a system integrating the schemes of embodiments 1-6 into a whole and aims at providing automatic acquisition and computing of vital sign data relying on an internet platform, real-time interaction and brain inhibition therapy, quantitative characteristic expression of brain status, blood perfusion quantitative characteristic expression of sleep structure, information exchange method of closed-loop independent vital signal data-driven treatment and implementation of longevity quantitative traction.

The invention is based on the thinking that medical behaviors and health care behaviors of people can be considered as a life maintenance mode. Life can be prolonged by utilizing a variety of methods. The current medical and health processes can be categorized and summarized as a western medicine diagnosis and treatment mode and a traditional Chinese medicine diagnosis and treatment mode. As a medical and health mode, the mode must have a perfect scientific theoretical system and a standardized complete operation process including inspection, diagnosis, treatment, rehabilitation and health care. Western medicine is a life maintenance science developed on the basis of anatomy and chemistry, while the traditional Chinese medicine is the life maintenance science developed on the basis of simple traditional culture of yin-yang coordination, balance, nature and allelopathy of substances. The western medicine is a process for intervention of life bodies by utilizing chemical substances and performing surgical treatment. The traditional Chinese medicine is a process for intervention of the life bodies by using plants, acupuncture, massage and the like. These two modes are both the methods for changing the trends of diseases and health by external forces. In the long-term medical development process, people also find that many diseases are related to spirit and psychology of the people and are also related to resistance of the people. It is indicated that an internal mechanism and activation force for treating diseases, improving health and promoting longevity exist in a human body. Of course, the reverse action force for damaging the health and causing diseases and death also exists in the human body. No matter whether the reaction force is good and favorable for the health, or bad and harmful to the health, it occurs unwittingly, the process can not be perceived by people through natural consciousness and awareness, let alone be actively and consciously intervened by the people. It can be concluded that there is a force capable of affecting the diseases and the health in the human body. At present, the force has not been excited and can only be attributed to natural and latent ability, the sixth sense and the like in the scope of metaphysics. That is, there is one half (tentatively scheduled as one half) of force which has not been used for treating the diseases and realizing health keeping and life prolonging, and particularly, the force for preventing diseases has not been used at all. This is one factor causing the situation that the human lifespan can only achieve about 4 times the development cycle, while the animal lifespan can achieve 7 times the development cycle.

Senior cognition of human cortex has learning ability, simultaneously has forgetting ability and tends to be lazy. The senior cognition emotional function of cerebral cortex is the difference between human beings and animals and is obtained after long-term evolution. It can be said that the cortical function consumes life resources, thereby becoming a factor shortening human lifespan. The correct lifestyle and the correct scientific use of the brain play a crucial role in realizing the health keeping and life prolonging of the people. If the senior cortex cognition function turns to have the effect of positively promoting the health keeping and life prolonging, the human lifespan can be greatly prolonged to the normal life cycle of 100-150 years old. Blood distribution and respiration are the factors determining that the life body obtains energy of oxygen component, which are affected and even indirectly controlled by the cerebral senior cortex function. In the life process, some diseases were originally controlled by the brain directly. Taking sleep as an example, people can sleep after birth and can fall into sleep immediately when feeling sleepy in the adolescence particularly. The reason causing insomnia, as well as anxiety and depression is that people are mainly subjected to corresponding external stimuli and must sacrifice the sleep to complete some cognition tasks, which may be accompanied by positive and negative emotional processes, for one part of people, if the process is accompanied by negative emotions, the brain will forget how to sleep, this process can be very short, and insomnia may be formed after several days. Drug dependence is another example. It's an instinctive process for the brain to secrete enkephalin. However, when external substances like opioids enter into the brain, the brain soon forgets the ability of secreting enkephalin and a pleasant mood is created at the same time, so that the brain falls into the habit of continuously requiring such substances from the outside and the drug dependence is formed. The detoxification process is a reverse process, the pleasant memories of the brain about the external substances are inhibited through the painful negative emotional process, and then the brain again learns how to secrete enkephalin. It can be seen that it is possible to enable the brain to re-learn how to sleep, or enable the brain to learn the ability which has been forgotten and even learn the ability to realize health keeping and life prolonging which is unavailable before by utilizing the pleasant emotions.

A third life maintenance mode which is in parallel to the western medicine diagnosis and treatment mode and the traditional Chinese medicine diagnosis and treatment mode is created, and the mode can excite and use the force in the human body as a method for disease treatment, disease rehabilitation, disease prevention, health keeping and life prolonging and is called as a life maintenance mode. With establishing a set of theoretical system with inheritance, innovation and development as the core, the third life maintenance mode is inherited from a traditional medicine clinical system based on the modern communication technology, internet technology, medical clinical information technology, digital signal processing technology, computational mathematical technology, artificial intelligence technology, automation technology, biomedical engineering technology and electronic technology. This mode freely controls the change of vital sign data upon independent consciousness by establishing perception channels among the cerebral senior cortex nerve center, the deep cerebral nerve center and the plant nerve center and taking the cognition of the cortex function and the emotional control as the means according to sustained and quantitative measurement and computing of the vital sign data, in order to achieve the final goal of treating diseases, keeping in good health and prolonging life.

The effects of the current conventional treatment method can be maximized by creating a background environment for treating the diseases. The background environment for treating the diseases can affect the disease treatment effect, and by using the refined background control means, the disease treatment process can be enriched and improved. The background control mainly takes the inhibition and dormancy of the cerebral senior cortex cognition function as the means to realize the background control.

With the rapid development of the modern scientific technologies and the increasing integration with the traditional medicine, conditions are provided for revolutionary development of the medical technologies and modes. By changing the isolated medical treatment way after the obvious feeling of the change of life status to the sustained way of being in a medical treatment and health care process when the change of the life status has not been obviously felt, the innovation mode of the standardized diagnosis and treatment process of the third life maintenance mode is established, wherein for most of the time, the process is of a medical grade, including inspection and measurement required for diagnosis, diagnosis process, treatment, rehabilitation, sub-health control and other sustained life process managements. All the links feature inheritance, development and innovation in comparison with the current diagnosis and treatment modes. For the diagnosis link, firstly, the inspection items required for clinical medical diagnosis are split in a refined manner to become the simplified operable vital sign data computing contents for home use, become the routine repetitive data acquisition and measurement items and can be combined to inspection analysis reports for disease diagnosis and prediction of diseases and health status. The innovation mode and the standardized contents of the inspection and measurement links for diagnosis are established. Secondly, a vital sign data acquisition and computation technology and a method which are sustained and can be applied to the disease inspection and measurement link in a home environment is used to establish a way of realizing the disease treatment, rehabilitation, health keeping and life prolonging by controlling the change of the vital sign data by the senior cortex function upon the independent consciousness, thereby forming the core innovation contents of the third life maintenance mode. The implementation of such innovation is similar to the contribution of anatomy and chemistry to the establishment of western medicine, and as a support platform, wireless communication, internet, computational mathematics, biomedical engineering and big data technologies in the modern scientific technologies serve as the foundation and guarantee of the implementation. By the adoption of the wireless internet communication system, the way of realizing the disease treatment, rehabilitation, health keeping and life prolonging by controlling the change of the vital sign data by the senior cortex function upon the independent consciousness is a regulation mechanism of a closed-loop feedback steady-state system in a cybernetic system, and a model algorithm for precise and quantitative data regulation in the cybernetic system is introduced to form a diagnosis and treatment health mode under a large closed-loop and big data artificial intelligence architecture. Being similar to the standardized and quantitative process in the cybernetic system, the standardized and quantitative process and method for realizing disease treatment, disease prevention, disease rehabilitation, health keeping and life prolonging can be established. The treatment and rehabilitation process adopts the application forms of entertainment and sports competitions, and by using the innate ability of learning of the brain, the cerebral cortex learns the skill of consciously regulating and controlling the vital sign data in the process of continuously and repeatedly trying to control the change of the vital sign data.

The implementation of the technology is based on wireless internet communication and hardware carriers. By taking a wireless mobile vital sign information acquisition terminal as a tool, taking automatic extraction of characteristic indicators of the vital sign data as the core, taking automatic computing and generation of medical inspection result reports as the basis, taking a big data cloud computing architecture as the support, taking tank-like deposit and withdrawal of the vital sign data and diagnosis and treatment data as a backstage, taking home-based sustained vital sign data acquisition and transmission as the representations, taking online real-time interaction between doctors and patients as a method, taking the processes of games and sports competitions as the means, a complete third life maintenance mode including the base, the tool, the core, the basis, the support, the backstage, the representations, the method and the means and in parallel to the traditional medical service modes is created for health keeping and life prolonging of the people, and the third life maintenance mode has realistic feasibility, service refinement, process scientization, technology inheritance, use economy and other powerful applicability, effectiveness and innovation. A whole set of brand new diagnosis and treatment service auxiliary method for disease prevention, disease diagnosis and treatment is created, opening a new door full of fun and excitement for the requirements of disease prevention, treatment, rehabilitation, health keeping and life prolonging of the people.

The core concept of the invention is to establish the third life maintenance mode, wherein the clinical diagnosis and inspection which can only be made after the disease occurrence are decomposed into a series of single automatic measurement and computing items of the vital sign data, popularized to daily life and work of the people and developed continuously to form the continuous diagnosis and inspection analysis report and trend; the vital sign data which is automatically measured and computed is taken as the tool, and the active control of the change of the vital sign data (the change of the use of the tool) by the cerebral cortex senior independent consciousness is taken as the means for treatment, rehabilitation, health keeping and life prolonging; the occurrence and development of the diseases are predicted and prevented based on the formed inspection analysis report and trend; and short messages are automatically sent to remind a patient at home to check the treatment behaviors, based on doctor's advices and prescriptions in the track record of medical treatment, and an automatic no-response alarming service is provided.

The hardware system comprises a vital sign information acquisition sensor, a wireless mobile vital sign information acquisition and transmission terminal, a subjective feeling information acquisition device, a family diagnosis and treatment health information prompt working station, a vital sign data perceptual learning control device, a corresponding data storage server, a data computing server and other parts to complete the acquisition of vital sign data, medical treatment track record and subjective feeling information, transmission and communication of the information, control of disease treatment and rehabilitation and other tasks. The software platform plays a role in managing the information exchange process of the medical and health information under an internet platform, and the platform exists as a mutually integrated and relatively independent modular system and comprises a health bank website system, a vital sign data computing system, an individual home inspection and treatment information prompt and alarm management system, a series inspection report generation system, a vital sign data characteristic indicator automatic extraction system, a cloud computing and automatic distribution management system, a single vital sign data computing service management system, a self-adaptive vital sign data perception closed-loop feedback system, a data broadcast information management system and other systems. The application mode comprises a health information consumption mode of prize sports competitions for disease treatment, disease rehabilitation, health keeping and life prolonging by using the computation results of the vital sign data and the process data of disease treatment and chronic disease control, an information consumption mode which integrates the process of disease treatment, disease rehabilitation, health keeping and life prolonging into individual games and three media propagation application modes of data publication template application of demonstration pioneers of disease treatment, rehabilitation, health keeping and life prolonging.

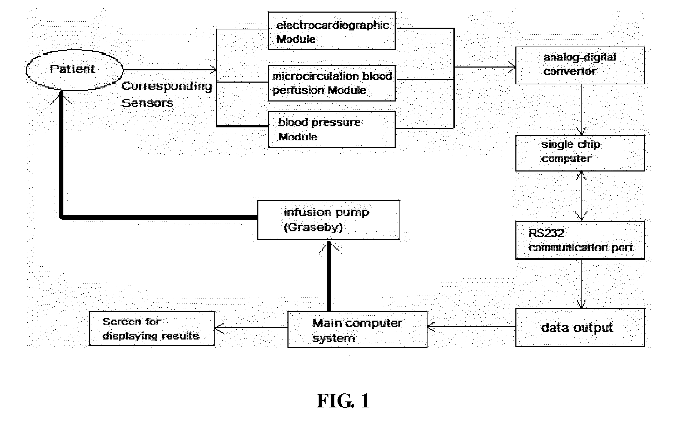

The wireless mobile vital sign data acquisition and transmission terminal comprises three units. The first unit is a physiological and vital sign signal acquisition transducer, the second unit is a vital sign information acquisition and transmission terminal host, and the third unit comprises a handheld communication terminal or a website input interface of a mobile or fixed computer. Vital sign and physiological data acquired by the acquisition transducer comprises waveform signals: electroencephalographic wave, cerebral and peripheral blood perfusion wave, penis blood perfusion wave, electrocardiographic wave, pulse oximetry wave, mouth-nose-chest-abdominal respiration wave, electromyographic wave, eye movement wave and breath-end carbon-dioxide concentration wave; numerical signals: pulse rate, non-invasive blood pressure, blood glucose, pulse oxygen saturation, breath-end carbon-dioxide concentration, inhaled oxygen concentration, body temperature, respiratory rate, penis cross-section diameter, penis hardness, penis temperature, penis arterial oxygen saturation, urine volume, relative exercise amount of limbs (upper and down limbs), body weight and exercise sweat amount; the acquired subjective feeling data: pain rating scale, emesis rating scale, dizziness rating scale, living quality rating scale, chest distress, urination frequency, urine color, hemorrhage, diarrhea, constipation, excrement color, palpitation, shortness of breath, impotence, vertigo, asthma, pyrexia, dysphoria, anger, anxiety rating scale, depression rating scale, difficulty of falling asleep, dysphylaxia and dreaminess. The basic circuits are as shown in Fig. 1-Fig. 15.

The terminal host completes amplification, shaping, conversion, reception, encryption, packaging, communication and other work of the physiological signals and vital sign proof signals, wherein the terminal host comprises a monitoring circuit which includes a signal conversion circuit, a signal amplification circuit, a filtering circuit and other signal shaping and amplification circuits, as well as a central computation and control management circuit, a communication interface circuit, a dynamic data link cache circuit, a wireless internet access control and automatic sub-packaging and uploading circuit, a power supply circuit and other parts; the connection relationship of the parts is as follows: corresponding vital sign and physiological signals of the human body are converted to electric signals and computer numerical signals by a probe of the transducer of electroencephalographic, blood perfusion, electrocardiographic, electroencephalographic, blood pressure, blood oxygen, respiratory, body temperature and other physiological waveform signals and the vital sign numerical signals of the human body, the electric signals are transmitted to a filtering module, a noise control module and an amplification and input module of the monitoring circuit, and are transmitted to an analog-digital convertor circuit of the central computation and control management circuit through an analog signal input port of the module after being computed and processed by corresponding monitoring modules (HXD_I), and the vital sign numerical signals are transmitted to a communication interface of the central computation and control management circuit through an RS232 interface circuit; the central computation and control management circuit encrypts and compresses the digitalized signal data received by the central computation and control management circuit to obtain a processed data stream, which is then fed into a dynamic data link cache queue; the dynamic data link cache queue is a varying data storage and output configuration, and the data configuration in a storage region is determined by network status; after an interruption event is triggered due to the fact that the central computation and control management part is notified of the change of network status, the central computation and control management circuit controls various permutations and combinations of the obtained data; the data is then written into a data buffer of the storage queue upon a write instruction; the data in the queue is output to a wireless internet access control circuit through a data port upon a read instruction of the computation and control management circuit; and the internet access control circuit carries out auto-dial to the network, network status recognition, TCP/IP mode signal modulation, sub-packaging and outputting, so as to allow the data to be uploaded to the data storage server and data computation server; and
the monitoring modules in the system adopt Huaxiang multifunctional combined monitoring devices (HXD_I) with registration and sales certificate. The multifunctional modules further comprise monitoring of conventional vital signs and brain function indicators and secondary computation of the conventional vital signs, thereby further obtaining multiple function monitoring indexes.

The data acquisition, including the data acquisition of the vital signs, is completed in different control units respectively in a distributed computing and processing mode, and the obtained data is integrated into the computation and control unit in a cyclic binary queue structure. The central computation and control management circuit mixes the name structure of the host in the network with the standard data structure and the uniform data frequency, and the name structure of the terminal is as follows:

$$\text{The address of the terminal machine} = \text{unique address number in the network range} + \text{fixed IP}$$

$$\text{address of the network server}$$

The maximum number of machines in the network = 65535

Encryption treatment and compression are performed on the data. An integrated data stream enters into the link storage queue. The data window is of 8 betys.

$$\text{L stream window} = \text{wavelet } (m1+m2+m3+m4+addr+asyn+data1+data2)$$

m1, m2, m3 and m4 are the data transmitted by the module, asyn is synchronization and data is data and encrypted packets.

Data sending, status reception and command reception of the system are fundamental guarantee of practicality of the system and also become one of the key links for safe use of the system. GPRS and CDMA are not stable internet access technologies in the mobile technologies. The data transmission results can be affected by many links. For the transmission of the numerical data, UDP packets can be used. By adopting a way of sending repeatedly, it is ensured that the server can receive one-time effective data entities. For the transmission of waveform images, continuity and exclusivity are required. The data can not be lost. This is the difficult point of the technology. Encryption and compression of the data is another difficult point of the waveform transmission. The transmission of the waveform images must adopt TCP packets. The GPRS access status and the internal cache size can be processed in real time by using the stream control ability of a single-chip computer. The real-time process of network communication is automatically recognized and self-corrected, thereby controlling the direction of the data stream and internet dial-up calls. The data link dynamic queue area which is as long as six minutes is set. The integrity of the data and the improvement of fault-tolerant ability are ensured.

The data waits for network access in the link storage queue. A network status triggering circuit triggers a network status event, and the event processing processes of the computation and control unit enter into different thread processing units respectively according to the natures of the events. Under the normal state, the link data is obtained, sub-packaging is performed according to TCP protocol, and the protocol packets are sent to a network address and a port which are set in the network through the wireless network.

$$\text{TCP protocol packets} \mathrel{+}= (\text{save\_point-read\_point}) * \text{real\_data} \rightarrow \text{read\_point}++$$

save_point and read_point are data pointers and real_data is real-time data.

Under an abnormal state, the system starts the processing process of waiting for a dialing thread, the network access status recognition circuit tries the network access situation in a timing manner, and simultaneously, a data cache event is triggered and the data structure is reorganized. Whether the network server is busy or network disconnection occurs is determined by network status recognition, different messages are triggered, and a re-dialing circuit is started or the communication with the server thread is tried (see Fig. 2).

$$\text{L stream window} = (m1+m1+m1+m1+addr+asyn+data)$$

m1 is key data for transmission, asyn is synchronization and data is encrypted packets.

The dynamic data link management comprises memory data queue management, data encryption management, data compression management, dynamic change of data structure and reorganization of data stream. The functions of dynamic mutual coordination sent by review data and current data and fast transmission of the key data are covered.

The data transmission of the system adopts public network and wireless modes, and the data for transmission must be the encrypted data. The system does not adopt a conventional data encryption technology. A dynamic data encryption method with independent intellectual property rights: a wavelet analysis data encryption technology is provided. The consideration basis is complexity and fineness, decomposition and reduction properties and powerful anti-interference ability of the wavelet analysis technology. The hardware design of the system fully considers the implementation of the technology. The data format is extended to eight-channel data transmission ability. Wavelet transform and indexation wavelet analysis results of specific data segments are preset. Wherein a generating function and resolution of data transformation are secret, and channel numbers of all compositional data are disordered. At the receiving end, inverse transformation is performed on the signals, so that the original properties of the data can be restored. By applying the technology, the interference of the signals can be eliminated. According to the application of the wavelet analysis technology, the signals can also be processed at the receiving end to extract some characteristic indicators of a variety of signals so as to be conveniently used as a data foundation of long-range feedback regulation and control. The application of the technology is the basis for practicality of the system.

The acquisition of living condition data and subjective feeling data is realized by the subjective feeling information acquisition device. The subjective feeling information acquisition device comprises a computerized equipment, such as a cell phone, a mobile tablet, a stationary computer and the like. Under the personal health bank account expressed in the invention, with a browser window of B/S configuration or an operation window of APP software as the input interface, the data is logged by manual input, and data items related to different feelings or parameters are selected by click-choice on the input interface, and the data items comprise pain rating scale, emesis rating scale, dizziness rating scale, living quality rating scale, chest distress, urination frequency, urine color, hemorrhage, diarrhea, constipation, excrement color, palpitation, shortness of breath, impotence, vertigo, asthma, pyrexia, dysphoria, anger, anxiety rating scale, depression rating scale, difficulty of falling asleep, dysphylaxia, dreaminess and other phenomena and feelings of daily behaviors. The data is uploaded to the data storage and computation server of the personal health bank account directly through wireless or wired internet access of the computerized equipment. The subjective information generates the contents of chief complaints of the patient of medical and health service, stores and records the contents; and simultaneously, the changes of the corresponding vital sign and physiological signals are synchronously recorded to realize the comprehensive acquisition of the health and medical information in life and work.

The integrated signal acquisition transducer of electroencephalographic wave and cerebral blood perfusion wave continuously acquires electroencephalographic wave on both sides of the forehead and supraorbital artery blood perfusion wave on both sides of the forehead. The sensor is divided into a plurality of transducer combinations integrated in the range of a silica gel body of $6\times0.7\times0.3$cm. The silica gel body is called as a silica gel bed. Each transducer adopts an industrial grade signal sensor. Each transducer comprises a group of pressure sensors, a group of near-infrared laser generators, a near-infrared laser receiver and a group of metal silver electrode pieces after oxidation treatment. Metal electrodes sense the electroencephalographic wave signals, the pressure sensors and the near-infrared laser sense the blood perfusion signals, and the metal electrodes are divided into two electroencephalographic signal guiding electrodes at the supraorbital part on the left side and the supraorbital part on the right side, and two electroencephalographic signal reference electrodes at earlobe parts of the double ears. After the combination, the electroencephalographic signals of left and right brains are acquired respectively, and the electrodes are designed to be subjected to physical and chemical processing, so that the electrodes meet the application requirements of acquisition of the electroencephalographic signals by dry electrodes in the aspects of shape and electric potential polarization, and the electroencephalographic signals which are in line with the medical standard can be conveniently acquired without performing professional processing on the skin in the vicinity of signal acquisition points. The pressure sensor and the near-infrared laser sensor are integrated in the vicinity of supraorbital artery anatomical positions on the two sides in the supraorbital positions near the middle part respectively, the pressure sensor and the light emitter at the parts on the left side and the right side select two groups of devices respectively and the light receiver selects a group of devices respectively. Under the using requirements of personal simple wearing, the reliable irradiation of the emitted light to a vascular part can be ensured and the pressure sensors can sense vascular pulse signals. The pressure sensor, the laser sensor and the electroencephalographic wave sensor on the two sides of the forehead are fixed on the silica gel bed and arranged from the left to the right according to the above rules, and the electroencephalographic guiding electrodes at the ear parts are fixed on two ear clips and form a whole with the silica gel body through conducting wires. The silica gel body is connected with an elastic band to form an elastic head cover capable of regulating the diameter. The conducting wire of the silica gel body is led out to the middle part of the elastic band, then goes down along the brain part and is matched and connected with a plug to obtain a complete head electroencephalographic and blood perfusion signal transducer.

The peripheral blood perfusion wave transducer integrates a set of near-infrared laser sensor, including a light receiving and transmitting device, and the sensor is fixed on the silica gel body of $2.5\times0.7\times0.3$cm to acquire blood perfusion wave signals at the finger part. The silica gel body is connected with a tear strip, and when in use, the silica gel body is wrapped at the tail end of the finger and fixed with the tear strip. Thus, the peripheral blood perfusion wave signal transducer is obtained. The signal conducting wire is connected with the head blood perfusion and electroencephalographic wave signal transducer and connected into a corresponding socket of the vital sign signal acquisition and transmission terminal through the plug. The outside view, the signal flow and the circuit diagram of the sensor are as shown in Fig. 3-Fig. 5.

The near-infrared laser sensor irradiates the blood stream flowing in the blood vessel, a reflective wave changes as the number of erythrocytes carried by the blood stream in the blood vessel changes, the blood stream simultaneously exerts a force onto the blood vessel wall so as to form a pulse, and the pressure sensor can sense the pulse. The maximum pulse of the blood vessel can be sensed by the pressure sensor, so that the positions of the supraorbital arteries can be found approximately; and the near-infrared laser emitter in the near-infrared laser sensor can irradiate to the blood vessel and produce the reflective wave, and the laser receiver arranged on one side receives a reflective signal as output. Simultaneously, the pressure transducer arranged at the part of the blood vessel receives the pulse wave of the blood vessel wall as an output signal. The two signals are output, converted to recognizable analog electrical signals by the electronic signal conversion and amplification unit of the monitoring circuit of the vital sign signal acquisition and transmission terminal, transmitted into the analog-digital convertor, and discretized and converted into the digital signals, and enter into the central computation and control circuit chip to complete the data processing process including distribution, computation, encryption, compression and binding of identity information. The processed data stream is obtained, fed into the dynamic data link cache queue and sent to the internet platform in a wireless manner, thereby obtaining the real-time blood perfusion wave at the server end. Similarly, the real-time peripheral blood perfusion wave can also be obtained at the server end. The electroencephalographic wave sensor senses the change of biopotential produced by polarization reaction of charged ions of the cerebral cortex and forms potential difference signals between the guiding electrodes and the reference electrodes as the output; the signals are converted to recognizable analog potential signals by the electronic signal conversion and amplification unit of the monitoring circuit of the vital sign signal acquisition and transmission terminal, transmitted into the analog-digital convertor, discretized and converted into the digital signals, and processed by the central computation and control circuit chip; and being similar to the blood perfusion wave, wherein the real-time electroencephalographic wave data is obtained at the server end.

The transducer integrating penis blood perfusion wave, penis cross-section diameter, penis hardness, penis temperature and penis arterial oxygen saturation into a whole acquires penis physiological change status signals in sleep, and the transducer device has the function of continuously acquiring the physiological parameters, such as change of penis local blood stream, change of hardness, change of cross-section diameter, change of temperature, change of relative blood oxygen and the like. The sensor is divided into a plurality of transducer combinations integrated in the range of the silica gel body of $5\times0.7\times0.35$cm. Each transducer adopts the industrial grade signal sensor. Each transducer comprises the light generator, the light receiver, the pressure sensor, the temperature sensor and the metal electrode piece. The sensors are arranged according to certain rules, and the conducting wires and the plugs are led out to complete the function of transduction and output of the physiological signals. Each sensor is of a strip-like structure, the middle part of each sensor has an elastic substance, and one end of each sensor is connected with a medical band aid and each sensor is sheathed at the root part of the penis in an annular way. A plurality of conducting wires are led out of each sensor, the multiple stands are interwined, and a joint is configured at one end for connecting to the input end of a preamplifier. The amplifier comprises a blood perfusion preamplifier, a hardness and cross-section diameter amplifier, a blood oxygen saturation module, a temperature input interface and all parts of amplifiers for pre-filtering, first-stage amplification, zero-point regulation, active filtering, time constant, signal isolation and second-stage amplification and output. The signals are converted to voltage signals of 0-5V. The signals represent the change of penis blood stream, change of penis hardness, change of penis cross-section diameter, change of penis temperature, change of penis relative blood oxygen saturation and other parameters. The blood oxygen saturation module adopts a commercially available medical device, namely a standard blood oxygen module, which is formed by transforming a blood oxygen monitoring probe. The determination of the penis relatively blood oxygen is realized. The penis cross-section diameter measurement needs to use the zero-point regulation, and after the sensor is mounted onto the penis, the tightness takes the flash of a zero-point indicator of the wireless mobile vital sign signal acquisition and transmission terminal as a mark. The signals after amplification and shaping treatment are converted to the digital signals after analog-digital conversion and fed into the central computation and control circuit chip to complete the data processing process including distribution, computation, encryption, compression and binding of identity information. The processed data stream is obtained, fed into the dynamic data link cache queue and sent to the internet platform in the wireless manner, thereby obtaining the real-time penis blood perfusion, penis cross-section diameter, hardness, temperature, relative oxygen saturation and other waveforms and numerical values at the server end. The penis hardness, the penis blood stream and the penis cross-section diameter are as shown in Fig. 6-Fig. 8.

The signal transducers for electrocardiographic wave, pulse oximetry wave, mouth-nose-chest-abdominal respiration wave, electromyographic wave, eye movement wave, breath-end carbon-dioxide concentration wave, pulse rate, non-invasive blood pressure, blood glucose, pulse oxygen saturation, breath-end carbon-dioxide concentration, inhaled oxygen concentration, body temperature, respiratory rate and the like can be implemented by the commercially available special functional modules, which are mainly based on the various functional modules of the monitors with HXD_I series multi-parameter combinations and are matched with the products of international and domestic corresponding module suppliers to realize the acquisition of the physiological and vital sign signals. The output signals of the various functional modules comprise two modes of waveform output and digital output, which are fed into the analog signal input end and the digital communication interface end of the central computation and control management circuit respectively. The signal communication is implemented by mixing wired and wireless ways, the wireless way is implemented by adopting the functional modules with a bluetooth or wifi mode, the functional modules mainly comprise a blood glucose minimally invasive measurement box and a respiratory component measurement box, the two parts of measurement units and the wireless mobile vital sign signal acquisition and transmission terminal can be split and are connected in the wireless way, and other functional modules are included in the terminal device and are connected in the wired way. The output signals of the various functional modules are converted to the discretized digital signals by the analog-digital converter of the wireless mobile vital sign signal acquisition and transmission terminal, enter into the central computation and control circuit chip and are subjected to the similar treatment and processing. Then, the processed data stream is obtained, fed into the dynamic data link cache queue and sent to the internet platform in the wireless manner, thereby obtaining the real-time waveforms and the numerical values of the various vital sign and physiological signals at the server end. The circuits of the various parts are as shown in Fig. 9-Fig. 14.

The signal acquisition transducers for the relative exercise amount of the limbs (the upper and lower limbs) are implemented by adopting acceleration sensors and are fixed at the four limbs of the body in a wearing way. The four acceleration sensors are divided into the upper part and the lower part and are fixed on a coat and trousers respectively in the wired way. Four signal lines are connected onto a hub of $4\times1\times2.5$cm, and the signal lines are converged at the part of the waist of the trousers and connected onto the hub in the way of being fixed onto the waist of the trousers. The hub comprises a signal output line connected onto the corresponding socket of the wireless mobile vital sign signal acquisition and transmission terminal, thereby realizing the function of the signal transducer for the relative exercise amount of the limbs. The limb exercise acceleration signals are alternating current signals which are fed into the part of the monitoring circuit of the terminal, become the discretized signals by analog-digital conversion after amplification, shaping and other treatments of the analog signals, enter into the central computation and control management circuit and are transmitted to the internet platform after digital treatment, so that the waveform signals of the relative exercise amount of the limbs are obtained at the server end. The acquisition circuit for the signals of the relative exercise amount of the limbs is as shown in Fig. 15.

The acquisition transducers for urine volume, body weight and exercise sweat amount are implemented by adopting digital weighing instruments, and the measured weight signals are transmitted into the wireless mobile vital sign signal acquisition and transmission terminal in the wireless way and are further transmitted to the server end of the health bank in the present invention. The wireless transmission of the weight signals is implemented by adopting a mode of a bluetooth technology or a wifi technology. The acquisition circuit for weight information is as shown in Fig. 16.

The family diagnosis and treatment health information prompt working station is a tabletop computer system used in a family and can also adopt a home computer system, a smart mobile phone and the like; and through the computer, in the situation of obtaining the authorization (purchasing the service), the computer emits sound and screen text information according to the instruction conveyed from the server and guides the user to complete the various steps of corresponding disease treatment, rehabilitation, health keeping and life prolonging. If the mobile phone is used as the equipment at the prompt end, the sound and the screen text information are mainly emitted in the form of short messages. The instruction of the server is generated according to the requirements of doctor's advices and prescriptions. The input of the medical treatment information of the personal health bank account in the invention comprises the doctor's advices and prescriptions, extracts the contents of medicine dosage and measurement in the prescriptions, generates a treatment instruction sequence according to the time stipulated in the prescriptions and sends onto the designated personal home computer or the mobile phone in a timing manner.

The vital sign data perceptual learning control device comprises two parts, namely a motion control tablet computer and an automatic robot. The tablet computer receives the computation results of the vital sign data computation and analysis server included in the invention through the wireless internet platform, emits a motion control instruction of the robot according to the computation results and controls the actions of the robot. The robot adopts a programmable and assemble modular robot product EV3 imported from the U.S. and comprises a group of action instruction systems. The bluetooth mode is adopted for communication between the robot and the control computer. Information exchange adopts a man-machine combination closed-loop mode under the architecture of the large closed-loop feedback control system. The control loop comprises the human physiological and vital sign signals, the wireless internet platform, the signal computation server, the control computer, an execution mechanism (robot) and a human sensory input system. The signal flow is as follows: the physiological and vital sign signals of the user are acquired through the wireless mobile vital sign signal acquisition and transmission terminal in real time and fed onto the signal computation and analysis server on the network through the wireless internet platform, the real-time characteristic indicators reflecting the life status of the people in real time are obtained through the cloud computing mode, the signals are directly transmitted into the control computer through the wireless internet platform, the computer emits different motion control instructions of the execution mechanism according to the characteristic indicators and the purposes of disease treatment or health care and cerebration literacy set by the user, the execution mechanism receives the instruction and then produces the corresponding motion forms, and after human eyes and ears see and hear the motion of the execution mechanism (being equivalent to perception of the change of the physiological and vital sign signals of the user), the motion of the execution mechanism is promoted by regulating the change of the physiological and vital sign signals of the user to achieve the desired state of the user, thereby forming the closed-loop feedback control mode of human, internet, execution mechanism and human beings. The control flow is as shown in Fig. 25.

The integrated signal acquisition transducer of electroencephalographic wave and cerebral blood perfusion wave continuously acquires electroencephalographic wave on both sides of the forehead and supraorbital artery blood perfusion wave on both sides of the forehead, as well as peripheral blood perfusion wave of the limbs. The multiple channels of waveform data are transmitted to a data computation service center and a data storage center through the internet wireless mode. The server of the data computation service center exerts cloud computation treatment on the various channels of the waveform data and extracts the indicators representing sleep characteristics. The supraorbital arteries of the forehead are from vascular branches and fundus arteries are from the branches of intracranial arterial blood vessels. The supraorbital artery blood perfusion is related to the intracranial blood perfusion. The intracranial blood perfusion is a fundamental factor for maintaining the brain functions and is also the fundamental factor affecting the brain status. The work of the cerebral cortex can be represented by the change of the cortical blood perfusion, and a modern brain scanning and measuring technology is mainly used for observing the change of blood stream images in the various functional regions of the brain. Medical studies prove that the blood volume of cerebral blood perfusion accounts for about 30% of the total blood volume of the body. It can be said that the work of the cerebral cortex consumes a lot of oxygen component and other endocrine substances carried in the blood. The main actions of blood perfusion are that the oxygen component is carried to realize systemic circulation and oxygen molecules and carbon dioxide molecules are continuously exchanged among the cells of various organs. The oxygen molecules are main components for completing the energy supply of the metabolic function of the cells and producing the oxidation reaction. Carbon dioxide is a subsidiary product of the oxidation reaction, reaches the lung of the human body by blood stream transportation and is exchanged to the outside by respiration. The measured cerebral blood perfusion, in particular the characteristic indicators which are measured by extracranial blood vessels and are related to the intracranial blood perfusion, can objectively reflect the change of the cerebral cortex function status. Experiments show that the blood perfusion of the brain changes in the sleep state, the blood perfusion of the body also changes, and the change can be reflected by measuring the blood perfusion. The measurement of the blood perfusion comprises the measurement of the cerebral blood perfusion and the measurement of the peripheral blood perfusion of the limbs, and the measurement results are two indicators, namely the cerebral blood perfusion index and the peripheral blood perfusion index, which are named as the HPi index and the Pi index respectively. The two indexes are vital sign output signals emitted by the body and represent the change of the blood perfusion at the two parts in the sleep state, and in turn, these indexes also directly represent the change of the sleep structure and the sleep quality, which are quantitative and objective expression of the measurement of the sleep. The expression way of the sleep structure represents the breakthrough of directly reflecting the sleep quality by the physiological data.

HPi and Pi are obtained by computing the supraorbital artery blood perfusion wave of the forehead and the peripheral blood perfusion of the limbs, and the computation adopts a composite algorithm, mode recognition, waveform recognition, calculus and a wavelet algorithm.

For the acquired blood perfusion signal wave, discretization treatment with a sampling frequency of 500/s, a sampling time window of 1.25s and a sampling precision of 10-bit bite is performed to generate a vector group of the various lead waveform signals:

$$Xi=[x1\ x2\ x3\ldots xm\text{-}2\ xm\text{-}1\ xm]$$

Wherein, m is the number of elements in the vector, element xi is the amplitude at the certain point of the waveform, and the time intervals $\Delta t$ between the two adjacent elements are equivalent. i represents the number of leads. The number of the blood stream leads is 3.

For the vector numerical values, the numerical values of various points in a characteristic group {a, b, c, d, e} are computed. Differential computation is performed on the vectors:

$$y(j)=x(j)\text{-}x(j\text{-}1)/\Delta t$$

j=0, 1, 2, , m

The maximum numerical value in y(i) is obtained, so that the characteristic value (a) can be obtained, wherein positive and negative reverse phase points are the characteristic value (b) and the characteristic value (d), the characteristic value (c) adopts a fuzzy recognition technology and an iterative differentiation algorithm is applied to the vector data:

$$y(j, i)=\sum(x(j+i)-x(j+i-1)/(\Delta t+i))$$

i is the increment of $\Delta t$ and in the range of 1 to N, and j is the numerical value serial number from the point (b) to the point (d).

For the various vectors in the matrix y (j, i), the minimum value in the vectors is selected and the corresponding j point represents the (c) point in the diagram.

The time-domain signal vectors of the waveform signals are obtained, namely Xi=[x1 x2 x3...xm-2 xm-1 xm], D(a, b, c, d, e), T(i=1, 2, ..., M), a given mode is classified into C categories, ω1, ω2, ..., ωc, and then judgment and classification are performed according to the distance functions between the modes. Wherein, T represents transposition; M is the number of sample points; and D is the number of characteristics of the samples. Each mode category represents the different forms of a variety of blood perfusion waveforms and computes the distance functions of the various blood perfusion waves:

$$Z(j)=(\omega j-D)$$

j=1, 2, , c

A group of vectors Z(j) is obtained, ωj corresponding to the minimum value is selected, and the various blood perfusion waves and the distance functions thereof are computed, thereby obtaining:

$$G=\sum(Di-Di-1)$$

i=a, b, c, d, e

The values are used for representing the variability of the various blood perfusion waves.

The slope and integration between the point (a) and the point (b) are computed:

$$H=[a-b]/\Delta t$$

$$T=\int y(i)*\Delta t$$

i=a, , b

The integration between the point (b) and the point (c) is computed:

$$T1=\int y(i)*\Delta t$$

1=b, , c

The integration between the point (c) and the point (d) is computed:

$$T2=\int y(i)*\Delta t$$

i=c, , d

The perfusion index is obtained:

$$P=((T*H)+T1+T2)*L/G$$

L is the distance between the point (b) and the point (e), and G is the variability of the various blood perfusion waves.

P is indexed to obtain:

$$Pi=(1-1/esp(P))\times100$$

Similarly, HPi can also be obtained by computation.

$$HPi=(1-1/esp(P))\times100$$

The electroencephalographic wave research method mainly takes the power spectral analysis as the key and takes the quantity of various frequency band components as the analysis indicator of the brain function status. Due to the individual differences in electroencephalograms, the quantification of the spectral power components of the electroencephalographic wave is difficult to achieve unity of the measured values. The expression of the various component waves in the electroencephalogram does not have continuity, the resolution of the brain function status is very low, and the linearity is not ideal. Simultaneously, due to the window effect of a power spectral mathematical algorithm on the processing of non-periodic signals, the random errors of the numerical values are caused. The commonality for being applied to the people groups is absent. The mutual comparability is absent, and it only has certain significance in self-comparison after calibration. The purpose of researching groups is very difficult to achieve.

Recently, the quantitative determination for internationally researching insomnia, anxiety and depression utilizes the method of differentiating levels of excitement of the left brain and the right brain. The data of the electroencephalogram of prefrontal lobes of the left brain and the right brain is acquired, and the traditional power spectral analysis technology is used to extract Alpha components in the electroencephalographic waves to represent the levels of excitement of the left brain and the right brain. Researches prove that the person with high level of excitement of the left brain has low probability of insomnia, anxiety and depression. The ratio of such components of the left brain to the right brain is taken as a quantification indicator, which is called as cerebral prefrontal laterality research. The problem of the research is that the spectral components in the electroencephalographic waves have no comparability in the absolute value significance due to the individual differences of the people. The sensitivity and the specificity are insufficient. The possibility of causing artifacts is large. The application value needs to be determined.

The electroencephalographic wave acquisition is generally applied to electroencephalogram rooms of the department of neurology of clinical medical units and sleep monitoring of other departments and rooms, and has become an item of basic clinical diagnosis. But the acquisition conditions of the electroencephalographic wave signals are relatively strict, so that good shielding or grounding is required. The positions of electrodes at the head of the patient still need to be processed to reduce the skin impedance. The acquisition requirements of the electrophysiological signals are achieved. For the popularized electroencephalographic acquisition application, the proposed requirements are that the environment and the conditions for acquiring the electroencephalographic signals are reduced, the deliberate treatment of the patient is not required, and the patient directly wears the electrodes to obtain the electroencephalographic signals which are in line with the electrophysiological requirements.

The electroencephalographic wave signals of the forehead of the left and the right cerebral hemispheres acquired by the transducer are transmitted to the data computation service center and the data storage center through the internet wireless mode. For the acquired electroencephalographic wave signals, the cloud computation treatment is exerted, and wavelet analysis, spectral analysis, waveform recognition, calculus and other algorithms are adopted for real-time computation, thereby obtaining a group of characteristic indicators for quantitative expression of the brain function status. The computation needs to acquire the electroencephalographic wave data of 6-10 minutes. The obtained quantitative brain function status indicators are as follows:

1. i_35: brain relaxing status index
2. i_22: brain concentrating degree index
3. i_60: brain activity index
4. i_48: cerebral thinking strength index
5. i_52: brain restraining index
6. i_66: left-right brain lateralization index (left-right brain coordination index)
7. i_45: brain stability index
8. WLi: brain sedation index
9. brain energy consumption: brain excitement level
10. brain concentration: average concentrating ability
divided into: internal concentration----concentration cohered to itself
external concentration----exchange cohered to the outside
11. brain restraint (feeling atresia): the input ability, and listening, watching and body feeling of itself are closed.

12. brain relaxation: average self-relaxing and meditating ability

13. memory processing: the situation that the brain is in the memory processing status mainly refers to the situation that the brain is in a hypnoid but not sleep status.

14. brain fatigue: brain fatigue degree

15. brain restraint: the sensory input ability is closed.

16. brain stability: the ability of maintaining a brain status

17. brain emptying: a brain empty status and a free imagination status

18. drowsiness and sleep: brain drowsiness degree

19. reaction strength: conversion speed among brain statuses

20. current status of the brain: total scores of brain ability and brain health

The extracted quantitative indexes are common components in the potential signals output by the cerebral cortex of the people and are also common components of senior thinking activities of the people. Related to the brain concentrating status, $i\_22$, $i\_52$ and brain concentration change regularly. Related to the improvement of brain relaxation and sedation, $i\_35$, $i\_48$ and WLi have significant changes of the numerical values. Related to the sub-health statuses of insomnia, anxiety, depression and fatigue, brain energy consumption, left-right brain lateralization, brain fatigue and brain drowsiness, $i\_35$, $i\_22$, $i\_66$, $i\_60$ and $i\_45$ have corresponding changes. Related to the learning ability and the problem-solving ability, $i\_22$, $i\_45$, $i\_66$, $i\_60$, internal concentration, external concentration and sensory atresia have specific representations. The changing property of the brain status indexes meet repeatability and relevance of application in the people groups. The individual differences in the electroencephalograms are also eliminated. The absolute values of the different people can be compared. Simultaneously, the brain function status revolution, specificity and linearity are good and the time resolution is relatively high.

The electroencephalographic wave signals are a main reference tool for artificial sleep staging. Clinically, manual staging judges the various stages of the sleep according to the characteristic waveforms in the electroencephalographic waves. The sleep structure is divided into two statuses, namely rapid eye movement sleep and non-rapid eye movement sleep. The rapid eye movement sleep is accompanied by rapid eye movements. The non-rapid eye movement phase is further divided into stage 1 sleep, stage 2 sleep, stage 3 sleep and stage 4 sleep, from shallow to deep. The rapid eye movement phase produces eye movement muscle electric waves on the guiding electrodes of the electroencephalographic wave on the forehead, the stage 1 sleep is marked by alternate appearance of electroencephalographic wave frequency components of 8-13hz ($\alpha$ waves) and electroencephalographic wave components ($\beta$ waves) above 13hz, the stage 2 sleep is represented by a group of 10 left-right $\alpha$ wave components or the appearance of the single electroencephalographic wave component below 4hz (top peak and k integrated wave), the stage 3 sleep is represented by the situation that the $\delta$ wave components below 4hz account for 30% of one-frame time (20 seconds). The stage 4 sleep is represented by the situation that the $\delta$ wave components account for 50% of the one-frame time. The electroencephalographic wave components between 4 and 8hz of the electroencephalographic components are called as $\theta$ waves. According to the characteristics of the electroencephalographic components, the staging of the sleep structure can be developed artificially. The invention uses the waveform recognition and the wavelet algorithm to realize automatic staging of the sleep structure by computation and analysis of the electroencephalographic wave data. After the server of the computation service center receives two lines of the electroencephalographic wave data, the cloud computation treatment is exerted, so that the nerve electrophysiological indicators representing the sleep characteristics can be extracted. Therefore, by combining with the automatic extraction of the sleep characteristic indicators of cerebral and peripheral blood perfusion waves, the precise automatic staging of the sleep structure can be realized.

The data computation service center exerts the cloud computation treatment on the received electroencephalographic wave signals, and the algorithms adopt wavelet analysis, power spectral analysis, waveform recognition, calculus and other algorithms, so that the quantitative and objective expressions for real-time reflection of the cerebral sleep structure and the current brain status are extracted. The computation of the sleep structure needs to acquire the sleep electroencephalographic wave and the eye movement wave data of 8 hours of one night. The indicators of the sleep structure obtained by computation comprise:

1. total sleep time

2. total awake time

3. arousal times

4. number of body movements

5. sleep latency

6. deep sleep efficiency

7. light sleep efficiency

8. proportion of each of stage 1, stage 2, stage 3 and stage 4 of non-rapid eye movement phase sleep in sleep time

9. rapid eye movement phase sleep latency

10. rapid eye movement phase sleep efficiency
11. number of cycles of rapid eye movement phase sleep
12. rapid eye movement phase sleep density
13. total time of rapid eye movement phase sleep
14. proportion of rapid eye movement phase sleep in sleep time

For the acquired electroencephalographic wave signals, discretization treatment with a sampling frequency of 500/s, a sampling time window of 1.5s and a sampling precision of 10-bit bite is performed to generate a vector group of the various lead waveform signals:

$$\text{fi}(x) = [x1\ x2\ x3\ldots xm\text{-}2\ xm\text{-}1\ xm]$$

i: number of leads of electroencephalographic waves, m: number of vector elements

The acquisition of the electroencephalographic wave data of each lead is performed for 1.5 seconds, and the electroencephalographic data vector constituting each lead is Ni. The pretreatment for removing the direct current component is performed on the vector data:

$$f(x)=f(x)\text{-}Av;$$

Av: direct current component of vector

The waveform recognition algorithm, the spectral analysis algorithm and the wavelet analysis algorithm are exerted on the electroencephalographic wave data after pretreatment. The wavelet analysis algorithm is taken as a latest mathematical treatment tool for computation of the electroencephalographic waves and has significant computation advantages. The wavelet algorithm is defined as follows:

If the f(x) function is the signal in the spatial domain {-∞, +∞}, the formula of the continuous wavelet transform algorithm is as follows:

$$W_c\, f(\tau, a) = \frac{1}{\sqrt{a}} \int_{-\infty}^{\infty} f(x)\phi\left(\frac{x-\tau}{a}\right)dx$$

The computation of the electroencephalographic wave vector adopts the binary discrete wavelet transform, and the formula is as follows:

$$W_{2^j}f(k) = \langle f(t), \phi_{2^j}(k)\rangle = \frac{1}{2^j}\int_R f(t)\phi\left(2^{-j}t - k\right)dt$$

The computation formula of the wavelet frequency domain adopts the following expression formula:

$$WT_x(a,\tau) = \frac{\sqrt{a}}{2\pi}\int X(\omega)\psi(a\omega)e^{j\omega t}d\omega$$

The spectral analysis algorithm adopts a discrete Fourier computation formula:

$$F(\omega) = f(\omega) = \int f(t)e^{-j\omega t}dt$$

The inverse transform adopts the following formula:

$$f(t) = \hat{F}(\omega) = \frac{1}{2\pi}\int F(\omega)e^{j\omega t}d\omega$$

For the electroencephalographic wave data, the binary algorithm and the waveform reconstruction algorithm in the wavelet analysis are firstly adopted for processing the specific electroencephalographic wave data vector, a first-order derivative (a spline function) of a smooth function is taken as the generating function, 64 points are constructed, the scale is in the range of $2^0$-$2^6$, and the binary wavelet transform is performed:

$$(\text{Wf}(2\hat{\ }j, x))j \in z$$

A group of wavelet transform basis functions under a bandpass filter group can be obtained:

(Wf(2^0,x)), (Wf(2^1, x)), , , (Wf(2^N,x))

The reconstruction adopts the reconstruction of the wavelet function and the reconstruction of various wavelet basis functions, and the formula is as follows:

$$f\ j\ (x) = \sum \text{Wf}(2\hat{\ }j, x) * X\ 2\hat{\ }j(x)$$

Wherein, X2^j(x) is the reconstructed wavelet; and j is the order of the time domain function.
For the reconstruction of the different wavelet basis functions, a group of reconstruction functions are obtained:

f1(x), f2(x),, fN(x); N=order;

The integration algorithm is adopted for the various waveform functions fi(x) obtained by wavelet basis reconstruction and computation.

$$E\ (i) = (\int fi(x)dx)\hat{\ }2\ \ ;$$

i=1, 2, , N;
The waveform potential power E(i) of each wavelet basis reconstruction function can be obtained. For each electroencephalographic wave vector:

$$f\ (x) = [\ x1\ x2\ x3\ \cdots\ xm-2\ xm-1\ xm\ ]$$

The power spectral function is synchronously computed by using the fast Fourier transform computation formula:

$$X(\omega) = \int_{-\infty}^{\infty} f(x)e^{-j\omega t}dt$$

The computation window N=512, the $\alpha$ wave components of 8-13hz, the $\delta$ wave components of 0.5-4hz, the $\theta$ wave components of 4-8hz and $\beta$ wave components of 13-30hz can be obtained. The dominant frequency, edge frequency and central frequency are as follows:

$$\alpha\ \%=X\ (\omega 8-13)\ ;\ \delta\ \%=X\ (\omega 0.5-4)\ ;\ \theta\ \%=X\ (\omega 4-8)\ ;\ \beta\ \%=X\ (\omega 13-30)\ ;$$

$$\text{Fsef}= (\int X\ (\omega)\ \hat{\ }2 * \Delta\omega > 0.95)$$

$$Fmax = (X(\omega)^2) max$$

$$Fc = (\int X(\omega)^2 * \Delta\omega > 0.5)$$

The extreme points of the reconstruction functions fi(x) and f(x) are computed by using the waveform recognition algorithm. The fi(x) is derived as follows:

$$Di(x) = (fi(x) - fi(x+m)) / \Delta x$$

Di(x) is sorted to obtain the maximum value and the minimum value, a threshold value a is set, the muscle action potential of the eye movement waves and the like can be figured out, and the eye movement phenomenon and the eye movement density of the rapid eye movement phase sleep are captured.

The positive and negative polar mutation points of Di(x) are figured out to obtain a group of extreme point vectors:

Mi(j) and mi(j); and j is the number of the extreme points.

For the obtained computation results, a cluster of intermediate computation result vectors are generated by combination.

Gi(x)={Mi(j), mi(j), Fsef(i), Fmax(i), Fc(i), $\alpha$%(i), $\delta$%(i), $\theta$%(i), $\beta$%(i)}

x is the number of intermediate results, x=0, 1, , N;

A series of normal mode regression coefficients are set as weighting factors and set as {ai1, ai2, , ain} and the following regression formula is used:

$$y = ai1*Gi(0) + ai2*Gi(1) + \cdots\cdots + ai(n-1)*Gi(N) + ain$$

A method of least squares is used to make the value in the following formula

$$q = \sum\{y - (ai1*Gi(0) + ai2*Gi(1) + \cdots\cdots + ai(n-1)*Gi(N) + ain)\}^2$$

be the minimum, and the regression coefficients meet the following formula:

$$(CC^T)\{ai, ai2, , ain\} = C\{y0, y1, , yt\}$$

$$\{y0, y1, , yt\} = \{i\_22, i\_35, i\_60, i\_66, i\_52, i\_48, i\_45, WLi\}$$

For the obtained regression coefficients, the real-time brain status indicators are computed as follows according to the weighted items of the most indicators:

$$\{i\_22, i\_35, i\_60, i\_66, i\_52, i\_48, i\_45, WLi\}j = \{ai1*Gi(0) + ai2*Gi(1) + \cdots\cdots + ai(n-1)*Gi(N)\}j$$

j=number of leads in electroencephalographic acquisition

For the acquired left-right real-time concentrating and relaxing indexes (i_22 and i_35), the real-time thinking strength index and relaxing index (i_48 and i_35), as well as the real-time sensory atresia index and thinking strength index (i_52 and i_48), a scatterplot is generated to represent the change of morphology expressing the left-right brain change of the brain status. For the coordinates, i_22, i_48 and i_52 are taken as the vertical coordinates, i_35 and i_48 are taken as the horizontal coordinates, vertical lines and horizontal lines are set at the scale mark of 240 of i_22, the scale mark of 40 of the i_35 horizontal coordinate, and the scale mark of 90 of i_48 horizontal coordinate to divide the coordinate region into four quadrants. Each of the i_22, i_35, i_48 and i_52 data points is shown as one point in the scatterplot. Different brain status changes can be expressed by the density and distribution of scatter points. The brain status can be defined from the image through the left-right brain scatterplot, and the vergence rate, clusters and other information are extracted from the distribution of the scatter points to realize quantitative extraction. See Fig. 17.

By computing the different combinations of the real-time brain status quantitative indicators and the relationship between the data of the different electroencephalographic wave leads, the left-right brain lateralization, brain energy consumption, brain concentration, brain activity, brain restraint (sensory atresia), brain relaxation, fatigue, drowsiness, reaction force, memory processing, brain stability, brain emptying and other quantification indicators can be figured out. The computation time window is 6-10 minutes, the medical application is 10 minutes, and the others cost 6 minutes.

$$\text{Brain energy consumption} = \{a0, a1, , an\} * \{i\_22, i\_35, i\_60, i\_66, i\_52, i\_48, i\_45, WLi\}$$

{a0, a1, , an} is the regression coefficient
Left-right brain lateralization={left (i_22)>right (i_22)} $\in$ T
T=6 minutes

$$\text{Brain concentration} = \sum(i\_22) < K0$$

$$\text{Brain restraint} = \sum(i\_52) < K1$$

$$\text{Memory processing} = \sum(i\_60) < K2$$

$$\text{Brain stability} = \sum\sum\{(i\_22)\}/N-(i\_22)$$

$$\text{Brain emptying} = \sum(i\_48) < K3$$

The generation is according to the distances between the clusters of the brain status in the scatterplot of (i_22, i_35).

$$\text{Reaction force} = |\sum(i\_22) < K4 - \sum(i\_35) < K5|$$

$$\text{Brain relaxation} = \sum(i\_35) < K6$$

$$\text{Brain activity} = \sum(i\_45)/N$$

$$\text{Fatigue} = \sum(i\_22) * K7 + \sum(i\_45) * K8$$

$$\text{Drowsiness} = \sum(WLi) < K9$$

{K0, K1,, K9} are the normal mode regression coefficients.

For the normalization computation of the quantified brain status characteristic indicators:

{brain status characteristic index group}=(1-1/esp(E/A))×100

A is a quantifying factor; and E is:

{brain energy consumption, brain concentration, brain activity, brain relaxation, brain restraint (sensory atresia), brain stability, brain emptying, brain fatigue, drowsiness, reaction force, memory processing}

The numerical values of all the parameters are set as shown in Fig. 18 under the normal mode.

The computation of the sleep structure adopts the combination of a variety of algorithms, such as waveform recognition, wavelet analysis, power spectrum, mode recognition and the like. The electroencephalographic waves acquired on both sides of the forehead comprise eye movement waveform components and forehead muscle electrical components. The wavelet formula is as follows:

$$WT_x(a,\tau) = \frac{\sqrt{a}}{2\pi} \int X(\omega)\psi(a\omega)e^{j\omega t} d\omega$$

For the electroencephalographic vector group:

$$WT_x(a,\tau) = \frac{\sqrt{a}}{2\pi} \int X(\omega)\psi(a\omega)e^{j\omega t} d\omega$$

i: number of leads of electroencephalographic waves, m: number of vector elements The real-time computation treatment is performed, and the wavelet basis functions under the various scale windows are decomposed by the multi-scale filter group algorithm (being the same as the electroencephalographic treatment).

$$(Wf(2^\hat{\ }j, x))j \in z$$
$$(Wf(2^\hat{\ }0, x)), (Wf(2^\hat{\ }1, x)), \,, (Wf(2^\hat{\ }N, x))$$

Similarly, the obtained group of time-domain reconstruction functions is as follows:

$$f_j(x) = \sum Wf(2^\hat{\ }j, x) * X 2^\hat{\ }j(x)$$
$$f1(x), f2(x), \,, fN(x);$$

N=order;

All the time-domain functions represent the electroencephalographic waves, eye movement electric waves and muscle electric waves in different frequency bands. For the various decomposed waveforms, the waveform recognition algorithm is adopted for extracting eye movement, electroencephalographic spindle waves, electroencephalographic slow waves, electroencephalographic fast waves, top waves, k integrated waves and other electroencephalographic sleep characteristic wave components. Firstly, the first-order derivative of each waveform is figured out:

$$Di(x) = (fi(x) - fi(x+m))/\Delta x$$

Di(x) is sorted to obtain the maximum value and the minimum value, and the threshold value a is set, so that the eye movement and other large noise wave components can be eliminated.

The positive and negative polar mutation points of Di(x) are figured out to obtain a group of extreme point vectors:

Mi(j) and mi(j); and j is the number of the extreme points.

The mode recognition algorithm is adopted to obtain the extreme point vectors Mi=[x1 x2 x3...xj-2 xj-1 xj], D (a1, a2, a3 ..., an), the extreme point vectors of the various given electroencephalographic sleep standard characteristic waves are classified into C categories, ω1, ω2, ..., ωc, and then judgment and classification are performed according to the distance functions between the modes. Wherein, M is the number of sample points; and D is the number of characteristics of the samples. Each mode category represents different sleep electroencephalographic forms, and the distance functions of the various vectors are computed as follows:

$$Z(j)=(\omega j-D)$$

j=1, 2, , c

A group of vectors Z(j) is obtained, ωj corresponding to the minimum value is selected and the sleep characteristic wave types included in the acquired electroencephalographic time-domain waveforms are obtained. Simultaneously, the integration algorithm is adopted for classification functions of the eye movement and muscle electric waves:

$$E = \int f0(t)^2 * \Delta t$$

$$E1 = \int f1(t)^2 * \Delta t$$

The power values of muscle electric waves and eye movement electric waves are obtained, the muscle electric components and the eye movement components in the electroencephalographic waves can be obtained through the threshold method, thereby obtaining the numerical values for the characteristic indicators of the rapid eye movement phase sleep.

For the time-domain electroencephalographic waves, the computation is the same as the treatment of the electroencephalographic waves, and the power spectral algorithm is adopted:

$$X(\omega) = \int_{-\infty}^{\infty} f(x)e^{-j\omega t}dt$$

The various components in the power spectrum of the electroencephalographic waves can be obtained, including the numerical values of $\alpha\beta\delta\theta$ wave bands, as well as Fsef, Fmax and other edge frequency, dominant frequency and other numerical values.

For comprehensive classification of the various quantitative numerical values, what is the percentage of the slow wave components in the electroencephalographic waves within a period of time (which is 20 seconds or 30 seconds generally) can be obtained, if the percentage is above 50%, it is the stage 4 sleep, if the percentage is above 30%, it is the stage 3, if the spindle waves or the top waves and the k integrated waves exist, it is the stage 2, if the percentage of eye movement and muscle electric waves is zero, it is the rapid eye movement phase, and it is the stage 1 sleep in other situations. In the classification process, the refining judgment is performed on the rapid eye movement phase sleep, the body movements and the awake status by combining with the edge frequency and the dominant frequency obtained by power spectral analysis, the recognition of the rapid eye movement phase sleep is supplemented, and simultaneously, the arousal times, the number of body movements and other quantitative data are obtained. The recognition of the number of body movements performs threshold computation on the computed power E1, the movements with the power which is above 2 times the power of the average eye movements are the body movements, and the computed number of times is the numerical value of the number of body movements.

In the complete one-night sleep, the recognition of the point falling into sleep and the computation of the sleep latency adopt the fuzzy method. The time length from the manual determination of the sleep time as the start or a period of time that the number of the body movements is zero as the start to the appearance of the stage 1 sleep or the appearance of other stages of sleep is mainly computed as the sleep latency.

Similarly, the computation of the vascular elasticity is performed against the data vectors of the blood perfusion waves and the electrocardiographic waves:

$$f(x) = [\ x1\ x2\ x3\ \cdots\ xm{-}2\ xm{-}1\ xm\ ]$$

$$e(x) = [\ x1\ x2\ x3\ \cdots\ xm{-}2\ xm{-}1\ xm\ ]$$

i: number of leads of electroencephalographic waves, m: number of vector elements

Similar to the computation of the blood perfusion waves, the extreme points of the blood perfusion waves are firstly obtained; and simultaneously, the wavelet algorithm is used for real-time computation of the electrocardiographic waveform, and three points, namely Q, R and S points of the electrocardiographic QRS wave are obtained. The wavelet transform of the electrocardiographic waves is as follows:

$$(\mathbb{W}e\,(2\hat{\ }j,x))j\in z$$

For the high-frequency band wavelet basis function, the reconstruction function is taken to obtain the reconstruction time-domain function of the electrocardiographic high frequency band:

$$e1\,(x)=\sum \mathbb{W}f(2\hat{\ }j,x)*X2\hat{\ }j(x)$$

The average value is taken by utilizing the threshold algorithm, the horizontal coordinates of the points which are above 50% higher than the average value are computed, the horizontal coordinates of the points which are smaller than the numerical value are 0 and the coordinate vectors are obtained:

$$e\,(t)=[\;t1\;\;t2\;\;t3\;\;0\;\;0\;\;0\;\;t4\;\;t5\;t6\;\;\;0\;\;0\;\;0\;]$$

The points which are adjacent to the numeric value which is computed to be 0 and are not 0 are the Q and S points. The middle point is the R point.

The vascular elasticity can be obtained by combining with the electrocardiographic computation and the computation of blood perfusion and computing the relationship of the various characteristic points.

The treatment of the respiratory waves is equivalent to that of the blood perfusion waves. The data vectors of the respiratory waves are as follows:

$$h\,(x)=[\;x1\;\;x2\;\;x3\;\cdots\;xm-2\;\;xm-1\;\;xm\;]$$

The differential computation is implemented on the vectors of the above vector numerical values:

$$y\,(j)=h\,(j)-h\,(j-1)\,/\,\Delta t$$

j=0, 1, 2, , m

Positive and negative reverse phase points, namely the maximum point and the minimum point, are obtained, the differences between the horizontal coordinates between the extreme points are the time intervals of the respiratory waves, and if the interval time is more than 10 seconds, the apnea occurs.

Longevity solves the life process problem of the person, the life process is a metabolic process of an organism, the metabolic process requires the flow of the blood stream, and it is concluded that the life process needs to maintain the smooth and reasonable systemic flow of the blood stream. What is the nature of the blood stream, one major point is that the blood stream carries the oxygen component and the carbon dioxide component. The oxygen component is ingested by respiration, and the carbon dioxide is also exhausted by respiration, thereby forming the basic viewpoint of qi and blood in the traditional Chinese medicine theory. The conclusion of the problem is that, the higher the content of the oxygen in the blood, the more helpful to the life process; and the more reasonable systemic distribution of the blood, the more helpful to the life process. The two problems are the key issues which the people face in the aspect of longevity. They are links which can be artificially controlled by the people, and the longevity quantitative traction theory and technology can be formed for implementation.

The quantitative traction for health keeping and life prolonging can be decomposed into the traction of quantitative physiological data expressing the core quality of life, and the traction can be the quantitative traction by using the independent consciousness control of the internal vital sign data and can also be external quantitative control traction of other people with the high-quality vital sign data. The core of the longevity is the action of the internal mechanism, and the external mechanism is auxiliary, which can be explained by the situation that the people with longevity exist all over the world no matter what the environments and conditions they are living in are. There is a law in the nature that the service life of most of the mammals is 7 times of their growth period, while the service life of the people is just 4 times of their growth period. The difference is the evolution of the human senior cortex cognition and emotional function. People with the cognition and emotional competence can perform scientific invention, realize scientific progress, improve the disease treatment effect of the people and prolong their lives. However, the energy supply for survival of the people can also be consumed, thereby affecting the prolonging of the life. The balance result is that the average service life of the people is 70-80 years old. The influence of the cognition and emotions on the service life is the consumption of a lot of energy, namely the oxygen metabolism of the cerebral cortex is increased, and the corresponding blood perfusion is also increased. The phenomenon results in the relative reduction in the blood perfusion of other functional organs of the human body related to the life development, so that the oxygen metabolic process and the discharge of metabolic products are affected, and the life of the people is relatively shortened. The life activities of the people can be understood as follows:

1. Blood stream is the energy and can also be known as mental energy.
2. The cerebral cortex is similar to a cup core of the computer.
3. Autonomic nerves (also including cerebellum) is equivalent to another cpu core of the computer.
4. The double cores constitute a control system of a human body, the cortex is responsible for cognition, emotions and other senior thinking activities, and the autonomic nervous system is responsible for the coordination work of all the systems of the body, namely health.
5. The double cores compete respectively to fight for the control right of the energy.
6. The energy is certain, if one core controls more energy, the other core controls less energy.
7. The psychological and spiritual effects on the body are represented by the physical change, namely the change of energy consumption and the amount of blood stream.
8. If the energy controlled by the cerebral cortex is increased (long-term), the energy controlled by the autonomic nerves is reduced, and the health further has problems.
9. Concentration, meditation, yoga, tai chi, qigong, mind concentration of traditional Chinese medicine and the like can reduce the demands of the cerebral cortex for the energy (the energy demands in part of the cerebral cortex region are reduced).
10. Sleep and motion also reduce the demands of the cerebral cortex for the energy.
11. The reduction in energy of the cerebral cortex has an important role for the physical health of people.
12. The amount of the blood stream, namely the amount of energy is determined by FMRI, which represents the intensity of activities.
13. The reduction in the energy demands of the brain is a kind of dormancy of the brain, which is also good for the brain itself.
14. Coma is a way of resisting the diseases of the person, the cerebral cortex gives up the demands for most of the energy and enters into a coma status (non-cerebral trauma and shock), and then the autonomic nerves take over most of the energy and resist against death.
15. A flash of lucidity of the dying means that after the failure of autonomic control, the energy further normally returns to the cerebral cortex, and the person has the flash of lucidity and then dies.
16. The slowing of the brain is another way of saying the reduction in the energy of the cerebral cortex actually.

Research reports announce that researchers use functional magnetic resonance imaging (fMRI) to seek for the brain differences between meditators and non-meditators, and it is found that meditation seems to be able to close the brain region which is associated with reverie, anxiety, schizophrenia and other mental illnesses. The conclusion is that the meditation can be applied to different health problems, such as smoking cessation and response to cancers, and even psoriasis. The brains of the experienced and novice meditators are scanned by fMRI, and it is found that the experienced meditators can reduce the activities of the brain region which is called as a default mode network, and reduce the blood perfusion in the related brain region.

Why the people with tension and anxiety are susceptible to neoplastic diseases, why many people suffering from the neoplastic diseases are scared to death, why the trust in one doctor can greatly enhance the treatment effect, why pleasure is an important factor of longevity, why a psychological placebo effect can be realized, why coma can occur before the death caused by the disease without injuries to the brain, and why the phenomenon of the flash of lucidity of the dying can occur, the appearance of such phenomena means that some physical and chemical changes occur in the human body, wherein we assume that the physical change is the change of the systemic blood perfusion, indicating that a life control mechanism adjusts the systemic blood perfusion due to psychological factors or the crisis of the human body and reflecting the influence of the psychological factors on the health and disease treatment.

Longevity is a way of thinking, thinking represents concentration, concentration represents that most of the functional regions of the brain are inhibited, the corresponding blood perfusion is reduced, the consumed oxygen energy is reduced, and the energy supplied to other organs is increased. One of the key factors of the longevity is the blood distribution in the body and the respiratory efficiency. The blood distribution represents the systemic distribution of the oxygen energy and the respiratory efficiency represents the total amount of the oxygen contained in the blood. The correct blood distribution can prolong the life of people and the correct respiration way can also prolong the life of people. The blood distribution is related to the sleep of the person, related to the thinking of the person, related to the emotions of the person and also related to the exercises of the person. The respiratory efficiency is related to the vital capacity of the person, related to the exercises of the person and also related to the respiration method of the person. The blood stream mainly affects the longevity in the amount of the carried oxygen component which is conveyed into the organs of the body or the amount of the metabolites which are taken out. Respiration mainly affects the longevity in the exchange efficiency of the oxygen and the carbon dioxide in the lung, the total amount of air which is inhaled into the lung every time, the heart rate and the oxygen consumption caused by strenuous exercises. The appropriate and reasonable blood distribution, the efficient respiration way and the high vital capacity are important conditions for health keeping and life prolonging. The traction of longevity is turned to the traction of the blood stream and turned to the traction of respiration. The traction of the blood distribution is against the blood perfusion of the body and the cerebral blood perfusion. The quantitative real-time indicators of the blood perfusion indexes are obtained by using the measurement method of blood perfusion in the invention, and the measurement results show that, sleep, meditation, yoga, meditation, tai chi, appropriate exercises and the like reduce the demands of the head for oxygen metabolism, relatively reduce the cerebral blood perfusion amount and increase the blood perfusion amount of the body. Tension, fear, anxiety, depression, insomnia and the like can cause reverse changes of blood distribution.

Sleep is favorable to health, trotting is favorable to health, and the general knowledge has been well accepted. The conclusion is that the action of sleep on health keeping and life prolonging is that the blood perfusion changes, so that the organs of the body can obtain more blood streams. The action of trotting on health keeping and life prolonging is that the respiratory rate is accelerated, the heart rate is accelerated, the oxygen content in the blood per unit time is increased, and the cycle that the blood carrying oxygen realizes systemic circulation is increased. However, excessive exercises can injure the health, and the reason is that the oxygen consumption of the muscles is more than the oxygen acquisition, and the oxygen metabolism is converted to the external acting.

Sleep can improve the blood distribution of people, electroencephalographic waves can also express the quantitative physiological data of the sleep, the longevity quantitative traction can also be converted to quantitative traction of the electroencephalographic waves, the electroencephalographic waves reflect the change of the brain status, and a real-time brain status measurement technology is the motive power for quantitative traction of the brain status, has great effects on longevity and is also a main tool for implementing sleep traction on other people by external high-quality sleepers. For a long time, there are some methods for controlling the brain status by internal independent consciousness at all times and in all countries, which are non-quantifying methods for longevity and are obtained by natural exploration of the people, the core significance is that the systemic blood perfusion distribution is affected, and meditation, hypnosis, meditation, yoga, tai chi, praying and various types of qigong are the manifestations of such methods. Doing appropriate exercises, adjusting the respiration, appropriately accelerating the heart rate and improving the muscle strength of the lung can positively promote health keeping and life prolonging, and quantitative traction of the respiratory efficiency and the quantitative traction of the vital capacity are direct methods for increasing the total oxygen amount in the blood stream and are another manifestation of longevity. The effects of blood distribution and respiratory efficiency on health keeping and life prolonging are represented by qi-blood principle and yin-yang balance principle in the traditional Chinese medicine theory. The quantitative and real-time independent traction of the blood distribution and the respiratory efficiency can continue life process and achieve the purpose of prolonging life.

The disease treatment mode in the background of brain suppression is a fine control mode for treating diseases in the background of the human body. The blood perfusion has an obvious effect on health keeping and life prolonging. The blood perfusion also has a clear effect on the treatment of diseases, especially the treatment of serious diseases. Active control of the change of the blood perfusion of the head and the body of the human body (in addition to the diseases of the head) can improve the treatment effect, and the senior cortex cognition function consumes the energy, consumes the oxygen carried in the blood stream and increases the perfusion amount of the blood stream into the head. Such energy consumption produces certain adverse effects on the treatment of diseases, controls the energy demands of the brain senior cognition function and temporarily realizes inhibition and dormancy of the brain cognition function, and in such a background, disease treatment is developed, the conveying of the blood perfusion into the brain can be reduced, the blood stream perfused into the part of a focus is increased, and immune cells and antibodies, as well as the oxygen component carried in the blood, can reach the part of the focus to a greater extent, thereby producing positive effects on the disease treatment; and such a method of controlling the disease treatment background can be regarded as the combination of external disease treatment means and internal disease resistance of the body for joint inhibition of the disease development. Coma can be regarded as a regulation mechanism (in addition to brain lesions or pathological coma) for resisting against diseases in the body, when the invasion of the disease into the body has achieved the life-threatening stage, the life control center automatically regulates the systemic blood distribution, reduces the cerebral blood perfusion, increases the blood perfusion at the disease part and resists against the development of the diseases by using the antibody cells, immune cells, oxygen energy and the like carried in the blood stream, thereby causing coma of the person. If the cerebral blood perfusion is reduced, the cerebral oxygen metabolic level is also reduced, most of the independent consciousness functions of the person are inhibited, and then cognition and emotions become meaningless at this time. This is the reflection of the disease resistance of the body. We can also see that the disease resistance of the human body has the same manifestation as the disease resistance of an animal. The phenomenon of the flash of lucidity of the dying is also one reaction of such ability and is the phenomenon losing such ability. The further development of diseases injures the functions of the life control center; and after such regulation mechanism is lost, blood perfusion further returns into the brain, and then the person falls into a moment of sober and a moment of consciousness. What is accompanied is that the memory of the person becomes more clear at this time and is greatly different from the cognition degree in the status of the disease, so that it can be concluded that the active reduction of the cerebral metabolic demands can reduce the cerebral blood perfusion and cause inhibition and dormancy of the brain, this is the protection of the brain actually, similar to the situation that the resting potential appears in the brain and the cerebral metabolism is reduced to the lowest level by anesthesia and sedation in the heart surgery and is consistent with the conclusion of best brain protection. Disease resistance of the body can be mobilized by using external means as early as possible, which is equivalent to the situation that the disease has not developed to the stage of realizing onset of action of the internal coma mechanism of the body and drives the coma mechanism to realize onset of action by an external force, thereby perfusing more blood stream to the site of the disease and macroscopically improving the disease treatment effect. It can also be regarded as the situation that the factors in psychological negative emotions affecting the disease treatment are eliminated as early as possible.

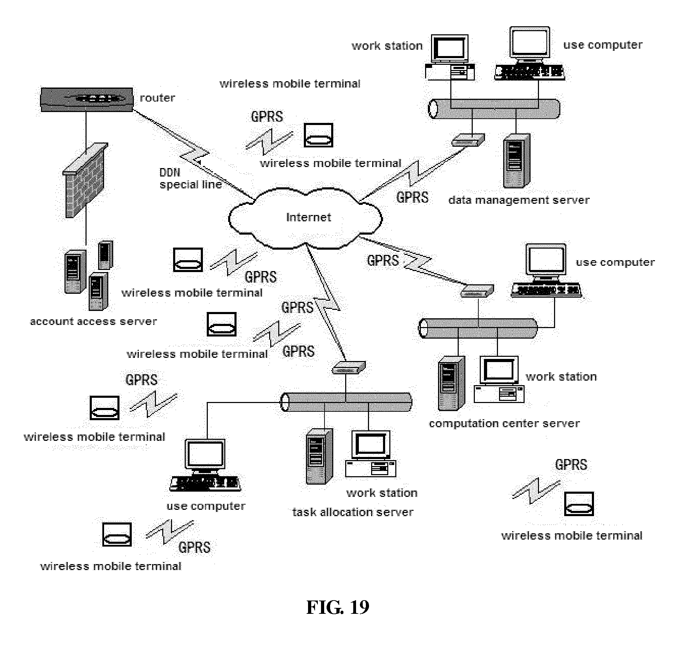

Closed-loop feedback control is performed over the infusion of the venous sedative with the infusion pump by using characteristic indicators of the brain blood perfusion index, wherein, the feedback control mechanism is a steady-state regulation system which controls the brain blood perfusion index to reach the minimum. Closed-loop feedback control is performed over the infusion of the venous sedative with the infusion pump by using the characteristic indicator of brain blood perfusion index of the supraorbital artery as a feedback parameter, to achieve the control target that the brain blood perfusion index of the supraorbital artery reaches the minimum. The brain blood perfusion signal of the supraorbital artery is acquired by using signal acquisition and computation technology of blood perfusion of the supraorbital artery, through a wireless mobile terminal for acquisition and transmission of vital signs, transmitting the signal to a processing computer through WIFI, allowing the computer to automatically compute and process the signal to obtain quantitative data of the real-time blood perfusion index, and then sending a control instruction to a computer interface of the UK Graseby 3400 infusion pump through a standard RS232 communication interface, so as to control the automatic infusion of sedative with the infusion pump, thereby achieving the purpose of inhibition of cerebral cortex cognition function. Target Controlled Infusion (TCI) is adopted as the control mechanism, with the blood drug concentration as a controlled object and the blood perfusion index as a feedback regulation parameter, the blood perfusion of the supraorbital artery is adjusted to the minimum by increasing or reducing the blood drug concentration, so as to realize the background method of quantitatively controlling the automatic sleep sedation therapeutic treatment. The control flow is as shown in Fig. 19.

The home-based using mode of measurement and computation of vital sign data is a new mode which can sustainedly analyze and express the data for the life status. Wherein, the contents for measurement and computation are acquired from clinical inspection and measurement service flow developed in a hospital after the onset of the original diseases. Part of the decomposable contents in the medical clinical inspection items are decomposed to single standardized life status quantitative measurement services. Each measurement and computation item can be freely developed in a simple and feasible way in any environment, and the data is transmitted and processed in an automated manner and automatically fused into a database for saving. The data review and browse take the web application of the personal health bank account as a management platform and have the integration ability of self-organizing, analyzing and computing the information of the life status, forming physiological and vital sign data inspection reports of a variety of diseases and forming health prediction reports. For most of the chronic diseases, the doctor's diagnosis is performed on the basis of quantitative medical inspection and measurement. Moreover, after the occurrence of the disease, inspection and measurement are performed, diagnosis and treatment are further performed, and the health information before the occurrence of the disease becomes a completely blank period, so that prevention and prediction can not be performed. By changing such mode, the modern technical development has been capable of supporting the appearance of new health and medical modes. In the aspect of various inspection and measurement processes required for analyzing diseases and performing diagnosis, for most of the medical inspection items, in addition to the images and part of the complex biochemical inspections, all the contents including the hardware for signal acquisition and all the indicators in the measurement items, which are included in the inspections and measurements need to be split and decomposed to form various independent single-item and single-indicator inspection and measurement contents; and in combination with the modern wireless communication technology and the automatic computation and analysis technology, the single-item inspections and measurements are popularized into family environments and working environments for sustained and regular execution, the characteristic changes of diseases are automatically extracted, so that the medical inspections and measurements after the occurrence of the diseases can be extended to be sustainedly conducted before the occurrence of the diseases, the characteristic changes can become private contents of personal health information for saving under the personal health bank account, a big data architecture, such as internet and the internet of things for real-time intelligent information exchange can be taken as application carriers, and such application realizes the process innovation of disease diagnosis, namely, in addition to the current inspections and measurements required for disease diagnosis, the doctor can also browse the recent home-based inspection and measurement results of related diseases. Simultaneously, and most importantly, a new disease treatment and health management mode for realizing prevention and prediction of related diseases is realized. The specific contents of the diagnosis and inspection items for various diseases are classified, and the vital sign data is acquired and computed in a wireless mobile way under the management of the personal health bank account. The various indicators in the diagnosis, inspection and analysis reports of the diseases are subdivided into independent units, acquisition and transmission are performed respectively, and the single-item vital sign data sets are further constituted. The data in the vital sign data sets can be automatically organized in the account according to the operation of the user and sub-classified into the vital sign data sub-sets of different diseases to obtain a decomposed cluster of characteristic indicator groups pointing to the development and change of different diseases, generate medical inspection and analysis reports of specific diseases and the prevention and prediction reports of occurrence and development of specific diseases. A data exchange method for converting the single indicator cluster to the disease or the health analysis report is established. The data in the account is stored in the format of single vital sign data, thereby forming a set of medical service contents which can be independently purchased and quantitatively point to the development and change of certain life status. After splitting, the inspection and measurement items which can perform the daily vital sign data computation service are as follows:

| Category | No. | Name | Explanation |
|---|---|---|---|
| **Measuring and calculating the sleep** | 1 | Analyzing and calculating the time accounted for by deep sleep in the sleep of a night | Sleep accounts for one-third of the human lifetime, the matter related to health and diseases and related to what will happen during this one-third of the lifetime are of significant values. For patients with severe neurasthenia, including insomnia, anxiety, depression, Alzheimer's disease, ADHD, mental disorder, and patients with sleep disorders, including sleep apnea hypopnea, etc., it is required to measure the amount of the time of deep sleep in the sleep of a night. |
| | 2 | Analyzing and calculating the time accounted for by light sleep in the sleep of a night | As mentioned above, for the above diseases, it is also needed to measure the amount of the time in light sleep. |
| | 3 | Analyzing and calculating the time of awakening in the sleep of a night | For the diagnosis of the above diseases, it is also needed to measure the time of awakening in the sleep of a night |
| | 4 | Analyzing and calculating the time of a latent period in the sleep of a night | For the above diseases, the adjoint representation is the occurrence of sleep disorders; thus it is needed to understand and measure precisely the length of the sleep latency, that is, the time from beginning to sleep to the time of falling asleep to express the severity of sleep disorders. |
| | 5 | Analyzing and calculating the time of dreaming in the sleep of a night | For the diagnosis of the above diseases, it is also needed to measure the condition of the fast phase asleep in the sleep of a night, that is to say, how long is the time for dreaming. |
| | 6 | Analyzing and calculating the total time of oxygenation decrease by 4% in the sleep of a night | If diseases such as sleep apnea hypopnea, or heart malfunction, cardiovascular embolism, etc. occur during the sleep, which show as the decrease of the blood oxygen saturation. Compared with a person in sober, the time when oxygenation is decreased to less than 4% is a major basis for disease diagnosis and the alarm for the diseases outbreak. At the same time, it is also an important presentation for human health keeping and life prolonging. |

(continued)

| Category | No. | Name | Explanation |
|---|---|---|---|
| | 7 | Analyzing and calculating the lowest value of oxygenation in the sleep of a night | In a similar way with the above-mentioned, the lowest value to which the oxygenation decreases may be also an important diagnosis basis, and an explicit indicator for good health keeping and life prolonging. |
| | 8 | Analyzing and calculating the value of the mean oxygenation in the sleep of a night | In a similar way with the above-mentioned, the value of the mean oxygenation in the sleep may be also an important diagnosis basis for good health keeping and life prolonging. |
| | 9 | Analyzing and calculating the time of turning over and moving the body in the sleep of a night | The above diseases show the problem on the sleep quality, and the time of turning over the body in the sleep of a night represents the sleep quality. |
| | 10 | Analyzing and calculating the times of the fastest heartbeat in the sleep of a night Analyzing and calculating the times of the slowest heartbeat in the sleep of a night | During the sleep, the variation of the heart function is an important indicator of the whole presentation of body functions, and the fastest and slowest beat may be the data needed to be measured for the diagnosis of heart diseases. |
| | 11 | | |
| **Measuring and calculating respiration** | 12 | Analyzing and calculating the times of apnea continuing for more than 10 seconds | During the sleep, the disorder of the muscular flaccidity or the central nervous system may result in apnea and hypopnea, thus the measurement of the times of apnea in the sleep is one of the indicators for the diagnosis of apnea hypopnea syndrome, is also one of the key indicators to understand the health keeping and life prolonging status for oneself. The time of apnea should be greater than 10 seconds. |
| | 13 | Analyzing and calculating the mean value of oxygenation in the sleep of a night | For the calculation of apnea and hypopnea, the mean value of the oxyhemoglobin saturation may be an important indicator representing the severity of apnea. |
| | 14 | Analyzing and calculating the longest time of apnea in the sleep of a night | During the sleep, if apnea occurs, the longest time of the apnea may be a main indicator for the severity of the diseases. |
| | 15 | Analyzing and calculating the period of apnea in the sleep of a night | The occurrence frequency of apnea is also an important data reflecting the severity of the diseases. |
| | 16 | Analyzing and calculating the mean respiratory rates per minute in the sleep of a night. | The mean respiratory rate per minute in the sleep is the assistant data for understanding the vegetative nerve function. |

(continued)

| Category | No. | Name | Explanation |
|---|---|---|---|
| | 17 | Analyzing and calculating the number of apnea accidents accompanied by awakening in the sleep of a night | In the sleep, the occurrence of apnea or hypopnea may interrupt the course of human sleep, and result in awakening, and has an adverse impact on human sleep quality, thus measuring and calculating the times of apnea accidents accompanied by awakening is also the key data for the severity of diseases. |
| **Measuring and calculating blood stream and blood pressure** | 18 | Analyzing and calculating your blood pressure variation trend in a designated time period | The value of the blood pressure may be a main indicator representing the function of the human circulation system. The trend variation of the blood pressure is the reference for diagnosing cardiovascular diseases caused by hypertension. Especially, the variation trends of the blood pressure in the sober time period and the sleeping time period respectively within 24 hours are significant for predicting the outburst cardiovascular diseases. |
| | 19 | Analyzing and calculating the highest value and lowest value of your blood pressure in the designated time period and how long the blood pressure at the highest value and the lowest value lasts. | The highest and lowest value of your blood pressure in the designated time period are the indicators for hypertension, and the difference between the two blood pressures is also the reference for the severity of diseases. When the hypertension occurs and when the blood pressure decreases are the reference for the drug administration and the activity during the hypertension treatment. |
| | 20 | Analyzing and calculating the value of the blood pressure before falling asleep and after awakening in the sleep of a night | The value of the blood pressure before falling asleep and after awakening is an indicator reflecting the severity of the impact of the occurrence of apnea on the blood pressure in the sleep, and the therapy effect may be reflected based on this. |
| | 21 | Analyzing and calculating the variation of your vascular elasticity in the designated time period | The vascular hardenability is the main presentation related to cardiovascular and cerebrovascular diseases and many severe diseases. The measurement of the vascular elasticity is mainly directed to the vascular of the bloodstream passage of the head blood perfusion and the blood perfusion in the limb ends, may also be directed to that of the bloodstream perfusion passage of the male penis, which reflects the comprehensive harden degree of the vascular passage. The data measured may predict the occurrence of the diseases or represent the therapy effect, and the level of the health keeping and life prolonging. |
| | 22 | Analyzing and calculating the variation of the vascular endothelial function | The vascular endothelial function represents the vascular elasticity, which is also a kind of variation of the endocrine, and has an explicit reference value for the variation of the heart function. Measuring and calculating and perceiving the vascular endothelial function is of an explicit significance to the disease prevention and prediction. |

(continued)

| Category | No. | Name | Explanation |
|---|---|---|---|
| | 23 | Analyzing and calculating the variation trend of the head bloodstream perfusion before and after you do sports | The head bloodstream perfusion amount accounts for about 30% of the human body bloodstream perfusion, and the bloodstream perfusion in the head cerebral cortex is able to provide the energy for the cognition and emotion; and the sleep and wakefulness, sports and stillness may cause the change of the whole body blood flow distribution, in particular the changes of the cerebral cortex blood perfusion. The effect of sleep and exercise to the brain blood perfusion changes the need of the brain for metabolism, and also represents the changes of the blood perfusion of the visceral organs. The change in the blood perfusion is one of the factors affecting health. By measuring the change in the blood perfusion before and after the sport, we may analyze the affecting degree of the sport to the health. |
| | 24 | Analyzing and calculating the variation trend in peripheral blood perfusion before and after the exercise | The change of peripheral blood perfusion, in combination with the change of head blood perfusion, is the necessary data for quantitative measurement of the effect of physical exercise to the health, and is also a quantitative method reflecting the change of vessel hard degree. |
| | 25 | Analyzing and calculating the changes in upper and lower limbs blood perfusion in the sleep of a night | The changes in upper and lower limbs blood perfusion in the sleep explain the change of angiosclerosis and the body blood perfusion value indirectly. The condition of the lower limbs blood perfusion has an effect on the athletic ability of the elder, and this measurement has a reference value for understanding the health status. |
| **Measuring and calculating blood glucose** | 26 | Analyzing and calculating your blood glucose variation trend within a specified period of time. | The measurement of blood glucose of the diabetic patient has a huge effect to the control of the blood glucose. We can find the best method for controlling the blood glucose by means of measuring the blood glucose and form the change trend and combining other life data and subject feelings, sport information, and the intake and output volume, etc. |
| | 27 | Analyzing and calculating the beat variation trend within a specified period of time | The number of the beats and the number of the beats during sports and rest are the main data for understanding the health status. The beat variation trend is also an important data for observing the gradient change of the heart function, and also the main content of the health compared between the individuals. |
| | 28 | Analyzing and calculating the number of the your premature beats within a designated time period | The number of your premature beats is the prediction factor for cardiac failure and coronary heart disease attack. The premature beats may be divided into the physiologic and the pathologic premature beat, and the number of the premature beats not greater than a certain value, and greater than a certain numerical value, can represent the degree of pathological and physiologic performances respectively.. |
| **Beat measurement and calcul ation** | 29 | Analyzing and calculating your heart rate variation within 24 hours | The heart rate variability represents the adjustment ability of your vegetative nerve function and the sensitive degree. The heart rate variability is one of the assistant indicators for the heart diseases diagnosis. |

(continued)

| Category | No. | Name | Explanation |
|---|---|---|---|
| | 30 | Analyzing and calculating your related change in the blood pressure and the heart rate within a specified period of time | The joint change trend of the blood pressure and the heart rate may reflect your adjustment ability to the circulatory function and the stress reaction adjustment ability; the change in the measured joint value of the blood pressure and heart rate is one of the performances of the health status and disease attack. |
| Measuring and calculating the body temperature | 31 | Analyzing and calculating your body temperature variation trend within a designated period of time | Measuring the variation trend of the temperature numerical value is mainly used in the evaluation of the treatment effect to the fever, and the disease prediction and prevention. The internal inflammation occurrence and continuous may be characterized by fever, and the occurrence of certain types of malignant tumor is also accompanied by fever. |
| | 32 | Analyzing and calculating the scatter distribution related to your body temperature and heart beats within a specified period of time | By measuring the body temperature and heartbeat, and forming temperature - the heart scatterplot, we can analyze the possibility of some serious complications such as myocarditis, thus we can avoid the danger due to misdiagnosis and the loss of important information. |
| Measuring and calculating sports | 33 | Analyzing and calculating your relative amount of exercise within 24 hours | Measuring and calculating the relative change in exercise, we may get the quantitative index reflecting the longevity and health status, and combined with heart disease measuring and examination, we can predict the possibility of heart failure and coronary heart diseases. |
| | 34 | Analyzing and calculating the relative exercise amount of your low limbs within 24 hours | Measuring and calculating the exercise amount of your low limbs may reflect the compression degree of the cervical vertebra and lumbar disc herniation to the nerve, and may also represent the objective effect on the rehabilitation process of lower limb activity of the paraplegia patients after stroke, which is also the quantitative measurements of the process development of the health keeping and life prolonging. |
| | 35 | Analyzing and calculating the relative exercise amount of your upper limbs within 24 hours | Measuring and computing the exercise amount of the upper limbs may reflect the compression degree of the cervical vertebra and the lumbar disc on the ridge column nerve, and may also represent the objective effect of rehabilitation process in the patients with hemiplegia after stroke, and is also the quantitative measurements of the development process of health keeping and life prolonging. |

(continued)

| Category | No. | Name | Explanation |
|---|---|---|---|
| **Sensitivity measurement and calculation** | 36 | Analyzing and calculating the sensitive degree distribution of your sense organ input within 24 hours | Measuring your sensory sensitivity represents the size of your brain's ability to eliminate the interference, and indirectly reflects the level of the concentration ability, or, degree of the sensitivity of response consciousness, sleepiness, drowsiness status, and the reduction degree of the reaction to the outside world. The measurement method is calculated by means of the electroencephalographic wave analysis. |
| | 37 | Analyzing and calculating your neuroregulation strength | The measured value of the plant nerve adjust tension may reflect the physiological reflex to the pain, stress, and the outside stimulus; and the greater the measured value is, the greater the change of your blood pressure, blood stream perfusion, and the heart beats to the stimuli are, which may cause the concurrent disease, and even affect the life. The less values show that your ability of neuromodulation is aging, weakening, and unable to find out some diseases of the body quckly and efficiently or will loss the vitality. |
| **Brain measuring and calculating** | 38 | Analyzing and calculating your habit of cerebrating and the left brain and right brain lateralization | The advanced functions of the cerebral cortex is mature gradually after birth, and the growing environment and learning environment exert a decisive influence on the development of cerebral cortex, and the left brain and right brain half balls of the cerebral are in either the cooperative, or the competitive relationship, and may complete certain functions such as cognitive and emotional functions relatively independently, and a mature and strong left brain can inhibit the development of right brain, and vice versa. Along with the growth, we can form cerebration habits. The left and right brain lateralization represents what kind of skills and emotions you are good at. In most of the situations, the advantage of the left cerebral hemisphere includes: language skills, management ability, communication ability and logical ability and happy ability. While the advantage of the right cerebral hemisphere: the creativity, the memory ability, the emotional ability, imaginary thinking, and the specialized skills. |
| | | | The measured value can show weather the left and right brain are developed coordinately, and severe cerebral lateralization represents that a certain disease or sub-health problem occurs, such as insomnia, anxiety, depression and fatigue. |

(continued)

| Category | No. | Name | Explanation |
|---|---|---|---|
| | 39 | Analyzing and calculating the current brain state (brain restraining, memory processing, drowsiness, relax brain, brain emptying, insomnia index, blink times, brain inhibition time , brain excited time, the time of opening and closing the eyes) | The measurement of the current brain state reflects the actual value of the current state of the brain, wherein, the brain restraining reflects the sensitivity degree of the brain sense organ signal input, and the concentration ability, the clouding of consciousness is directly related to the sleep. The memory processing shows that the brain is in the memory processing state, at this time, every function region of the brain handles with the data input into the brain in the synchronously coordinated way, thus forming the memory. The sleepy degree reflects directly that the current brain is in the sleepy state, and has a tendency of sleeping. The brain relaxation reflects the state of mental relaxation, and represents that the brain is in the inhibition state and the resting state. The brain emptying reflects the empty degree of the brain, at this time, the thought intensity is very low, and is similar to the thinking of nothing. The insomnia index reflects the degree of difficulty for sleeping. The high value indicates a difficulty in falling asleep. The times for blinking the eyes are the times of blinking the eyes in the measurement. The brain inhibition time is the sum of the time for the relaxation and the time of the brain emptying. The brain excitement time is the sum of the time for brain tension, outgoing, and communication. The time for opening and closing your eyes is the sum of the time of opening and closing your eyes measured. |
| Measuring and calculating the brain response speed | 40 | Analyzing and calculating your current brain reaction speed status | By measuring the brain's reaction speed, we may understand the brain's response speed to the outside information, and the changes speed of the brain from one state to another state may be related to the action and the tongue speed of people, and may be also related to the carelessness and fast speed in doing things. |
| | | | |
| Fatigue measurement and calculation | 41 | Analyzing and calculating the fatigue status of your brain | The measured degree of brain fatigue is the important data to know the work efficiency of the brain and the current state of the brain. The brain fatigue reflects that the brain is in an overload background in the recent period, which shows directly the reduction in the work efficiency and work ability, and reflects the people's health status indirectly, especially, the fatigue state may cause serious events such as the failure of viscera, sudden death, etc. |
| Measuring and calculating the energy dissipation of the brain | 42 | Analyzing and calculating the energy dissipation by your brain | The numerical value of the brain energy dissipation represents the work intensity and pressure of the brain, and anxiety, depression and insomnia often represent as the high brain energy dissipation; thus the changes in the energy dissipation of the brain may be used for predicting the energy supplying size of the body organs, which is in an inverse proportion to the value of energy dissipation, and is an important quantitative basis representing the health level recently. |

(continued)

| Category | No. | Name | Explanation |
|---|---|---|---|
| **Measuring t and calculating the inner attentiveness** | 43 | Analyzing and calculating the thinking changes condensed in your brain, and measuring the inner concentration | The concentration ability is the elaborative faculty of the brain, which is a strong degree of thinking of the brain focusing on something.<br>The brain is a whole, which consists of a lot of function areas, and handles with all sorts of cognitive and emotional information in a concurrent multi-task mode.<br>The inner concentration presents as a mode that the brain focuses on the internal cognitive and emotional thinking and inhibits the external information functional area.<br>The inner concentration may also be multiple tasks to be handled within the brain at the same time, or be known as with multiple excitement areas.<br>Inhibiting other exciting areas or excitement points and retraining an excitement point is the best concentration mode, and is also the thinking mode for solving science problems, technical problems and learning problems; and the stronger the excitation level of inhibiting other functional areas is, the higher the concentration degree is, the stronger the ability is, and the higher the achievement is.<br>If you are thinking about a thing, with the excitement of the negative emotional function area, that is the representation of anxiety and depression, and even there are many exciting areas in excitement in the brain at the same time, including the negative excitement, and worrying emotions and so on, which can lead to different external performances, that is the emotional performances.<br>Thinking something that must be thought of and you care about, and accompanied by the excitement in the excitement centers in left brain is happiness and anticipation.<br>The excitement accompanied by the excitement of the external sense feel organ function is the poor concentration ability, resulting in the failure to calm down to think.<br>At the same time, the excitement accompanied by the excitement in emotional center of the right brain and the negative emotions may be the anxiety disorder.<br>The external sense function being inhibited but accompanied by negative emotions is the representation of worrying and unhappiness.<br>Being unable to get rid of such thinking and negative emotions may cause depression.<br>If the excitement points are too much to be suppressed, we may suffer from insomnia, once insomnia occurs for more times, people will forget to sleep and become a stubborn sex insomnia patient. |
|  |  |  | And the more the excitement points are, the more blood perfusion is needed, and the more energy may be consumed.<br>So, concentration is influential to the health. |

(continued)

| Category | No. | Name | Explanation |
|---|---|---|---|
| | | | The best situation of the inner concentration is that only a question that you care about is processed, and only corresponding brain region is excited, or is accompanied by little excitement of a pleasure center of the left brain, this excitement is the best state for people's health and sleep, or is one of the best solutions for the science technology problem or the learning problems.<br>Both great joy and great compassion are all the performance not conducive to health and problem solving.<br>The measurement of inner concentration level in the brain is actually the measurement of the brain's ability to inhibit various functional areas, the measurement of your state of anxiety and depression, and also effective quantitative data for measuring the children's learning ability and the ability to work.<br>The deeper the inhibition is, the stronger the outbreak is.<br>This is the evaluation criteria for measuring the inner concentration. |
| **Measuring and calculating the outer concentration** | 44 | Analyzing and calculating the condition of your concentration on the outer information, and measuring the outer concentration | The outer concentration is equal to inner concentration, and the outer concentration is to the processing and thinking of the outer information.<br>The concentration is just to get external input information, and process, neaten, understand, answer the information and so on. Similarly, the outer concentration is also accompanied by the excitement or suppression of various functional regions, and different excitation and inhibition of various brain functional regions represent different emotional states and external behaviors, which may inhibit most of the functional regions, and solely focus on taking in external information, such as reading, writing articles, doing homework, listening to the teacher carefully, communicating with other individuals and so on, of course, the communication is also accompanied by the excitement of the language centre.<br>These are the best performances of the outer concentration, which also may be watching comedies, tragedies, accompanied by the excitement of the pleasure center of the left brain or the emotional center in the right brain.<br>This concentration may make the heart pleasant or release the emotions.<br>There is another kind of outer concentration, which is accompanied by the excitement in the sport center, and is often characterized by labor, physical exercise, and even fighting.<br>The same is that the stronger the inhibition not related to the brain is, the better the outer concentration is. |
| | | | Such performance on children may be the sensory which inputs the excitement and the sport centre is excited but is not excited in processing the information, which is just the embodiment of ADHD.<br>Measuring the quantitative values of the outer concentration can reflect your physical and mental states, the interpersonal communication, coordination ability, and the ability to learn and work. |

(continued)

| Category | No. | Name | Explanation |
|---|---|---|---|
| | | | The quantitative numerical expression of the ADHD, the autism, and so on may be related. |
| Measuring and calculating the brain activities | 45 | Analyzing and calculating the cerebral ability of cognitive orientation, and the reserve force to complete the task of the brain | The cognitive and emotional work of the brain is the advanced function of the brain. The better the brain rests, or the better the brain is inhibited, the better people sleep, the better the cognitive orientation ability of the brain is, and the stronger the ability to solve complex problems is, especially for the problem of thinking. The quantitative value measurement of the brain activity may represent whether the brain in the current state has enough ability to accomplish a complicated task, how much the cerebral cognitive and affective ability are reserved. For the children and the elder, the brain activity is very poor, with the measured value being very high. This shows that the brain development of children has not been completed, thus they can't use the brain to complete cognitive tasks correctly. The elder, whose brain is aging, atrophy, and even suffering from the Aalzheimer's disease, are also unable to accomplish a specific cognitive task. For the youth and the adults, if the brain cannot fully rest and be inhibited, for example, after staying up late, the brain activity would significantly reduce, the problem-solving skills cannot be assured, and the brain has no reserves. Thus, the brain activity can reflect the brain's aging degree and the development degree of children's brain, and whether the young and the adults are in the condition of the best ability to complete the task. |
| Sexing function for the male | 46 | Analyzing and calculating the times of telotism in the sleep of a night | For male impotence, the functioning erectile dysfunction is one of the problems affecting the quality of life. By measuring the telotism times in the sleep of a night, you may actually understand whether the telotism function is normal, and know whether the problem is due to psychological reason or due to the physical organs, and provide the analysis data for the treatment and improvement of the quality of sexual life. |
| | 47 | Analyzing and calculating the most pressure of the telotism in the sleep of a night | Telotism is due to penis congestion, because the value of the pressure generated for the penis congestion is one of the data indicators for the penile erectile function quality, by which we can understand your heart health measurement and the physical aging trend. |

(continued)

| Category | No. | Name | Explanation |
|---|---|---|---|
| | 48 | Analyzing and calculating the relative blood perfusion when the penile erected in the sleep of a night | In the sleep of a night, the penile erection is accompanied by the rapid increase of the blood f perfusion, thus measuring the increase of blood perfusion, and the blood stream morphologic change of the blood flow perfusion wave when the penile is erectile may reflect the function status of the telotism; and also reflects the condition of the reaction vessel elasticity, especially reflects the heart pumping function and can be served as the prediction of the heart disease attack. |
| | 49 | Analyzing and calculating the relative relationship between the telotism and the sleep structure | Telotism occurs naturally during sleep, and the normal penis erection generally occurs in the fast phase sleep, that is in the dream. Measuring the relationship between the telotism and the sleep is to analyze the main data for analyzing the change in the erectile function, and is also the main data to judge the cause of erectile dysfunction. |
| **Historical information of seeing the doctor** | 50 | Analyzing and calculating your subjective health feeling within a designated time period | The feeling on your own health in daily life is an important content in the disease diagnosis, when seeing a doctor, we need to describe our own state, that is the chief complaint, but which is often in a form of memory, and is very difficult to be accurate and complete. Recording various subjective feeling of discomfort in real time, writing down the detailed and true self feeling and the corresponding events are of a great value for the disease diagnosis in the future. |
| | 51 | Analyzing and calculating the variation trend of the biochemical examination in your medical diagnosis within a designated time period | The medical diagnosis process is actually the process of examination and test, during which many data may be generated, especially the biochemical data. The data are quantitative objective data, and can be generated in every diagnosis; by making these biochemical inspection data measured into a trend chart change curve according to the time coordinates, we may understand biochemical change in life intuitively, and it is of an important significance for the health keeping and life prolonging and the disease diagnosis. |
| | 52 | Extracting the prescription and the content of the medical case record displayed in all the previous medical records within a specified time period | During the diagnosis process, a lot of medical books, including the medical record, the doctor's advice, prescription, and so on may be generated; and saving and reading and viewing the medical books, and extracting the prescription and the treatment information in the doctor's advice, the hint and alarm may provide a complete medical track and diagnosis reference for the disease rehabilitation and development. |
| | 53 | Extracting the content of the image examination displaying in all of the previous medical records within a specified time period | The imaging examination is also the medical treatment information generated during seeing the doctors, which is also the reference data for the health, and is also the important diagnostic reference data when going to a doctor next time. |

(continued)

| Category | No. | Name | Explanation |
|---|---|---|---|
| **Antenatal health care** | 54 | Analyzing and calculating the change of the fetal heart for the pregnant woman within a designated time period | Measure the fetal cardiac wave shape of the pregnant woman, record and review at any time, and go to a doctor for medical treatment timely once a problem is found. |
| | 55 | Analyzing and calculating the time of quickening in a designated time period | Measure the number of the quickening, record, and generate the trend diagram, review and observe at any time, and go to a doctor for medical treatment timely once a problem is found. |
| **Entering the medical clinic information** | 56 | Medical health information electronic bank storage services (free of the activation fee) | All of the medical clinic data and the subjective feeling data, the physiological and the vital signs data, some biochemical data (may be home-use), the sports momentum, and the data on input and output volume may be electronically stored and read. The way of reading is using the individual account under the management of the Internet web site and using the user name and password to enter. |
| | 57 | Electronic collection service of the ordinary outpatient medical information generated in the medical clinic | All sorts of medical information generated in the outpatient service, including the medical trajectory such as doctor prescription, test documents, medical record, image documents, fees detail, and so on, may be input into the corresponding storage unit under the personal health bank account for long-term storage. |
| | 58 | Electronic collection service of the emergency treatment information generated in the medical clinic | All sorts of the medical information generated in emergency treatment, including the medical trajectory such as doctor prescription, test documents, medical record, image documents, fees detail, and so on, may be input into the corresponding storage unit under the personal health bank account for long-term storage. |
| | 59 | Electronic collection service of the ward medical information generated by the hospital treatment of the medical treatment | All sorts of medical information, including the medical trajectory such as doctor prescription, test documents, medical record, image documents, fees detail, and so on generated for hospitalization (not including the surgery and intensive care clinical information), may be input into the corresponding storage units under the personal medical health bank account for long-term storage. |
| | 60 | Electronic collection service of the surgery diagnosis and treatment information generated in the medical clinic | The medical trajectory including all kinds of medical information generated for the surgical treatment, including medicine, operation consent, preoperative visit, postoperative follow-up, anesthesia record sheet and nursing record sheet, cost detail and so on, are input into the personal health of the corresponding storage units bank accounts for long-term storage. |
| | 61 | Electronic collection service of the ICU (intensive) care and treatment information generated in the medical clinic. | The medical trajectory including all kinds of medical information generated for the intensive care treatment, including drug treatment, medical record, laboratory test, the nursing records, nursing procedures, fees detail, and so on, are input into the corresponding storage units under the personal health bank account for long-term storage. |

The human body not only possesses resistivity and immunity, but also possesses a strong potential force. It was reported that, a 12-year-old girl moved the piano out of the house by herself when the room is on fire, however, in normal time, such forces will never be stimulated by her own autonomous consciousness. It is demonstrated that the latent capacity and the subconsciousness exist objectively in the human body. It may be supposed that, the human body has forgot or hasn't learnt how to mobilize these potential capabilities, while the brain's learning capability is inherent, and by using such an ability, the brain can feel the change of the human body information about life in real time, and repeat this process, thus the brain may learn how to control the information, stimulate some potential ability of the body represented by the information.

The mode of the big closed-loop feedback diagnosis and treatment platform is a real-time large system with transparent spatial distance under the background of big data and cloud computing, which connects the senior cortex center of the human brain, a physiological system and a life data acquisition terminal, a wireless Internet platform, a computer, a mobile communication display equipment, a robot and an executing equipment. It is also a multiple input and multiple output system. It may gather the life data in the context of wireless internet, subdivide the types of the life data and process by calculating, extract the characteristic index, and transform these data into the sensory information of the brain through the Internet and wireless terminal equipment. The brain perceives the change information about life data in real time, and repeats the process constantly, thus forming the method and the skill on how to control the data change under the independent consciousness, and learning the ability on how to consciously change the working process of the body organs which is forgotten by the human body or which has not been learned by the senior center of the cerebral cortex, and the ability of mobilizing the subconsciousness of the brain and stimulating some potential in the body represented by the data. This kind of perception is a process of standardization and may be suitable for all people. Firstly, it needs to dig up the life information content representing the health and disease treatment effect. The information should be measurable, real-time and quantitative, and may be applicable to and repeated on all the people. These information come from the human body, come from various medical diagnosis check indexes in the medical diagnosis and treatment effect evaluation, and exist in the way of quantitative data. The changes of these indexes may be the judgment criterion for doctors to judge the disease and the health. By stripping these life examination indexes from the hospital medical behavior, in addition to using them as the quantitative information for the medical staff in the diagnosis of disease, the life examination indexes can be used as the core content of people's daily entertainment, sports competitions and risks. Secondly, after the living-at-home collection, the Internet transmission, the cloud computing center calculation, the characteristic index generation, and after the big closed loop processing of the entertainment, and athletics transformation of the characteristic indicators, these indexes become the tool used in home entertainment, sports competitions and exciting adventures which can be utilized repeatedly, consciously and pleasantly. The course of entertainment and game is accompanied by a reward system, the brain itself runs the reward mechanism principle in the physiological sense; thus by using and adapting to this principle, the brain may create a kind of pattern integrating the process of treatment, rehabilitation, prevention and longevity into people's instinctive behavior of being keen on the entertainment and adventure and pursuing the reward.

The games and sports competitions include the real object mind control and the web computer games. The used presentation platforms comprise a computer and a cell phone, wherein, the computer may be a mobile tablet, a mobile computer or an immobilized computer, etc. The used life information collection device is a portable wireless mobile data collection and transmission terminal; and according to different purposes of disease treatment, rehabilitation, prevention and health keeping and life prolonging, we may collect different vital signs, extract different character indexes for calculation, point to different disease development changes and health levels, and select different terminals, but the conversed entertainment game carriers are the same; thereinto, the web computer games are mainly used for training, improving the skills, of which the electronic games are the main presentation form, including music, flashing light, the change of the sight and smell. While the main presentation content of the real object game is to control the walking routes and speed of the physical robot, which are mainly used in sports competitions and daily training. The web game realizes the game process on the personal computer or the mobile phone directly. The game process of the real object games are also implemented in the Internet via a personal computer or a mobile phone, but the real robot is set in the robot control center and managed by the service provider; the game process adopts the way of the video broadcasting, and may display a panoramic video of mobile robots moving on the personal computer or the mobile phone. The users may be distributed in any place within our country, and the using conditions are identical for the two games, that is to wear a wireless mobile life data collection and transport terminal to access to the Internet and the website, and start to complete the game control process of the closed loop thinking via the life data collection, the wireless transmission, the cloud computing index extraction, the feedback control to disperse the web games or the real object robot action. The real object robot control center sets the number of the robots according to the number of users and assigns the robot to a user with 30 minutes as a machine-hour. A service center of the data calculation is matched with each center to manage 256 or 512 wireless mobile life data collection and transmission terminals. 8 to 16 real object robots need to be matched if 32 machine hours are in calculated in a day. The reward mechanism is an independent driving factor in the whole process of disease treatment, rehabilitation, prevention, health keeping and life prolonging, and is also one of the conditions accompanied by a positive mood during the process. The game process is a positive mood process making person's mental and psychological moods cheerful. Along with the gradual ending of the game process, the process is also accompanied by the improvement of health indicators, and the acquisition of the capability of the independent cognitive function in controlling the change of the life data. The reward exists in the form of integrates which may be accumulated unceasingly, of which the scoring method is on the basis of the change of the life and health indexes, and which may be exchanged into a prize as the goal of the people.

Excitement and adventure have always been the nature of people. In the application process of this life maintenance mode, the properties of excitement and adventure are embodied in the introduction of the reward mechanism of competitive games, which embodies the attributes of gambling, where people use the skill and ability of finish the game better and faster to participate in the competitions, and with the opportunity to win great wealth. All the methods of gambling such as the chip, the bet and the like may be used in this application process by the monolithic movement. The control game to the real object robot is the main carrier of the sports competition, which is implemented in the form of inviting the rivals online, allowing the viewers to bet on, and win the points, thus forming a humanitarian corridors of non-charitable donations where the healthy person helps the patients in the treatment and rehabilitation process. The service providers hold the prize competition at regular intervals, which may be divided according to the areas and the time periods, and of which the prize values may be distributed from low to high, and collect the financial resources produced by modern media propaganda such as sponsorship, advertising, etc., and use the resources in the actual action of disease treatment, rehabilitation and health keeping and life prolonging. The real object robots include the mobile robots and the real object cars, and the winners may even win a real car. The competition may be divided according to the order and the kinds of diseases, but regardless of their age, and no matter they are male or female. All the people including men, women, the elder and children may be allowed to participate in the competition, no matter whether you are ill or healthy, no matter you are an adult or a child, you may become rivals, and the equities in age, sex and health conditions are not needed. As long as using the control ability based on your own certain life and health indexes to participate in the competition, it is reasonable to win the game, which embodies the characteristics of fairness, wide range and universality and privacy. The competitors may be hierarchical, and the hierarchical principle considers the factors of age, health, and diseases. The basic hierarchical rule is that the older the age is, the poorer the health condition is, or the more serious the disease is, the higher the corresponding level is. The rules of the game are with weighted attributes, that is, the higher the level is, the higher the weighted coefficient for the score is, which embodies the concept of the disease treatment and a preference in children and the elder. The two parties of the competition may be an individual, and may also be a group, and the individuals in the group may adopt their control specialty for a certain life data to control a motion dimension of the real object robot, for example, forward, backward, turn left, turn right, low speed, high speed, move the left arm, move the right arm, and so on, may be controlled by different people independently (or a person's full control), everybody may complete a specific thinking control task by means of cooperation. The game participators may be distributed in their respective living place, are not needed to reach the real robot control center, and can complete the sports competition through the wireless at anytime and anywhere. The prize competitions held by the Internet service providers regularly need the participants to take part in the game on the scene, thus reflecting the purposes of reality, objectivity, fairness and influence. By means of the learning of this continuous life data control ability and skill, and the sports competition, and in a state of happy and stimulated positive psychological mood mode, we may learn to control the life status index under independent consciousness, along with promoting the traditional treatment curative effect. We may learn the ability of autonomous disease treatment and stimulation of the body's potential ability to fight against disease, even may learn some specific potential survival technique and the knowledge acquisition ability, and may comprehensively improve people's wisdom and moral quality.

For using the advanced cognitive abilities of the cerebral cortex and the autonomous consciousness to control the change of life data, the main action object is the brain, which acts as the central control unit and may realize the function of life maintenance. Aiming at the physical adjustment and control technology of the brain function and disease, at present, in addition to the development of the relevant brain magnetic stimulation technology and electric shock technology in the application of psychiatric disorders, the application of the micro electric current constant stimulation technology has begun to be reported. At the same time, the open loop sound and light stimulation products using the electroencephalographic wave resonance concept also has been advertised in the treatment of sub-health applications. But most advertisements are the false science or the hyped products misappropriating the partial results concept of the scientific research. The products on the technology to realize the closed loop feedback control of the brain state by the acquisition of the electroencephalographic wave signals have been available, but have no refined state index as the basis, especially the characteristics indicators on the medical grades haven't been extracted, thus no real large-scale application has been made. A problem existing in this treatment and adjustment is that perhaps, no real-time quantitative monitoring to the brain state results has been implemented in the process of treatment adjustment to judge the effect, and change the stimulation output, or perhaps the inaccurate measuring characteristic indicators results of the brain state signals results in the decrease of the efficiency of the treatment adjustment, and even that the results may be inverted and lead to adverse consequences. This is the important reasons that the closed-loop feedback treatment technology hasn't accomplished an effective application in the field of brain state regulation for decades.

The method controlling the life data change by the autonomous consciousness may be seen as the implementation of the brain state traction technology, one of the key application bases is the state control to the brain itself, which mainly relies on the collection of the brain state signal and the extraction of the quantitative index. Under the condition of objectively and quantitatively reflecting the current state of the brain status, applying an external stimulation of the sensory signals may allow the human brain to understand the state change process of the brain itself in real time, thus learning the explicit regulation method to the inhibition and the excitement of the brain through adaptive and learning ability of the brain. The providing way of the stimulus is different from a lot of open-loop brain stimulation modes at present (most of these ways give a fixed set of stimulation process in the absence of the brain receptor, and are unable to identify the change rule of the brain in the stimulated status, and the effect achieved may be limited, and the effect tends to be offset by the adaptability of the human body and is unsustainable), which uses the quantitative characteristics indicator of the brain state as the brain perceptor to drive the control process and drag the changes of the brain state. The external sensory stimulus is divided into: sound, which includes music and the brain- machine language dialogue; light: which includes the visual frequency stimulation and the light wave traction; the body sense: which includes the body position changes and the changes in skin sensation. The stimulating ways are mainly presented as the game in client terminal eventually, which are divided into the operability game and the sensibility game. The game process relies on the changes of brain states, for example, the left cerebral hemisphere and right cerebral hemisphere coordination index may drive the game, increase the level of excitement of the left side of the brain, and reduce anxiety and depression, or improve the excitement level of the right left cerebral hemisphere, and enhance the learning and memory abilities. The operability games are mainly the electronic games, and also include the real object games, for example, the brain state indexes drive he rotation the brain SiNa spoon handle, and drive the robot movement, and so on. The sensibility games are mainly in the form of feeling by closing the eyes, including the brain-machine dialogue, and the relaxation in the music, and so on. All of the stimuli are time-varying, and change along with the change of the brain status. In a similar way, the principle by which we use the life data into the disease treatment is the same as that used in the traction process of the brain state, and is also accompanied by the traction process of the brain state. By means of the changes of brain state, the other life data changes may be controlled. The maintaining items which may provide the life data control are as follows:

| Life information independent treatment and the disease prevention | 1 | Vascular elasticity motion control learning game under the independent consciousness | The vascular elasticity is related to the occurrence and development of cardiovascular diseases and cerebrovascular diseases, and angiosclerosis is the primary cause. Promoting the vasomotion to make it expand and contract may soften the vessel, promote the blood circulation, and reduce the blood pressure. |
|---|---|---|---|
| | | | The learning of the blood vessels elasticity movement is implemented under the quiet state without body telecontrol, which influence the expansion and contraction of the blood vessels by means of the ability of autonomous consciousness (this effect has existed, only you don't even know it). It makes great contribution to the angiosclerosis; once learning the blood vessels elasticity movement game, we also learn the ability of allowing the blood pressure stable, controlling the vascular expansion and contraction under the autonomous consciousness at any circumstances, and improving the occurrence and development of cardiovascular disease. This kind of process of learning thrombus needs to follow the doctor's advice. |
| | 2 | Learning game of cortex inhibition under the independent consciousness and dormancy control | By means of learning the method to make the cerebral cortex inhibit the dormancy under their own independent consciousness, and learning to enter into the stage of inhibiting the dormancy at any time, we may learn to enter into the sleep stage at any time, enter into the stage of the whole body blood flow redistribution, and thus harvesting the results of longevity and health. |

| | | 3 | Independent consciousness perception sleep relearning, insomnia treatment learning game | For patients suffering from insomnia, the ability of sleep has been forgotten. To complete the ultimate treatment of insomnia, it is needed to input the sleep state to the feeling channel of the insomnia patients repeatedly, and to form the relearning process of sleep under the active consciousness. Thereinto, the most important is to make the learners learn how to control too much excitement center of the cerebral cortex, and to suppress the excitation degree of the cognitive emotional area including each cortical functional area system gradually, including the listening, watching, speaking, somatosensory, motion and positive emotions, negative emotions, and so on. |
| | | 4 | Learning game of the cortex motion relearning, neural network reforging under the independent consciousness | For the stroke patients, the damaged portion of the cortex results in the body movement disorders, hemiplegia or paraplegia. The cerebral cortex nerve central possesses plasticity, which may form new compensation function regions or the new neural network reflex arc to control the movement of the body again. This process is very long or with occasionality. By means of the movement of games, mainly the movement of real object games, perceive the reengineering of the neural network reflex arc, and perceive the promotion and interference of the independent consciousness to the construction of the reflex arc. This may speed up the process greatly. Especially, by combining with the conventional means of rehabilitation medical treatment, relearning by movement under consciousness, and repeated imagination of the body movement, the change of the real movement on the senses happens. Control the imagination method to allow the movement of the object to conform to the imagined result, and thus we may learn the method of independent consciousness to promote the establishment of the reflex arc. |
| | | 5 | Learning game on the oxygenation efficiency of the respiratory control under independent consciousness | Learning how to control respiration may allow the pulmonary blood stream exchange to achieve the highest ratio of the oxygen composition, thus exchanging the highest ratio of the carbon dioxide composition, and improve the efficiency of every respiration. |
| | | 6 | Vital capacity motion learning game under independent consciousness | Learning how to achieve the maximum vital capacity. International research proves that the greater the lung capacity is, the longer the service life is. Through this kind of learning, we may learn how to control the respiration and accommodate the most air in the lung. |
| | | 7 | Sympathetic nerve tension adjustment learning game under independent consciousness | The autonomic nervous system is the major nervous system regulating the normal work of human organs directly, wherein the sympathetic nerve is the main adjustment channel for the stress reaction of people and is related to a person's mood, blood pressure, heart rate, sweating, etc. Learning the method of raising or lowering the sympathetic nerve tension under independent consciousness is helpful for learning the ability to handle with matters in calm when encountering with accidents. |
| | | | | The characteristic indicator of the sympathia tension adjustment comes from the peripheral blood perfusion index. |

(continued)

| 8 | Blood circulation control learning game under independent consciousness | The influence of the blood flow distribution in the human body on the human health is absolute, and that on the people's cognitive ability is also absolute. To learn to control the distribution of the blood flow in the whole body is one of the basic methods to treat the disease correctly and realize the disease rehabilitation and the longevity and health. By learning how to control the excitation and inhibition in different regions under independent consciousness by means of the cognitive function of the cerebral cortex and change the relative changes including the head blood perfusion and the limbs peripheral blood perfusion, we can control the distribution of the composition carried by the blood stream oxygen and the immune cell components in the body, in particular for the disease treatment phases, the blood stream distribution tends to affect the continuation of the life indirectly. |
|---|---|---|
| 9 | Learning game of perceiving the temperature adjustment control learning game under independent consciousness | Perceive the local surface temperature of the body and change the body temperature by using independent consciousness. Improve the local blood perfusion and improve the local surface temperature. The change in the temperature represents the change in the blood perfusion, and may improve the local blood flow, and thus achieving the goal of adjusting the health. |
| 10 | Learning game of perceiving the kinetic energy of the lower limbs under independent consciousness | During the course of the rehabilitation process of the lower limb paralysis patient, the lower limb motion kinetic energy measurements may reflect the changes in the rehabilitation effect. Perceiving the movement kinetic energy change of the lower limb may contribute to control the rehabilitation process under independent consciousness, and by means of learning how to think, we may control the constant increasing of the kinetic energy. |
| 11 | Learning game of perceiving the kinetic energy of the upper limbs under independent consciousness | During the rehabilitation process of the upper limb paralysis patient, the kinetic energy measurements of the upper limb movement reflect the changes in the rehabilitation effect. Perceiving the kinetic energy change of the lower limb movement may contribute to controlling the rehabilitation process under independent consciousness, and learning how to think to control the constant increasing of its kinetic energy. |
| 12 | Learning games of perceiving the head blood perfusion metabolic demand under independent consciousness | The head blood perfusion is related to the head oxygen metabolism level; if the head oxygen metabolism demand is very high while the head blood perfusion can't satisfy this demand, insufficient brain blood supply may occur, and which may result in a series of results including headache, dizziness, vision ambiguousness, language barrier and so on, so improving blood perfusion and increasing blood supply are the solution to the problem. At the same time, learning to lower the head oxygen metabolism demand to adapt to the head perfusion is also a solution to the problem. Sleep and coma are generally the results of the falling head oxygen metabolism demand, including meditation, static state, concentration, and so on. Once learning to restrain the brain excitement point, then we learn to reduce the cerebral metabolic demands. The characteristic indicators for expression refer to the cerebral state index and blood perfusion index. |

(continued)

| | 13 | Learning game of perceiving the peripheral blood perfusion metabolic demand under independent consciousness | As the above-mentioned, the peripheral blood perfusion is related to the level of the body oxygen metabolism, and thus learning how to reduce the metabolic demands is of a significant value to reduce the burden of the heart, to accumulate energy, and alleviate cardiovascular diseases and cerebrovascular diseases, especially to tolerate the low oxygen environment and the low blood perfusion in the state of disease, and contribute to people's longevity process. The expressed characteristics indicators are the same as the above. |
|---|---|---|---|
| | 14 | Blood pressure regulation learning game under independent consciousness | Learn to control the level of the blood pressure by independent consciousness. The emotional effects on the blood pressure is clear. Mood is the human senior recognition ability, and the ability to achieve the stable blood pressure initiatively and consciously may be achieved by means of repeated consciousness and cognitive learning. The expression indexes are the blood stream perfusion index of the middle blood vessels and the microcirculation. |
| | 15 | Heart rate regulation learning game under independent consciousness | As mentioned above, the heart rate is also affected by the emotion. Learning to control the mind and emotions and adjust the heart rate is important conditions to health keeping and life prolonging. The expression characteristics index is the heart rate. |
| | 16 | Perceiving the brain inhibition adjustment learning game under independent consciousness | By perceiving the brain inhibition degree under the independent consciousness, you can learn how to control the excitement degree of the functional areas of the cerebral cortex, and learn to make your brain sleep, and to reduce the oxygen consumption of your brain, you can also learn to shut down the sensory input of the brain, and to dispel the interference of the external sound and light to go into sleep faster, or go into the meditation and static state, and so on. |
| | 17 | Perceiving the brain activity learning game under independent consciousness | The brain activity decrease is one of the consequences after the brain excitement for a long time, and brain aging may be accompanied by the brain activity decrease which often means that the cognitive orientation force decreases, and the brain's ability to solve the problems decreases. By perceiving the change of the brain activity, we can understand the cognitive readiness state of the brain, know whether the brain is overused, and know whether the use of the brain affects the health. By means of controlling active thinking we can learn how to improve the brain activity, improve the cogntive orientation, and learn the cerebral metabolic requirements indirectly. |
| | 18 | Perceiving the brain reaction ability learning game under independent consciousness | The brain reaction ability refers to the speed size of the brain state changes, which is expressed as the distance of the cluster in the i_22 and i_35 scatter plot. The brain response ability is often related to the speed, the motion speed and the work speed; and perceiving the change of brain response ability under independent consciousness may contribute to improving the sleep, improving the brain control ability, and improving the reaction speed to the outside world. |

(continued)

| | | | |
|---|---|---|---|
| | 19 | Sense organ input sensitiveness learning game (brain restraining) under independent consciousness | The sensory input sensitivity of the brain refers to the threshold value of the external information input, the brain in the low threshold is easy to accept the outside information input, and is also often vulnerable to the interference of external information. The brain in the high threshold value may be blunt, sleepy, but may also show a strong ability of concentration. Learning to adjust the sensitivity of the sensory input actively under independent consciousness may help to learn how to eliminate the interference from the outer environment, learn how to concentrate the mind, learn how to go to sleep, or how to accept the external communication information with more concentration. |
| | 20 | Perceiving the inner concentration adjustment learning game under independent consciousness | The inner concentration reflects the concentration state of the brain condensed in the internal thinking. The inner concentration is also a kind of thinking ability. The too-long-time inner concentration means excessive thinking. Besides the thinking aimed at the scientific problems, anxiety and depression may arise, and the continuous inner concentration will affect our health. |
| | | | Through learning to rest the brain under independent consciousness, and to lower concentration level, you may learn the ability to concentrate at any time, and learn to switch the state of concentration and resting freely, that is, learn how to improve the quality of the concentration. |
| | 21 | Perceiving the outer concentration adjustment learning game under independent consciousness | As the above-mentioned, the outer concentration is similar to the inner concentration, merely focusing on the reception of the external information. The too high level of the outer concentration generally means restlessness, tension, and fear; and thus learning to decrease the outer concentration level under independent consciousness means learning the ability to control the emotions and stress reaction, learning to close the sensory input, and indirectly learning the ability to sleep. |
| | 22 | Controlling the brain relaxation learning games under the independent consciousness | The relaxation ability is a good brain function state people pursue, and is also a kind of normality which should be possessed in life. In order to learn to relax, people adopt many methods, including the respiration method, the music method, and massage method and so on. The most direct method is to learn to control the relaxation of the brain with independent consciousness, to feel the characteristic indicators of the relaxation directly, and to adjust the metal state of the brain constantly, thus achieving a deeper level of relaxation. |
| | 23 | Perceiving the memory and processing learning game under independent consciousness | Memory processing is a kind of particular state of the brain, and is a state of closing the sense input before the sleep or just the state of closing the eyes after awakening. The autonomous consciousness perceives the memory processing repeatedly to control the active thinking and learn to enter this state, then learn the way to improve the memory, and be able to delay the memory failure, and delay the aging of the brain, even be able to realize the adaptive learning model of knowledge acquisition automatically. |

(continued)

| | | | | |
|---|---|---|---|---|
| | | 24 | Brain stability control learning games under independent consciousness | The brain stability is a kind of ability of the brain to maintain a state, such as the state of closing the sensory input, relaxation, memory processing state, meditation and so on, which is a cluster of points gathering together presenting on the scatter plot. Perceive the stability, learn to adjust the thinking initiatively, and control the stability state of the brain, which are more conducive to the health, and more conducive to the learning of knowledge after a long time. |
| | | 25 | Brain emptying control learning games under independent consciousness | Learn how to empty the brain, and make the brain in a state of empty-bright as far as possible, and try not to cognize and think, then we learn a method to make the brain sleep. The learning process is a repeated brain emptying process of the independent consciousness perception, which can adjust the active thought, we should learn to empty the brain. |
| | | 26 | Left cerebral hemisphere control learning games under independent consciousness | The left cerebral hemisphere lateralization is a usage habit state of the brain. Generally, the left cerebral hemisphere lateralization is not easy to produce the tendency of anxiety and depression. The left brain lateralization may strengthen the function of the functional domain distributed in the left brain, including logical thinking, happiness, languages, sports, etc. By perceiving the left brain excitement continuously, and controlling the active thinking, we may learn the thinking method of making the left brain excite, where the excitement of the left brain may result in the inhibition to the right brain (the left and right brains are in a competition relationship), thus we may learn the left brain lateralization, and also learn a certain skill that the left hemisphere is responsible for. |
| | | 27 | Right cerebral hemisphereadvantage adjustment learning game under independent consciousness | |
| | | | | The right cerebral hemisphere lateralization is a usage habit state of the brain. Generally, the right cerebral hemisphere lateralization is easy to produce the tendency of anxiety and depression, which may strengthen the function of the functional domain distributed in the right hemisphere, including the function of creativity, memory, imaginable thinking, emotion, etc. By perceiving the right brain excitement continuously and controlling the active thinking we may learn the thinking method of making the right cerebral hemisphere excite, where the excitement of right cerebral hemisphere may results in the inhibition to the left cerebral hemisphere (the left cerebral hemisphere and the right cerebral hemisphere are in a competition relationship), we may learn the right cerebral hemisphere lateralization, and also learn a certain skill that the right hemisphere is responsible for. |

(continued)

| 28 | Controlling attention recovery learning games under independent consciousness | The attention deficiency is one of the influence factors to the children ADHD, thus realizing the attention regression, and is also one of the ways to adjust the children ADHD. For the method of learning the attention regression using the children's instinct of hyperactivity, the characteristic index expressed is the inner concentration attached to the moving of the object, only by means of focusing on the object, the object may be moved; and thus by repeating the process of game, we can learn the thinking method of attention regression. |
| --- | --- | --- |
| 29 | Perceiving the meditation controlling learning game under independent consciousness | The meditation is a kind of concentration behavior, accompanied by the inhibition state of the other brain regions, except only an excitement point of meditation. Perceiving the degree of the meditation is helpful for learning and training of the meditation directly. By perceiving the meditation using independent consciousness actively, we can learn the correct way of meditation, and the correct way to control the thought. |
| 30 | Perceiving the quick brain fag recovery learning game under independent consciousness | After a period of mental activity, the brain may fall into a fatigue state, and may perceive the brain fatigue, and learning the fast fatigue recovery under the state of independent consciousness is a method to improve the work efficiency and ease pressure. To control the brain fatigue, we may use the brain restraining, brain activity and brain lateralization index. |
| 31 | Learning game on controlling the move the object with the ideal under independent consciousness | Thought controlling the movement of the entity material refers to the conversion of the change of the brain state into the control command to the movement of the objects, thus implementing the object movement by the independent thoughts. The used brain state may be any measured characteristic indicators. By means of learning to move things by thought, we may learn to control the corresponding brain state. |
| 32 | Remote control game to the sleep at fixed time | We may implement the control game of moving things by means of the thought by using the Internet. For the big closed loop mode, the control index of moving things by means of the thought is the characteristic index on the inhibition state of the brain after the game finishes, the brain may enter into a sleep state. |
| 33 | Living-at-hom e, and remote control competition to the sleep at fixed time | In the athletics competitive race on the remote and living-at-home control to the object moving by the thought, we can use any item of the cerebral state index as the content of the mind control to remote control a mobile object, to learn to control the objective changes of some brain state. This control is valuable for the longevity and health and disease treatment. |
| 34 | Real time client-provider interaction service for the private and remote psychological and metal disease diagnosis and treatment | For the diagnosis and treatment mode of remote interaction, the real-time interaction between doctors and patients suffering from the mental illness and the psychiatric illness may realize the living-at-home disease control and services in communities. |

A new way, a new conception, a new system, a new industry, a new thought, a new mode, a new field, a new procedure, a new process or a new behavior on health keeping and life prolonging, disease diagnosis and treatment, disease rehabilitation and disease prevention can be developed by utilizing an independent consciousness life data therapy. A process of making people healthy and have a long life is changed into a money-earning process, a money-earning behavior or an industry; the value and wealth is created by himself in the process of making people healthy, and people are willing to concentrate on persistently participating in the process; along with happiness, stimulation and pleasure, the health keeping and life prolonging, the disease rehabilitation and the disease prevention are realized, and the possibilities of award gaining and wealth accumulation are increased; as a result, a positive healthy medical treatment atmosphere, the scientific and advanced health guidance, the universal education on medical knowledge and the training on disease treatment and disease prevention can be created. As people pursue the stimulation and the wealth, and have indomitable spirit and strong driving power when facing great temptation, the disease is objectively healed and prevented, and the health keeping and life prolonging are realized. Meanwhile, the gaining of materials and money is achieved, together with information consumption and all the attributes of the modern service industry.

It is important that because of the psychological effect of people, diseases and rehabilitation can be greatly affected; therefore, the side effect on rehabilitation and disease treatment is generated; doctors in the medical field has always sought for a solution to eliminate the poor effect of psychology on diseases and health. To build a new human body environment, a positive effect on the treatment and rehabilitation of diseases by the psychological effect is realized; a new channel is developed, a new health driving system is built, and in the processes of disease treatment, health keeping and life prolonging and disease rehabilitation, the influence of psychological factor on the effect is changed into positive energy, the worry and the fear from disease treatment is changed into happiness, stimulation, concentration and a behavior of consciously keeping on. Under the pleasant and concentration atmosphere, the over-concentration on the disease can be put away; instead, the recreation and stimulation mentality can be satisfied by utilizing the disease treatment and rehabilitation process, and it is predicted that a great benefit on the disease treatment and rehabilitation can be realized.

Secondly, the process of recreation, risk stimulation and pursuit of great wealth temptation is a scientific behavior of disease treatment, disease prevention and health keeping and life prolonging, so that the effects of treatment and health keeping are enhanced. People have a natural instinct of pursuing recreation, wealth and stimulation, in particular to recreation, wealth and simulation risk behaviors with reward systems, which results in that a focus and persistent training process is formed so as to improve the entertainment ability and the risk ability; under the stimulation of possibly gaining great wealth, a process of learning and training, risking, making a failure, learning and training again, risking again, making a progress and having a small gain, learning and training again, risking again, making a failure or success again is easily formed; the learning and training contents, the processes and the gained ability and skill are the ways and the results of disease treatment, disease prevention and health keeping and life prolonging; the strong driving power for immersing people into some hobbies is transplanted into health promotion to produce a positive function, and because of the positive function, an unimaginable great progress on medical modes and health management is made. Aiming at different diseases, the third life maintenance mode is similar to a diagnosis and treatment process of the traditional evidence-based medicine, the disease treatment is changed into sustainable maintenance and a learning process of prevention of disease against body interior function under independent consciousness; for the disease treatment, in the third life maintenance mode, the perception and the learning are used as main ways, which is called the perception of the disease and the learning of the disease. The third life maintenance mode is a method in parallel to the traditional medical method for treating the disease; the third life maintenance mode and the traditional medical method are not rejected mutually and can be carried out synchronously as much as possible. The treatment of the third life maintenance mode is a physical process of non-medicine taking, and the regulation prescriptions of the perception and learning of different diseases are as follows:

Chronic hypertension: learning games about blood vessel elasticity motion under independent consciousness, learning games about blood pressure regulation under independent consciousness, learning games about cortex inhibition and dormancy under independent consciousness, and learning games about brain relax under independent consciousness.

Diabetes (all types): learning games about blood vessel elasticity motion under independent consciousness, learning games about heart rate regulation under independent consciousness, learning games about brain inhibition under independent consciousness perception, and learning games about tip blood perfusion metabolic requirements under independent consciousness perception.

Heart and cerebral vessels sclerosis: learning games about blood vessel elasticity motion under independent consciousness, learning games about cortex inhibition and dormancy under independent consciousness, and learning games about head blood perfusion metabolic requirements under independent consciousness perception.

Congestive heart failure: learning games about cortex inhibition and dormancy under independent consciousness, learning games about sympathetic nerve tension regulation under independent consciousness, learning games about brain relax under independent consciousness, learning games about external focusing regulation under independent consciousness perception, learning games about breathing control oxygenation efficiency under independent consciousness.

Stomach inflammation: learning games about head blood perfusion metabolic requirements under independent consciousness perception, learning games about temperature regulation under independent consciousness perception, learning games about tip blood perfusion metabolic requirements under independent consciousness perception, and learning games about cortex inhibition and dormancy under independent consciousness.

Pneumonia symptom: learning games about sympathetic nerve tension regulation under independent consciousness, learning games about heart rate regulation under independent consciousness, learning games about head blood perfusion metabolic requirements under independent consciousness perception, learning games about breathing control oxygenation efficiency under independent consciousness, and learning games about cortex inhibition and dormancy under independent consciousness.

Hepatitis symptom: learning games about tip blood perfusion metabolic requirements under independent consciousness perception, learning games about head blood perfusion metabolic requirements under independent consciousness perception, learning games about cortex inhibition and dormancy control under independent consciousness, learning games about head blood perfusion metabolic requirements under independent consciousness perception, learning games about blood pressure regulation under independent consciousness, learning games about heart rate regulation under independent consciousness and learning games about blood flow circulation under independent consciousness.

Nephritis symptom: learning games about tip blood perfusion metabolic requirements under independent consciousness perception, learning games about head blood perfusion metabolic requirements under independent consciousness perception, learning games about blood pressure regulation under independent consciousness, learning games about blood flow circulation under independent consciousness, learning games about sympathetic nerve tension regulation under independent consciousness, learning games about vital capacity motion under independent consciousness, and learning games about cortex inhibition and dormancy control under independent consciousness.

Inflammation of other body organs: learning games about tip blood perfusion metabolic requirements under independent consciousness perception, learning games about head blood perfusion metabolic requirements under independent consciousness perception, learning games about blood flow circulation under independent consciousness and learning games about cortex inhibition and dormancy control under independent consciousness.

Tumor: learning games about tip blood perfusion metabolic requirements under independent consciousness perception, learning games about head blood perfusion metabolic requirements under independent consciousness perception, learning games about sleep and re-learning and insomnia treatment under independent consciousness perception, learning games about cortex inhibition and dormancy control under independent consciousness, learning games about brain relax under independent consciousness, learning games about left brain lateralization regulation under independent consciousness, learning games about brain inhibition under independent consciousness perception, learning games about sympathetic nerve tension regulation under independent consciousness, learning games about breathing control oxygenation efficiency under independent consciousness, learning games about blood flow circulation under independent consciousness, living and long-distance thought-controlled competitive match, timing sleep long-distance thought-controlled games and meditation-controlled learning games under independent consciousness.

Fracture: learning games about tip blood perfusion metabolic requirements under independent consciousness perception, learning games about head blood perfusion metabolic requirements under independent consciousness perception, learning games about cortex inhibition and dormancy control under independent consciousness, learning games about breathing control oxygenation efficiency under independent consciousness, and learning games about vital capacity motion under independent consciousness.

Insomnia, anxiety and psychoneurosis: learning games about head blood perfusion metabolic requirements under independent consciousness perception, learning games about sleep and re-learning and insomnia treatment under independent consciousness perception, learning games about cortex inhibition and dormancy control under independent consciousness, learning games about brain relax under independent consciousness, learning games about left brain lateralization regulation under independent consciousness, learning games about brain inhibition under independent consciousness perception, and learning games about sense organ input sensitivity regulation under independent consciousness (brain restraining), learning games about sympathetic nerve tension regulation under independent consciousness, and timing sleep long-distance thought-controlled games.

Depression: autonomous-thinking control thought material shift learning games, game controlled by brain relax under independent consciousness, learning of games controlled by internal focusing regulation under independent consciousness perception, living and long-distance thought-controlled competitive match, learning games about sleep and re-learning and insomnia treatment under independent consciousness perception, and timing sleep long-distance thought-controlled games.

Senile dementia: meditation-controlled learning games under independent consciousness, learning games about cortex inhibition and dormancy control under independent consciousness, learning games about sympathetic nerve tension regulation under independent consciousness, living and long-distance thought-controlled competitive match, the autonomous-thinking control thought material shift learning games, learning games about right brain advantage regulation under independent consciousness, learning games about memory processing under independent consciousness perception, learning games about external focusing regulation under independent consciousness perception, learning games about sense organ input sensitivity regulation under independent consciousness (brain restraining), learning games about blood flow circulation control under independent consciousness, and learning games about brain activity under independent consciousness perception.

Children hyperactivity and infantile autism: learning games about attention reply control under independent consciousness, learning games about left brain lateralization regulation under independent consciousness, learning games about right brain advantage regulation under independent consciousness, learning games about brain stability regulation under independent consciousness, learning games about brain stability regulation under independent consciousness, learning games about brain relax under independent consciousness, autonomous-thinking control thought material shift learning games, living and long-distance thought-controlled competitive match, timing sleep long-distance thought-controlled games, learning games about external focusing regulation under independent consciousness perception and learning games about brain reaction capacity under independent consciousness perception.

Network addiction: learning games about brain relax under independent consciousness, learning games about brain inhibition regulation under independent consciousness perception, learning games about right brain advantage regulation under independent consciousness, learning games about memory processing under independent consciousness perception, learning games about brain reaction capacity under independent consciousness perception, autonomous-thinking control thought material shift learning games and learning games about quick brain fatigue recovery under independent consciousness perception.

Psychological and mental disease: real-time doctor-and-patient interactive service of private and long-distance metal illness diagnosis and treatment, and game controlled by timing sleep long-distance thought.

Paralysis rehabilitation: learning games about cortical motion relearning and nerve network reconstruction under independent consciousness, learning games about head blood perfusion metabolic requirements under independent consciousness perception, learning games about lower limb motion kinetic energy under independent consciousness perception, learning games about lower limb motion kinetic energy under independent consciousness perception, learning games about brain activity under independent consciousness perception, learning games about brain inhibition regulation under independent consciousness perception, and learning games about blood flow circulation control under independent consciousness.

Life prolonging and health keeping: meditation-controlled learning games under independent consciousness, learning games about cortex inhibition and dormancy control under independent consciousness, learning games about left brain lateralization regulation under independent consciousness, learning games about right brain advantage regulation under independent consciousness, learning games about brain relax under independent consciousness, learning games about brain activity under independent consciousness perception, learning games about heart rate regulation under independent consciousness, learning games about brain inhibition under independent consciousness perception, learning games about blood flow circulation under independent consciousness, learning games about tip blood perfusion metabolic requirements under independent consciousness perception, learning games about head blood perfusion metabolic requirements under independent consciousness perception, learning games about blood pressure regulation under independent consciousness, learning games about blood vessel elasticity motion under independent consciousness, learning games about breathing control oxygenation efficiency under independent consciousness, and learning games about vital capacity motion under independent consciousness; all the plans corresponding to the above diseases are carried out in a mode of controlling competitive match and training by living and long-distance thoughts. Automatic computation of life data is a radical core of the third life maintenance mode, the living life data in wide areas are acquired and transmitted to an internet platform and are automatically computed and analyzed in real time by using a cloud computation mode; distributing type tree data service centers are established, 10,000 wireless mobile life data acquisition and transmission terminals are bound in each data computation service center; the received individual life data are processed and computed by each data computation service center, and the transmission of the life data per person binds address information and machine information; after the data are transmitted to a data management server, machine numbers which are defined in advance and individual account information of owners are retrieved, the individual information corresponding to the machine numbers is combined into a data structure to generate a data series to be processed; meanwhile, the data are uploaded to a storage area under an account storage server individual account.

A computation structure is decomposed into a multi-task mode, a regular computation task is finished by each computation nodes of the center, and the computation result is bound to the individual account information and sent to the management server of the center; the management server classifies and packs the data which are bound with individual account information and are transmitted by all nodes, feature codes binding with the data computation center is uploaded to the data server in which a user account is located for being stored.

The data computation center consists of the data management server, a task management server and a group of computers, the computers can be distributed on different places, all the life data in feature index groups pointing to different disease development changes are computed, and the computation structure is a filtration mode. The original data are computed by virtue of a one-level node computer to acquire a group of results; the individual account information is bound and uploaded to a second-level node computer, by parity of reasoning, the data sizes which are computed by each level of node computers are greatly reduced so as to form a filtration type data computation mode. The data management server is responsible for receiving the data transmitted by the acquisition terminal, and forwarding the data to the task server and accepting the final result acquired by one cluster of computation finally sent by the task server. Feature indexes which are bound with the individual account information are uploaded to the account storage server in which the individual account is located. The data management server consists of a plurality of servers or computers and is determined according to the quantity of the bound wireless mobile data acquisition and transmission terminals; the task management server is responsible for monitoring the states of all the node computers, accepting the original data forwarded by the data management server and the intermediate result data sent by the node computers, and distributing the data to the node computers and the data management server, and automatically distributing the data transmitted by all the terminals to computers with different nodes according to the classification rules; the classification rules are divided by the life data types and are automatically decomposed, and the decomposed independent life data are sent to the first level of node computer.

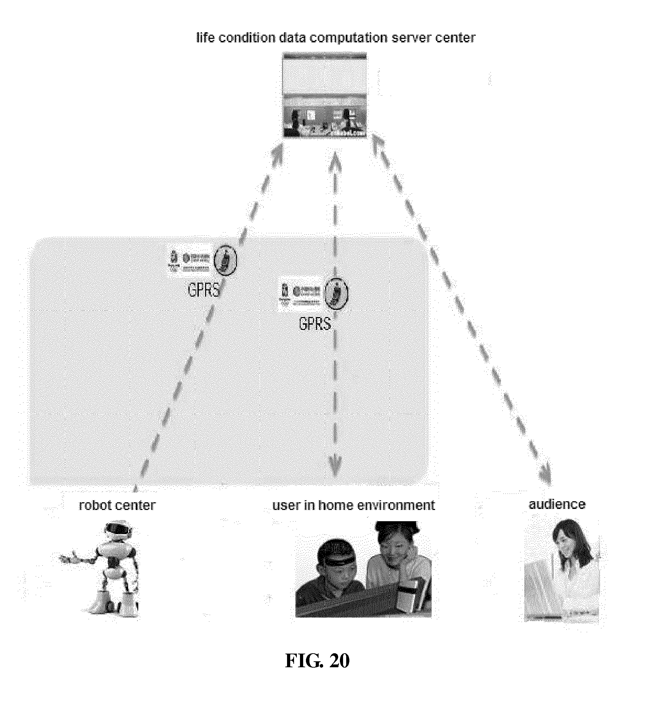

The task management server is used for monitoring the node computers of the center in real time to determine the states of the computers; the sending of the data is determined according to the states of the computers; the sending of the data or the buffer of the data is determined by monitoring the states. The sending of the data is carried out in a circulating mode, the result data returned by the low-level node computers are sent again to the superior level of node computers in an idle state, and the final computation result is sent to the data management server. The computers automatically compute the accepted data in a regular algorithm, and the acquired computation result is returned to the task management server. The task management server can also synchronously transmit the life data computation result to other computers at any node on the required internet in real time, and the life data computation result is used as information exchange data accepted by the body sense organs. The system structure is shown in Fig. 20.

A thought-controlled robot challenge match is built, the original instincts of games and competitive adventures of human are utilized, the interior reward system of the brain is used as a persistent process of driving the disease treatment, prevention, rehabilitation and health keeping and life prolonging, so that a good psychological environment of focusing and pleasant disease diagnosis and treatment and healthy process is created. Because of psychological factors, a positive function on the disease treatment and rehabilitation is achieved. A new channel is developed, a new health driving system is built, and in the processes of disease treatment, health keeping and life prolonging, and disease rehabilitation, the influence of the psychological factor on the effect is changed into positive energy, the worry and the fear caused by disease treatment is converted into pleasure, stimulation, focusing and a behavior of consciously keeping on. The pleasant mentality and the desire to excel over others are introduced into the health keeping and life prolonging process in a mode that the wealth is gained by utilizing the disease treatment technology and the effect. The change of the life data is a technology which can be used as a competitive match skill, the reward can be gained from the match and can improve the life and increase the wealth, and the skill of the match is increased by the mentality of wealth gaining; with the increment of the skills, the health is improved and the life is prolonged. Due to the repeated process, the disease diagnosis and treatment and the process of rehabilitation are promoted; the pleasure and the focusing are in the training brain state, so that the fear and the worry of the disease are greatly reduced, and the change of the life data is an autonomous control result.

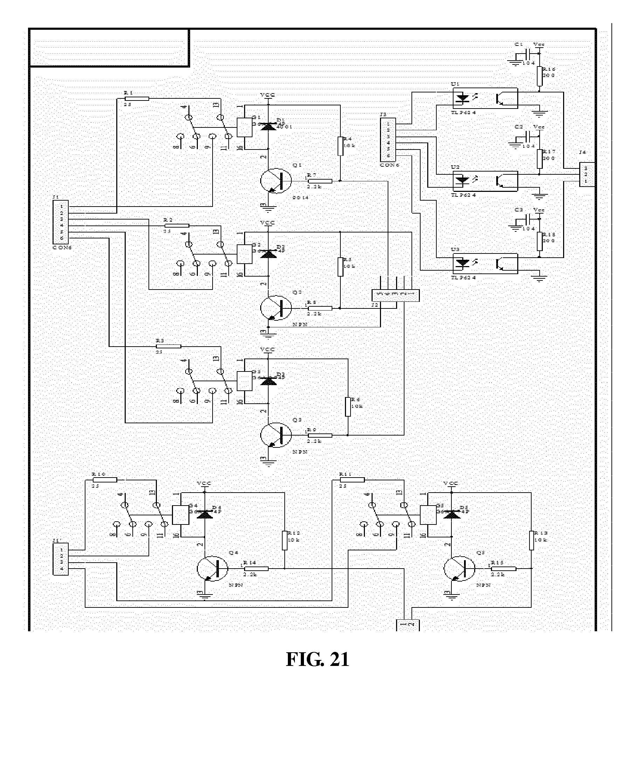

A robot adopts a programmable control robot NXT type imported from America, has a Bluetooth wireless communication interface, acquires different real-time life data by the wireless mobile life signal acquisition and transmission terminal, uploads the data to the computation center server in real time for performing real-time computation processing through a wireless internet platform, transmits the computation results to a robot control computer by the wireless internet platform, and controls the computer to generate a robot control command along with the consideration of the life data computation results through the Bluetooth wireless communication interface, so that the motion of the robot in four directions is driven. The thought-controlled robot structure, and control and motion signal acquisition circuit are shown in Figs. 21-23.

Match introductions and competition rules:

Introductions: electroencephalographic waves and a plurality of physiological signals are acquired by the rented or purchased carry-on wireless mobile life data acquisition and transmission terminal and are transmitted to the data computation service center in real time by virtue of 3G and wireless internet; after computation processing, the feature indexes in the electroencephalographic waves or the plurality of physiological signals are extracted, converted into control commands and sent to a brain sense thought-controlled robot by virtue of a wireless internet access so as to drive the actions of marching, drawing back, turning left, turning right and the like of the robot, and therefore, the closed-loop autonomous regulation and sub-health treatment of the brain function state or psychological life data is realized. Users can open the thought-controlled training of the long-distance robot in the regular time everyday and also can participate in the long-distance thought-controlled competition match which is held in the fixed date of one week.

Application environment: under the family environment of the users, the perception measurement technology related to the brain state is used, different actions of the robot in the thought-controlled robot center can be remotely controlled and set by utilizing a self-brain state signal, a psychological signal and a public communication platform of the users on different living places, so that the autonomous regulation of self-brain state is finished.

Participation ways: training: the wireless mobile life data acquisition and transmission terminal is rented or purchased, a video address of a health bank individual account is opened, and a self-controlled robot is determined to enter a self-thought-controlled training mode; every 30 minutes of the training is taken as one unit, which is called one machine hour.

Competition: the wireless mobile life data acquisition and transmission terminal is rented or purchased; during the designated competition time, a video address of a health bank individual account is opened and the self-controlled robot is determined, after the control commands are unified, the competition match of the thought-controlled robot is started; the standard competition time is 30 minutes/round.

| Common rent | 100 yuan/machine hour | Starting from 5 machine hours (returning within 5 days, deducting 100 yuan per day in case of delayed return) | Guarantee deposit: 20,000 yuan |
| --- | --- | --- | --- |
| VIP rent | 100 yuan/machine hour | Starting from 30 machine hours (returning within 30 days, deducting 100 yuan per day in case of delayed return) | Guarantee deposit: 20,000 yuan |
| Purchase user | 0 yuan/machine hour | None | None |

After common users purchase the machine hours, the common users can use within the designated time; instead, VIP users and purchase users can freely choose the use time.

Rewarded competition: in the match of using the thought-controlled robot, the victory or defeat is used as a reward evidence, and an operation mode of reward competition match is carried out, so that people carry out the competition match in the focusing state or even in a good mental state. The winner can win four machine hours at least in every competition, so that the possibility of winning prizes is increased while the health is promoted.

Entry requirements: whatever the robot mode is used, once the training time of persons is accumulated to 50 machine hours, the persons are qualified to participate in the competition match activity of the thought-controlled robot held by the health bank website; for the persons who participate in the competition match, there is no limitation of ages, genders, occupations and the like; for the persons who participate in the competition match, the surplus machine hours of the individual account are not less than 5 machine hours, the system locks five machine hours as a competitive chip (the minimum) during the match; in the competition match, a live broadcast way is adopted, all the competitors can participate in the competition in a long distance at home; each competition consists of the same level of players (see the description behind); all the competitors voluntarily participate in the competition and need to obey all the relevant official rules stipulated by Yifei Huatong.

Pre-competition preparation: 20 minutes before the regulation match, competitors are in position, the controlled robot is tested, and the thought-controlled machine center is required to guarantee the full electricity quantity of the participating robot; after the beginning of the competition, if the insufficient electricity quantity of the wireless life information acquisition and transmission terminal is caused by individual reason of the competitor, the robot on his own cannot be continuously controlled, and the competition is still valid; after the countdown of competition is 1 minute, all the participating robots cannot walk out of a start range, otherwise the competitor is required for abandoning the competition.

Competition rules: the challenge match of the thought-controlled robot includes three items (A, straight line competition: robots who arrive at the destination at first are winners; B, obstacle-crossing competition: obstacles need to be crossed, and robots who arrive at the destination at first are winners; C, arena competition: robots who completely go out of arena boundary lines are losers); the robot competition area environment is a cold light source and low in illumination level; meanwhile, the magnetic field interference of the camera is reduced to the minimum, and each video live broadcast competitive platform needs three staff including a referee, a greffier and a commentator; the time for the competition time of the A item is 15 minutes, if the time is up, among the competitors who do not arrive at the destination, the competitor who is most close to the destination is a winner; the time for the competition time of the B item is 20 minutes, if the time is up, among the competitors who do not arrive at the destination, the competitor who is most close to the destination is a winner; the time for the competition time of the C item is 30 minutes, if the time is up, if there are still more than 2 robots in the arena, an extra-time competition is carried out until only one robot is left and is an only one winner;

Competition sequence: competitors determine the sequence of participating in the competition by network draw lots; the competition sequence is not changed once the sequence is arranged. All the competitors must participate in the match according to the set sequence, and the next round of competition is started after the first round of competition of all the teams is finished.

Reward rules:

1. Same-level standard competition

After the finish of the competition, a presenter determines the result of the competition, the competitor who wins the first prize can win four machine hours, the machine hour of the competitor who wins the second prize is neither added nor reduced, and five machine hours of the competitor who wins the third prize is deducted;
the levels of competitors: (Stoneware level) the trainers who have consumed 50 machine hours, with not less than 5 machine hours of the surplus machine hours for participating in the competition;
(Bronze level) the trainers who have consumed 70 machine hours, with not less than 15 machine hours of the surplus machine hours for participating in the competition;
(Cast iron level) the trainers who have consumed 90 machine hours, with not less than 30 machine hours of the surplus machine hours for participating in the competition;
(White silver level) the trainers who have consumed 110 machine hours, with not less than 35 machine hours of the surplus machine hours for participating in the competition;
(Gold level) the trainers who have consumed 150 machine hours, with not less than 50 machine hours of the surplus machine hours for participating in the competition;
In addition, if the trainers who participate in the competition win for 10 times accumulatively, their levels will upgrade automatically to a higher level.

2. Challenge levels

Competitors who are at certain levels can choose the challenge competition, and the match can begin with only two competitors.
Low-level competitors VS high-level competitors: the low-level competitor challenges one superior competitor, namely, the loss percent is increased by 0.4 time. For example, grass-roots level competitor VS petty bourgeoisie level competitor: if the grass-roots competitor provides five machine hour chips, the petty bourgeoisie level competitor who accepts the battle needs to provide seven machine hour chips; the grass-roots level VS the superior level: if the grass-roots competitor provides five machine hour chips, the superior level competitor who accepts the battle needs to provide nine machine hour chips.
High-levels VS low-levels: the high-level competitor challenges the lower level of competitor, the loss rate is reduced by 0.4 time. For example, the high-level competitor VS petty bourgeoisie level competitor: the high-level competitor provides seven machine hour chips, the petty bourgeoisie level competitor who accepts the battle only needs to provide five machine hour chips; the high-level competitor VS the grass-roots level: if the high-level competitor provides nine machine hour chips, the grass-roots level competitor who accepts the battle needs to provide five machine hour chips.

3. Annual Champion Cup

The system generates the monthly champion according to the competition condition every month, the health bank website cooperates with domestic large-scale satellite television media, the finals of the annual champion cup is played in a live broadcast mode, and the annular final champion accumulates the condition according to the competition system and a big prize comes out. Reward exchange: the competitors who have more than 50 machine hours of the total surplus machine hours can exchange the rewards; the prizes include rechargeable cards, shopping cards, toys, gifts, mobile phones, household appliances, precious metal ornaments, domestic/overseas travel and the like. An individual diagnosis and treatment health account is managed by an electronic intelligent medical treatment track, along with an individual medical health electronic data storage platform including individual medical treatment information, daily persistent life features and psychological data information, daily individual health subjective feeling and disease complaint information, input quantity and output quantity and individual living oral administration medicine treatment. Human-machine interaction on the storage and the use of the data is realized by virtue of an operation interface of an internet explorer; a medical health bank account, similar to the bank deposit, with a personal security control function, is built, and the medical health data are stored and checked. The medical treatment information is recorded into the individual medical health account in a manual mode, the daily persistent life features and the psychological data are acquired by virtue of the wireless mobile life data acquisition and transmission terminal and are automatically uploaded to the individual account by virtue of the wireless internet communication platform. The information about individual subjective feeling and complaint, medicine treatment, eating quantity, drinking, excrement quantity, urine quantity and the like are input and uploaded to the individual account by virtue of an APP application program of the mobile communication terminal. Based on the medical clinical informationalized (CIS) structure in the account, the medical treatment health information includes medical records, prescriptions, treatment, examination, images, medical advices and body feature information. The individual account data are shown in modes of numeric values, pictures, trend change curves, text reports and real-time waves, and the multiple data showing modes of flexible selection of time window, data combination and data excavation are adopted. The acquired life data are computed and stored item by item, the examination report list is produced by the disease diagnosis examination analysis report form and content combination for storage and viewing. The relevant treatment and medical advice indication information in the account is automatically sent to an individual communication terminal (mobile phones, mobile computers and fixed computers) by virtue of a connective short message communication platform according to the time requirement; the individuality executes the diagnosis and treatment process in modes of voice, short messages, screen display and the like; if the execution replay information is not received within the set time, the account automatically sends the short message to the set mobile communication equipment or the fixed computer and proposes the favor request and the alarm information to others or mechanisms; the account automatically does primary health prediction on the medical treatment health information in a period of time and regularly sends to the individual mobile terminal or the computer of the designated health service provider for displaying and prompting. A real-time interactive window is set in the account, mutual real-time communication is made between doctors and individuals and between individuals and individuals in modes of videos, characters, waveforms, numeric values, trend changes and the like, so that real-time consultation is carried out. The application of the account is bound with a computer and a piece of intelligent mobile communication equipment, and the data contents are viewed and displayed on two computers by means of network website browsing.

Building of health bank individual account: an exclusive individual medical health account (hereafter referred to as individual account) is registered by using real names on the health bank website. Human-computer interaction is realized through an operation interface of the internet explorer based on the storage and the use of the data in the individual account, the medical health bank account, similar to the bank deposit, with individual safety control function is built and is used for storing the medical health data and viewing. The individual account content comprises six parts including:

Personal medical treatment tracks: individual medical treatment information comprising paper or picture data like medical history, diagnosis, laboratory test report, radiographic images, examination report lists, prescriptions and medical advices; the data are received by the health bank data center after the treatment every time and are input into the individual account in a manual or scanning mode according to the medical treatment electronic informationalized CIS system format.

Daily persistent life feature and psychological data information: automatic acquisition and input of the daily persistent life feature and psychological data information; by utilizing the wireless mobile life data acquisition and transmission terminal, the daily persistent life feature and psychological data information is automatically uploaded to the individual account through the wireless internet communication platform in real time.

Daily individual health subjective feeling and disease chief complaint information: the input of the daily individual health subjective feeling and disease chief complaint information; the subjective information comprising dizzy feeling, sick feeling, weak chest, pain, cough, fever, having a cold, having loose bowels and the like are input in real time by individuals and uploaded to the individual account through the APP application program of the mobile communication terminal or the individual computer system.

Input quantity and output quantity: the input of the input quantity and output quantity information; the input quantity and the output quantity comprise the information about intake quantity, urine quantity, excrement quantity, sweat quantity and the like; the data are acquired by means of evaluation and are input by individuals and uploaded to the individual account through the APP application program of the mobile communication terminal or the individual computer system. Individual living oral administration medicine treatment information: the input of individual living oral administration medicine treatment events and life measurement events; the information is input by individuals and uploaded to the individual account through the APP application program of the mobile communication terminal or the individual computer system.

Amount of exercise: the relative exercise amount of the human is acquired through the wireless mobile life information acquisition and transmission terminal and is automatically uploaded to the individual account through the wireless internet communication platform.

The health bank individual account information structure is shown in Fig. 24; the presentation of the individual account contents is as follows: based on the medical clinical informatization (CIS) structure, the medical treatment health information comprises cases, prescriptions, treatments, examination, images, medical advices, body feature information and subjective feeling, daily treatment, input and output quantity, exercise amount and the like; the data in the individual account are displayed in modes of numeric values, pictures, trend change curves, text report and real-time waveforms, and the multiple data showing modes of flexible selection of time window, data combination and data excavation are adopted. By utilizing the individual health account, the followings can be realized:

(1) The life data in the account are computed and stored item by item, an examination report list is generated according to the multiple ways of disease diagnosis examination analysis report format and the content combination; when the individual sees a doctor, does a long-term health-care plan, and is in emergency and in need, the data are convenient to store and view. Many people go to a hospital to see a doctor, the main complaint is unclear and cannot have a good communication with doctors; once there is the information in the account, the doctor can easily see the body feature change of a patient in a period of time; the previous medical treatment track is noticed, so that the correctness of the diagnosis is conveniently improved and the diagnosis time is shortened, the misdiagnose is reduced while the working efficiency of the doctor is improved. When a nutritionist makes a long-term dietary therapy recipe for the patient, the conditions of the diet, the output quantity, the medicine oral administration in the past time can also be known, and the life data in the account can completely help the nutritionist to make a best plan for the patient; if the patient have an accident and is in need of first aid, if the doctor can grasp the life data of the patient before emergency, the success rate of the emergency doctor is certainly much higher than the success rate of doctors who do not grasp the diagnosis and treatment health information in the recent time.

(2) The account automatically makes primary health prediction on the medical treatment information in a period of time, and regularly sends the information to the individual mobile terminal or designated computer of the health service providers for displaying and prompting. The serious illness prevention can be well done in time, when the disease is not developed to a certain degree, a health abnormal prompt is started so as to remind people of going to see a doctor in advance, so that the life track is improved in advance, the treatment is carried out in advance, and the disease cannot be further developed to a serious degree; therefore, the daily health-care and the disease prevention can be done to the best. For example, for patients who orally take hypoglycemic drugs in a long term and are still high in blood sugar, the report shows that whether the drug is appropriate for being used, whether the medicine dosage needs to be added or insulin is injected and whether the recent input quantity is strictly controlled, so that the key of the problem is easily found out and the problem is solved to prevent complication. For people who have no behavior ability, particularly the elder population, the individual account can send the information to guardians; the husband and wife who live in different places for a long time can mutually send the report; older people who live alone can also send the report to sons and daughters; family members can take care of each other.

(3) Interaction windows are set in real time in the account and are browsed and reviewed in the forms of videos, characters, waveforms, numeric values, trend changes and the like. Mutual real-time interaction and real-time consultation between doctors and individuals, between individuals and individuals and between doctors and doctors can be realized; under the family environment, real-time interaction communication about complete medical treatment track information, life data information, subjective feeling information and daily activity information between the individuals and the doctors is carried out; the medical treatment health service at anytime and anywhere for disease treatment, disease rehabilitation and chronic disease management can be realized; meanwhile, the resources of community hospitals, the resources of secondary hospitals and even the resources of tertiary hospitals also can be enjoyed, and the aim of seeing specialists without going out can be fulfilled; the interaction between the individuals and the individuals can be competition matches, wardmate parties, experience communication and the like, which enriches the health knowledge of the people, widens the health view and gains a healthy body.

(4) Automatic medical treatment management prompt service: According to the life data information, the medical advice information and the treatment prescription information of the doctor after treatment produced in the account, the system can provide personal customized prompt service according to the individual concrete requirement. The system automatically extracts daily treatment and examination contents under the individual account, and treatment examination prompt information is automatically sent to an individual communication terminal (mobile phones, mobile computers and fixed computers) by the connective short message communication platform, and the individuals are notified to execute the diagnosis and treatment process in modes of voices, short messages, screen display and the like, such as medicine-taking, exercise prompt and the like. According to the set individual service, the entity staffs are notified by alarm short messages which are bound with one-level alarm, two-level alarm, three-level alarm, four-level alarm and the like. For example, hypertension patients setting the service need a medicine-taking prompt service and a blood pressure trend pre-alarming service, the medical advice is that hypotensive drugs of 5mg are taken at 7:00 a.m. and 16:00 p.m., and the blood pressure is kept below 140/90 mmHg; at 6:50 a.m. and 15:50 p.m., the system can inform the patients of taking medicines; if the patients take the medicine on time, the short message of reply is confirmed in the set time.

If the executive confirmation information is not received within the set time, the account automatically starts a one-level alarm service, the short message is sent to mobile communication equipment or fixed computer of relatives of patients so that the family members supervise to take medicines and confirm the reply; if the one-level alarm has no response in the set time, the two-level alarm service is automatically started, the message is sent to doctors of the community hospital, and the doctors of the community hospital perform the door-to-door service for the patients and the reply is confirmed; if the reply of the two-level alarm is not received in the set time, the three-level alarm service is started again, and a designated hospital emergency service can be directly contacted. If the blood pressure is higher than the 140/90 mmHg required by the medical advice, the alarm service also can be started according to the levels. The alarm levels and the customized emergent contact persons are customized according to the requirements of the individuals. Once the daily health-care service is provided, many serious diseases cannot be happened, and even if serious diseases are really happened, patients can also be rescued in time.

(5) Other customized services such as treatment games, appointment of nursing workers, appointment of door-to-door services, company of activities and the like. There are many other customized service forms, such as the elders who can take care of themselves, suffer from chronic diseases and have no sons and daughters to accompany choose to keep pets for spending time and gaining psychological comfort; the elders have a deep emotion with pets, accompany with pets for walking, eating and even sleeping, thus the elders can quietly sleep; even if the emotion between the pets and the human is deep, the expression way is limited, and therefore, we can customize a mobile terminal which is in a pet necklace ring form to supplement the defect that the pets cannot talk, the medicine taking is prompted by the pets, the elders have a lunch and do exercises in the company with the pets; the interaction process of the elders and the pets and the requirements on mentality and psychology are met, thus improving the quality of the life; for example, old patients who suffer from serious diseases and are inconvenient to move and have no son and daughter to always stay at hand are served by the wife or husband for eating or drinking; because of the electronic intelligent medical treatment health account, the possibility of running about between the home and the hospital for the patients is reduced to the maximum extent so as to provide convoy for the health of the patients; when the patients need to be looked after by persons who have physical power in need of taking a bath and going out, the patients can enjoy the door-to-door bath-taking service from the professional nursing workers through a customized service; compared with a way of inviting the nursing workers for a long term, the customized service is economical and practical, and the life quality of the elders also can be guaranteed.

A daily popular disease treatment, rehabilitation, prevention and health keeping and life prolonging information stream on a media is built and is used as a daily tool for health consumption. A demonstration head person leading mode of various disease rehabilitation, prevention and life-prolonging persons based on entire space-time life data is built, and a leader health information issuing platform is built.

With the increment of the social life level, how to delay the aging and prolong the life becomes the most concerned problem in the 21st century. Once people are old, the worried health problem is followed; particularly the most important is the health of the elders, which is related to the happiness of the whole family. At present, the elders who are more than 60 years old reach nearly 200,000,000, which is possibly close to a half of the total population of China once the persons who are more than 40 years old are counted in; these persons basically have a stable career or are already retired, and therefore, the health problem is the key to be concerned by these persons. Different from the young people, the middle-aged persons and the elder do not like the popular idols who are famous by faces and acting skills and should set up their own idols for themselves. The idols of the young people are movie and television stars and venture successful people; the words of the idols are imperial commands and are absolutely obeyed, and sometimes, the obsession to the idols makes them obsessed; instead, the middle-aged persons and the elder concern more about pronging the life and keeping away from the diseases and more like the topics such as how to prevent diseases, how to prolong the life of 100-year elders, how to avoid senile dementia and how to get rid of invasion of serious illness for cancer patients. The health maintenance program is very popular, which fully demonstrates the concern of people on the health. In recent years, as people concern the self-health more and more, a great upsurge in health maintenance is followed, and even some health-maintenance stars appear through the media. The health maintenance and the relevant books are also very hot; the upsurge in health maintenance shows it is a good thing of the concerning to the self-health; whatever the books or the television programs, it is certainly beneficial for the popularization of science of the massive medical treatment and public health knowledge, some health maintenance guidance advices are provided for the public, and therefore, the popularization of science has a certain positive meaning; however, most of the contents mainly include the mechanical expounding without any substantial content, thus it is difficult to truly help people to fulfill the aims of health keeping and disease prevention, and the obvious function in improving the disease knowledge of people cannot be achieved.

A public media health reference template for middle-aged persons and the elders is built, and the definite quantitative life data are used as the cores, the content comprises all life data which are publicly displayed to the public at regular intervals; the data belong to the excellent disease rehabilitators or life-prolonging elders, and the excellent disease rehabilitators or life-prolonging elders are taken as health tractors, the middle-aged persons and the people who are eager to keep the health in the whole society are led to keep away from the diseases and keep the health and prolong the life by using the health life data of the excellent disease rehabilitators or life-prolonging elders.

The publicly-displayed life data are imitative samples of people. By the individual account of the health bank, the own life data are comprehensively compared with the template data, the comparison including superposition comparison of waveform data, trend fitting comparison of numeric value data, content comparison of disease examination reports, comparison of treatment processes and numeric value comparison of life data (including diet, input quantity, output quantity, exercise quantity, subjective feeling and the like). The difference of the own life data and the life data of tractors can be found, the development trend of their own disease rehabilitation process can be discovered, and the change of the own health can be discovered, so that the diseases can be found in time, and the goal of disease prevention can be fulfilled. Meanwhile, by continuous comparison, people are even more aware of the medical knowledge and the disease characteristics; the mastered medical knowledge is continuously accumulated, even to reach the semi-professional levels, so that the final health education goal of making people become the doctors is truly realized, and the health education and the popularization of the medical knowledge can be perfectly solved. Relative to the current health maintenance and health propaganda expounding mode, the data expressions of the health tractors obviously play a great role and are more direct.

The activities of the health tractors are characterized in that based on the individual health bank account, the complete medical treatment tracks and the life data of some natural persons are issued regularly every day on the media (network, television and plane), these persons are elected by the screening of the medical treatment committee and the success of the life data competition match and completely show the medical treatment track and the life data on public in a payment mode. By holding a competition program, the health tractors are elected in a combined mode of talent show and health maintenance programs. In recent years, people still have a passion on the talent show programs, and the new forms of the show programs are continuously introduced. By the ultrahigh popularity of the show programs, along with the health hot topic, a good effect can be produced. By holding the talent show programs, idols who pursue the health and are eager to keep the health and prolong the life are elected. The programs can be in a current fashionable entertainment form such as the Voice of China, Super Boys and the like; health keeping and life prolonging idols like life-prolonging idols and anti-X idols are created in a competitive choosing mode.

Election ways: on-line mass-election, off-line election and final competition. All the competitor data of the participation of the election are totally and publicly displayed, and the final competition is in live broadcast by means of television and network. All the persons who participate in the election must be individual account registers of the health bank website. The first step is on-line election, all the persons can participate whatever the nations, the races, the gender; the competitors upload their real life data by the health bank individual account; the second step is off-line election, at first, the authenticity of the life data filled in on the internet needs to be checked on the spot, and then, the on-site election can be carried out.

Election contents:

1. Screening according to the physical health standard of the World Health Organization

   1) About the definition of the physical health, ten standards of the health provided by the World Health Organization are referred;
   2) Needing enough and sufficient energy and unhurriedly handling with the daily life and the working pressure
   3) Optimistically dealing with affairs, having positive attitudes, being pleased to undertake the due obligations and having no hyper-criticality of transaction
   4) Being good at having a rest and having a good sleep
   5) Having a high ability of dealing with ambiguity, and being adapted to all environment changes
   6) Capability of resisting against a generalized cold and infectious disease
   7) Appropriate weight, uniform body figure and coordination of head, shoulders and arms during standing
   8) Bright eyes, keen reaction and no inflammation of eyelids
   9) Clean teeth, no holes, no pain, normal color of gingival and no bleeding
   10) Luster hairs and no dandruff
   11) Resilient muscles and skins and feeling relaxed during walking

2. Confront competition match of the life data

1) Meditation control under independent consciousness perception
2) Cortex inhibition and dormancy control under independent consciousness
3) Brain relax control under independent consciousness
4) Brain activity under independent consciousness
5) Heart rate regulation under independent consciousness
6) Brain inhibition regulation under independent consciousness perception
7) Blood flow circulation control under independent consciousness
8) Tip blood perfusion metabolic requirements under independent consciousness perception
9) Head blood perfusion metabolic requirements under independent consciousness perception
10) Blood pressure regulation under independent consciousness
11) Blood vessel elasticity motion under independent consciousness
12) Breathing control oxygenation efficiency under independent consciousness
13) Vital energy motion under independent consciousness

Competition rules: the robots are controlled by target data of all items to move, and the robots which first arrive at the designated position win the game.

3. Expert review

A jury is set for electing participating competitors. The jury includes domestic and overseas famous doctors and experts, and the jury aims to choose the health tractors who meet the standard and are the spreaders of the correct health conception.

The second step: publicity and promotion of the health tractors by utilization of media resources The health tractors who are elected sign an agreement and are created to be idols who are sought by the public, and the agreements comprise the following items:

1. The health tractors issue the own real-time life data. By utilizing the wireless mobile life information acquisition and transmission terminal and the individual health account, the real-time life data and the medical treatment data of the health tractors are acquired, are automatically uploaded to the display window of the media, and are played to the whole nationwide crowd at regular intervals. The data comprises medical treatment doctor-visiting information, treatment information, subjective feeling information, exercise amount information, the diet information, the excrement amount information, the life body information, the psychological parameter information and living simple biological measurement information.
2. The regular incomings can be gained every month based on the condition that the health tractors issue their own real-time life data.
3. The opportunities of advertisement and endorsement are provided.

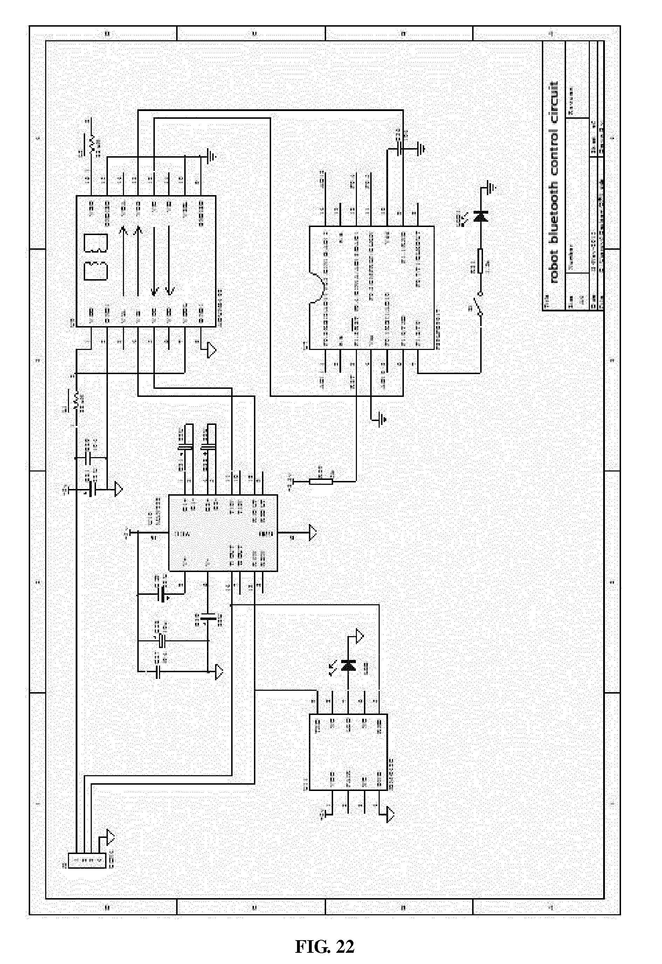

The third step: the medical treatment knowledge is propagated by utilizing the health tractors so as to make everybody be their own doctor. The health tractors represent the processes of correct treatment and correct rehabilitation of some diseases, including rehabilitators of various types of cancers, outstanding persons in controlling chronic disease, healthy and outstanding pregnant women and more than 100-year-old elders, and represent a correct disease treatment method, a correct disease rehabilitation process, a correct chronic disease management method, a correct life track and diet content, a correct life attitude and mood change , a correct sports exercise and labor track, a correct disease doctor-visiting method and look-after of elders. Health tractors are just like a mirror, from whom others can learn to find out the way for health keeping and life prolonging. Meanwhile, due to continuous repeated comparison, everybody can be his own doctor. Fig. 19 is a computer system structure; Fig. 20 is a robot match sketch map; Fig. 21 is a thought traction controlled circuit map; Fig. 22 is a robot action signal acquisition circuit map; Fig. 23 is a health bank individual account information structure; Fig. 24 is a disease treatment feedback structure based on the life data.

Because of the health tractors, the self-adaptive health management mode of market economy behavior is finally realized, the contents are provided for pushing the positive energy for the media. The mode of the influence of the fashion such as star creation, star seeking, big V, talent show and the like is added into the processes of health keeping and life prolonging, disease treatment and disease rehabilitation. As health-keeping demonstration leaders, the demonstrators become health stars and health experts, leading people to go through a scientific and rational health-keeping and life-prolonging process based on the quantitative data; meanwhile, the health tractors can reasonably gain fame and fortune for themselves.

**Claims**

1. A life maintenance mode, comprising the steps of
   automatically measuring and computing life condition data, and classifying inspection items into quantitatively measured life condition data converted from individual tables;
   performing active control of the change of the life condition data by means of senior independent consciousness of cerebral cortex in combination with brain inhibition therapy to serve as a quantitative traction means for treatment, rehabilitation, and health and longevity;
   composing a report of inspection analysis and health trend prediction by analyzing and computing the life condition data, so as to predict and prevent the occurrence and development of diseases;
   automatically sending messages to remind a patient at home to check the treatment behaviors, based on doctor's advices and prescriptions in the track record of medical treatment, and providing an automatic no-response alarming service.

2. The life maintenance mode of claim 1, wherein, the process of automatically measuring and computing life condition data comprises the following steps of
   converting signal of vital signs and physiological data into digital signal,
   transmitting the signal to the filtering module, noise control module and input amplification module of the monitoring circuit, and after being computed and processed by corresponding monitoring modules, the signal is transmitted to an analog-digital convertor circuit of the central computation and control management circuitry through an analog signal input port;
   sending the processed signal to a communication interface of the central computation and control management circuitry through an RS232 interface circuit;
   encrypting and compressing the digitalized signal data received by the central computation and control management circuitry to obtain a processed data stream, which is then fed into a dynamic data link cache queue;
   the dynamic data link cache queue is a varying data storage and output configuration;
   data configuration in a storage region is determined by network condition;
   after an interruption event is triggered due to that the central computation and control management circuitry is notified of the change of network condition, the central computation and control management circuitry controls various permutations and combinations of the obtained data;
   the data is then written into a data buffer of the storage queue upon a write instruction;
   the data in the queue is outputted to a wireless internet access control circuit through a data port upon a read instruction of the central computation and control management circuitry;
   the internet access control circuit carries out auto-dial to the network, network condition recognition, TCP/IP mode signal modulation, sub-package and outputting, so as to allow the data to be uploaded to the data storage server and data computation server.

3. The life maintenance mode of claim 1 or 2, wherein, the process of automatically measuring and computing life condition data further comprises the following steps of
   acquiring living condition data and subjective feeling data through a subjective feeling information collector which includes a computerized equipment, such as a cell phone, a mobile tablet, a stationary computer, with a browser window of B/S configuration or an operation window of APP software as the input interface, logging the data by manual input, and selecting data items related to different feelings or parameters by click-choice on the input interface;
   uploading the data to the data storage and computation server of the personal health bank account directly through wireless or wired internet access of the computerized equipment.

4. The life maintenance mode of any of claims 1-3, wherein, in the process of automatically measuring and computing life condition data, the acquired signal includes:

   (1) vital signs and physiological data, including:

   waveform signal: electroencephalographic wave, cerebral and peripheral blood perfusion wave, penis blood perfusion wave, electrocardiographic wave, pulse oximetry wave, mouth-nose-chest-abdominal respiration wave, electromyographic wave, eye movement wave, breath-end carbon-dioxide concentration wave;
   numerical signal: pulse rate, non-invasive blood pressure, blood glucose, pulse oxygen saturation, breath-end carbon-dioxide concentration, inhaled oxygen concentration, body temperature, respiratory rate, penis cross-section diameter, penis hardness, penis temperature, penis arterial oxygen saturation, urine volume, relative exercise amount of limbs, body weight, exercise sweat amount;

(2) acquired living condition data: medicine dosage, food intake, water intake, defecation amount;

(3) acquired subjective feeling data: pain rating scale, emesis rating scale, dizziness rating scale, living quality rating scale, chest distress, urination frequency, urine color, hemorrhage, diarrhea, constipation, excrement color, palpitation, shortness of breath, impotence, vertigo, asthma, pyrexia, dysphoria, anger, anxiety rating scale, depression rating scale, difficulty of falling asleep, dysphylaxia, dreaminess.

5.  A brain inhibition therapy, comprising the steps of
    acquiring a real-time blood perfusion index of the supraorbital artery as a feedback parameter,
    performing automatic feedback control over infusion of venous sedative with an infusion pump under sedative dosage,
    letting the oxygen metabolic requirement of senior cognition function of cerebral cortex decrease to the lowest level,
    controlling inhibition of the senior cognition function of the cerebral cortex.

6.  The brain inhibition therapy of claim 5, wherein, the step of performing automatic feedback control over the infusion of venous sedative with an infusion pump under sedative dosage further comprises the following sub-steps of
    performing closed-loop feedback control over the infusion of the venous sedative with the infusion pump by using characteristic indicators of the brain blood perfusion index, wherein, the feedback control mechanism is a steady-state regulation system which controls the brain blood perfusion index to reach the minimum;
    performing closed-loop feedback control over the infusion of the venous sedative with the infusion pump by using the characteristic indicator of brain blood perfusion index of the supraorbital artery as a feedback parameter, to achieve the control target that the brain blood perfusion index of the supraorbital artery reaches the minimum;
    acquiring the brain blood perfusion signal of the supraorbital artery by using signal acquisition and computation technology of blood perfusion of the supraorbital artery, through a wireless mobile terminal for acquisition and transmission of vital signs, transmitting the signal to a processing computer through WIFI, allowing the computer to automatically compute and process the signal to obtain quantitative data of the real-time blood perfusion index, and then sending a control instruction to a computer interface of the infusion pump through a standard RS232 communication interface, so as to control the automatic infusion of sedative with the infusion pump, thereby achieving the purpose of inhibition of cerebral cortex cognition function.

7.  The brain inhibition therapy of claim 6, wherein,
    Target Controlled Infusion (TCI) is adopted as the control mechanism, with the blood drug concentration as a controlled object and the blood perfusion index as a feedback regulation parameter, the blood perfusion of the supraorbital artery is adjusted to the minimum by increasing or reducing the blood drug concentration, so as to quantitatively control the automatic sleep sedation therapeutic treatment.

8.  An encephalic signal acquisition method for the brain inhibition therapy, comprising the steps of
    acquiring electroencephalographic wave on both sides of the forehead, supraorbital artery blood perfusion wave on both sides of the forehead, and peripheral blood perfusion wave;
    letting the electroencephalographic wave, the supraorbital artery blood perfusion wave and the peripheral blood perfusion wave be transmitted to a front electrical signal amplifying circuit through a transducer, and then transmitted to an analog-digital convertor circuit to be converted into a group of discretized computer data set after being integrated, filtered and amplified by an analog circuit;
    computing a brain blood perfusion index based on the supraorbital artery blood perfusion wave on both sides of the forehead;
    computing a peripheral blood perfusion index based on the peripheral blood perfusion wave; processing the electroencephalographic wave to obtain characteristic indicators of the brain condition.

9.  The encephalic signal acquisition method of claim 8, further comprising the step of
    computing vascular elasticity based on electrocardiographic wave and the blood perfusion signal and also computing a brain power spectrum based on the electroencephalographic wave and the characteristic indicators of the brain condition.

10. The encephalic signal acquisition method of claim 8, wherein,
    the characteristic indicators of the brain condition includes one or more of brain relaxing index, brain concentrating index, brain activity index, brain thinking index, brain restraining index, left-right brain lateralization index, brain stability index and brain sedation index.

11. The encephalic signal acquisition method of claim 8, further comprising
establishing a brain condition scatter point diagram based on the characteristic indicators of the brain condition, so as to obtain the change of the brain condition.

12. The encephalic signal acquisition method of claim 8, wherein,
supraorbital artery blood perfusion wave on both sides of the forehead is acquired with a pressure sensor, a laser sensor and an electroencephalogram sensor on the forehead.

13. An device with a signal transducer of electroencephalographic wave and forehead blood perfusion wave and an independent transducer of peripheral blood perfusion wave integrated therein, comprising
an integrated signal transducer of double-conduction electroencephalographic wave and double-conduction supraorbital artery blood perfusion wave, and an independent four-conduction transducer unit of peripheral blood perfusion wave, wherein, due to change of erythrocytes carried by blood stream of a blood vessel, the blood stream exerts a force onto the blood vessel wall so as to form a pulse;
a near-infrared laser emitter of the sensor illuminates the blood vessel to produce a reflective wave, and the reflective wave signal is received by a laser receiver on the lateral side and used as a first output signal; meanwhile, the pulse wave of the blood vessel wall is detected by a pressure transducer at the blood vessel and used as a second output signal;
the first output signal and the second output signal are processed by a control computer chip to obtain a blood perfusion wave;
dry electrode metal pieces are used in the electroencephalogram sensor as medium to be affixed onto the forehead, so as to conduct cerebral electrical signal as a third output signal.

14. The device of claim 13, wherein,
the integrated transducer is fixed on a flexible silica gel, the size of which is within $12 \times 1.4 \times 0.4$cm and which is elastic, strip shaped, and able to be formed into an annular shape and affixed by adhesive tape of a medical band-aid; the peripheral blood perfusion transducers detect blood perfusion signal and are fixed on a flexible silica gel, the size of which is within $3 \times 1 \times 0.3$cm.

15. An operation method of the device of claim 13, wherein,
the output signal of the transducer is converted into recognizable analog electrical signal by an electrical signal conversion and amplification unit which comprises an electroencephalographic signal acquisition and amplification module and a blood vessel blood perfusion wave signal acquisition and amplification module, the tiny signal acquired by the transducer is amplified and filtered to eliminate interference and void parts, transmitted to an analog-digital convertor, discretized and converted into digital signal, and then allocated, computed, encrypted and compressed by the control computer chip;
the processed data stream is fed into a dynamic data link cache queue, the dynamic data link cache queue is a varying data storage and output configuration, and the data configuration in a storage region is determined by network condition;
after an interruption event is triggered due to that the central computation and control management circuitry is notified of the change of network condition, the central computation and control management circuitry controls various permutations and combinations of the obtained data;
the data is then written into the storage queue upon a write instruction; the data in the queue is outputted to a wireless internet access circuit through a data port upon a read instruction of the control computer chip; the internet access circuit carries out auto-dial to the network, network condition recognition, TCP mode signal modulation, sub-package and outputting.

16. A sleep analysis method, comprising the steps of
extracting physiological signal indicating change of sleep quality which includes a brain blood perfusion index and a peripheral blood perfusion index, by using nerve electrophysiological measuring method in conjunction with brain blood perfusion measuring method, so as to establish a vital sign expressing means and characteristic indicators for quantitatively expressing and indicating condition change of sleep process, sleep structure and sleep quality;
objectively describing sleep quality by using the brain blood perfusion index and the peripheral blood perfusion index as a major physiological signal basis for sleep measurement, assisted by electroencephalographic signal, so as to reveal the relative change of brain blood perfusion and peripheral blood perfusion during sleep, and indicate the quantitative physiological change process of sleep influencing disease treatment and health and longevity;

automatically extracting electroencephalographic characteristic indicators which quantitatively describe change of sleep structure and brain blood and peripheral blood perfusion distribution characteristic indicators which quantitatively reflect changes of sleep quality and health condition, by real-time computation of the acquired multi-conduction electroencephalographic wave and multi-conduction blood perfusion wave;

acquiring the multi-conduction electroencephalographic waves and multi-conduction blood perfusion waves of a human in various conditions by using transducers for acquiring the signal of electroencephalographic wave, brain blood and peripheral blood perfusion wave, and computing and processing the data at real-time through a computer system which receives the same, so as to obtain real-time data of quantitative characteristic indicators;

performing real-time data computation with data pre-processing algorithms including waveform recognition algorithm, fuzzy control algorithm, spectrum analysis algorithm, wavelet analysis algorithm, multiple regression algorithm, and calculus algorithm;

computing frequency domain power and phase change of the electroencephalographic wave signal under a specific time domain window, and computing multi-variant components of multi-scale wavelet decomposition scale-space under a specific generating function with spectrum analysis algorithm and wavelet analysis algorithm of the electroencephalographic wave data with time window weighting, obtaining converted and inverse-converted cluster of data with inverse transformation algorithm of time domain component reconstruction, and obtaining a cluster of wavelet discrete values after processing the converted and inverse-converted cluster of data with fuzzy algorithm and threshold value extraction algorithm;

obtaining a series of quantitative electroencephalographic characteristic indicators representing various sleep conditions, after multiple regression algorithm incorporating sleep condition and weighting indexation;

obtaining the waveform change-point of multiple-cluster time domain blood perfusion waveforms of the multi-conduction blood perfusion wave with multi-scale wavelet analysis algorithm of the blood perfusion wave data under a specific generating function, and then obtaining the dynamic time domain power and relative speed of the blood perfusion wave with calculus algorithm and fitting computation, so as to produce quantitative characteristic indicators describing brain blood and peripheral blood perfusion signal for various sleep quality and sleep structure during sleep.

17. A personal health information platform, comprising:

obtaining vital signs of a human body, and acquiring medical health data;

decomposing medical clinical inspection items into individual standardized quantitative measurements for life condition, and self-organizing, analyzing and computing life condition information, so as to compose various inspection reports of disease physiological and vital sign data and health prediction reports;

establishing a system for real-time quantitative multi-index data extraction for brain condition under the mode of Internet of Things, so as to establish an integrated extracting and expressing method of quantitative characteristic indicators of nerve electrophysiological and blood perfusion signal related to human natural sleep structure and quality;

defining a brain blood perfusion unit and a peripheral blood perfusion unit through measurement of blood perfusion distribution, and establishing a training routine and method to respectively reduce metabolic requirement of the two units upon the relationship between blood flow and metabolism, so as to allow the organs to be able to withstand low blood perfusion level under disease condition;

establishing a new life inspection and maintenance mode as a third life maintenance mode for disease treatment, disease rehabilitation, disease prevention and health keeping and life prolonging, so as to control the change of vital sign data upon independent consciousness, in order to achieve the goal of keeping healthy and prolonging life;

introducing continuous quantitative precise measuring and computing of vital sign data and medical health data in the processes of disease treatment, disease rehabilitation, disease prevention and health keeping and life prolonging, changing negative emotion of a person under disease condition into positive emotion, and continuously perceiving the real-time change of outputted quantitative characteristic indicators of vital signs and physiological data, so as to enhance the disease treatment effect and disease rehabilitation effect and achieve the goal of disease prevention and life prolonging, wherein, the data and technique produced during the processes of disease treatment, rehabilitation, disease prevention and health keeping and life prolonging are used as a tool, and based on wireless network communication, the system of aiding or directly driving the processes of disease treatment, rehabilitation, disease prevention and health keeping and life prolonging for people is established.

18. The personal health information platform of claim 17, further comprising
establishing a media broadcasting system for demonstration pioneers of health keeping, life prolonging, disease treatment and disease rehabilitation, broadcasting treatment track record and vital sign data of the pioneers at regular time, so as to provide a demonstration example for quantitative health data comparison, learning and imitation of the processes of disease treatment, disease rehabilitation and health keeping and life prolonging for people through real-time media transmission.

19. The personal health information platform of claim 18, wherein,
the platform comprises a system software platform and hardware devices including a wireless mobile terminal for vital sign data acquisition and transmission, a data computation service center server and a data storage center server, a mobile computer terminal or a stationary computer, and a mobile communication terminal.

20. The personal health information platform of claim 17, wherein,
the platform adopts an internet communication platform as a communication transmission media, and the users are allowed to register their own application accounts with the health bank website and bind the accounts with their personal identification information.

21. The personal health information platform of claim 17, wherein,
on the platform, a wireless mobile terminal for vital sign data acquisition and transmission is used by each user in living and working environment, each terminal is bound with the application account of the user, informations of vital signs, subjective feeling and medicine intake events of the user are automatically acquired in daily usage through the wireless mobile terminal for vital sign data acquisition and transmission and automatically transmitted to a data computation center server and an account storage center server on the internet through wireless means;
the transmitted data chain is bound with the user's personal identification information and automatically stored into a database corresponding to the application account which is established by the user for permanent storage and timely presentation;
after receipt of the data, the data computation center server performs automatic real-time computation and analysis of massive data with cloud computing, various individual life condition characteristic indicators are computed respectively upon the principle of elaborating various indicators in an inspection and analysis report required for disease diagnosis, the characteristic indicators are used to form an inspection and analysis report which meets the corresponding disease diagnosis standards of a medical institution, the report is transmitted to the account storage server and stored in the user's application account for timely presentation.

22. The personal health information platform of claim 17, further comprising the extraction process of multiple characteristic indicators of brain condition, wherein,
the original data of multiple characteristic indicators of brain condition is derived from real-time electroencephalographic wave data of the wireless mobile terminal for vital sign data acquisition and transmission, and automatically mathematically processed and computed by the computation service center to obtain multiple quantitative numeric indexes representing various brain conditions, and an effective quantitative cerebrate habit is able to be established by using objective quantitative real-time characteristic indexes of brain condition, so as to learn the method and skill of quantitatively controlling the brain condition.

23. The personal health information platform of claim 14, further comprising the extraction of characteristic indicators of blood perfusion distribution, wherein,
the original data of characteristic indicators of blood perfusion distribution is derived from real-time blood perfusion wave of the wireless mobile terminal for vital sign data acquisition and transmission, and automatically continuously mathematically processed and computed by the computation service center to obtain quantitative numeric indexes representing blood perfusion distribution at various positions, the blood perfusion is required by human body metabolism, and reducing of local metabolic requirement is able to be learned by training of reducing the blood perfusion and withstanding of the reduced blood perfusion, so as to establish a basic environment for human body organ protection and low perfusion withstanding in disease condition, rehabilitation and life prolonging.

FIG. 1

**FIG. 2**

laser emitter

adhesive part

laser receiver

temperature transducer

blood stream transducer

**FIG. 3**

```
blood stream          first-stage amplifier,
transducer            isolator, second-stage
                      amplifier

                      first-stage amplifier,
                      isolator, second-stage      0-5V
                      amplifier                    voltage
          front                                    output
          passive      first-stage amplifier,
          filter       isolator, second-stage
                       amplifier

laser receiver                                     data
                                                   serial
                                                   port
temperature           interface
transducer
```

**FIG. 4**

**FIG. 5**

**FIG. 6**

**FIG. 7**

**FIG. 8**

**FIG. 9**

**FIG. 10**

**FIG. 11**

**FIG. 12**

FIG. 13

FIG. 14

**FIG. 15**

FIG. 16

**FIG. 17**

| No. | Name (Index) | | Threshold value (numeric) | Description |
|---|---|---|---|---|
| 1 | brain information flow real-time condition quantitative (index) | i_22 concentrating index | 240 | value below is concentrating |
| 2 | | i_56 relaxing index | 35 | value below is relaxing |
| 3 | | i_48 thinking intensity | 110 | value above is thinking |
| 4 | | i_62 attentiveness | 130 | value below is gradually sense shut |
| 5 | | i_60 energyconsumption | 50 | |
| 6 | | i_46 stability | 45 | |
| 7 | brain learning ability quantitative (index) | memory processing | 10 | particular condition |
| 8 | | inner concentrating | 30 | higher is more focused |
| 9 | | outer concentrating | 10 | |
| 10 | | sense shutting | 35 | higher is better sense shutting |
| 11 | brain fatigue and sleepy quantitative (index) | fatigue | 20 | higher is more fatigue |
| 12 | | sleepy | 15 | higher is sleepier |
| 13 | | drifting in and out of sleep | 20 | |
| 14 | emotion and sub-health quantitative index | energy consumption in brain | 35 | the lower the better |
| 15 | | right brain work load | 50 | higher than 50 is heavy load for right brain |
| 16 | | sub-health | 150 | the lower the better |
| 17 | | brain activity | 310 | the higher the worse |
| 18 | | relaxing level | 30 | the higher the better |
| 19 | | brain blood perfusion | 50 | |
| 20 | | microcirculation perfusion | 50 | |
| 21 | cerebrating habit quantitative (index) | left-right brain preference | 1 | higher than 1 is left brain preference lower than 1 is right brain preference |
| 22 | | task intensity | 90 | lower than 90 is empty and quiet mentality |
| 23 | | response intensity | 10 | higher is faster response |
| 24 | calm and sleep quality (index) | calm | 95 | lower is calmer |
| 25 | | sleep quality | % | |
| 26 | sleep stage index | REM stage sleep | 20% | |
| 27 | | first stage sleep | 10% | |
| 28 | | second stage sleep | 20% | |
| 29 | | third stage sleep | 25% | |
| 30 | | fourth stage sleep | 25% | |

**FIG. 18**

EP 2 918 222 A1

**FIG. 19**

94

**FIG. 20**

**FIG. 21**

FIG. 22

**FIG. 23**

**FIG. 24**

## INTERNATIONAL SEARCH REPORT

| International application No. |
|---|
| PCT/CN2014/082449 |

**Box No. II      Observations where certain claims were found unsearchable (Continuation of item 2 of first sheet)**

This international search report has not been established in respect of certain claims under Article 17(2)(a) for the following reasons:

1. ☒    Claims Nos.:1-7
    because they relate to subject matter not required to be searched by this Authority, namely:
    [1] Claims 1-4 relate to life maintenance mode, comprise that providing treatment, recovery, forming a health trend forecast report and the like using brain inhibition therapy, and belong to a disease diagnosis method.
    [2] Claims 5-7 relate to brain inhibition therapy, implement treatment for human being using mainline and belong to a human-being or animal disease diagnosis method.
    [3] Above all, claims 1-7 belong to the subject matter not required to be searched by this Authority defined in Rule 39.1 (iv).
2. ☐    Claims Nos.:
    because they relate to parts of the international application that do not comply with the prescribed requirements to such an extent that no meaningful international search can be carried out, specifically:
3. ☐    Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

[1] Claims 8-23 comprise five groups of claims:
[2] Claims 8-12 relates to a brain sigal collection method for brain inhibition therapy;
[3] Claims 13 and 14 relates to integrated with signal transducers of brain wave and forehead blood-flow and a independent set of tip blood-flow wave transducer;

[4] Claim 15 relates to a working method of a transducer of claim 13;

[5] Claim 16 relates to a analytical method of sleep;

[6] Claims 17-23 relates to a personal health information platform;

[7] And:

[8] Indenpent claims 8, (13, 15), 16 and 17 do not share a same or corresponding technical features, and do not meet the requirement of unity of invention as defined in PCT Rule 13.1 based on comprison two of 8, (13, 15), 16, and 17 with each other.

1. ☐    As all required additional search fees were timely paid by the applicant, this international search report covers all searchable claims.

2. ☒    As all searchable claims could be searched without effort justifying additional fees, this Authority did not invite payment of additional fees.
3. ☐    As only some of the required additional search fees were timely paid by the applicant, this international search report covers only those claims for which fees were paid, specifically claims Nos.:

4. ☐    No required additional search fees were timely paid by the applicant. Consequently, this international search report is restricted to the invention first mentioned in the claims; it is covered by claims Nos.:

**Remark on protest**          ☐    The additional search fees were accompanied by the applicant's protest and, where applicable, the payment of a protest fee.

☐    The additional search fees were accompanied by the applicant's protest but the applicable protest fee was not paid within the time limit specified in the invitation.

☐    No protest accompanied the payment of additional search fees.

Form PCT/ISA /210 (continuation of first sheet (2)) (July 2009)

# INTERNATIONAL SEARCH REPORT

| International application No. |
|---|
| PCT/CN2014/082449 |

**A. CLASSIFICATION OF SUBJECT MATTER**

A61B 5/00 (2006.01) i; A61B 19/00 (2006.01) i; G06F 19/00 (2011.01) i

According to International Patent Classification (IPC) or to both national classification and IPC

**B. FIELDS SEARCHED**

Minimum documentation searched (classification system followed by classification symbols)

A61B; G06F

Documentation searched other than minimum documentation to the extent that such documents are included in the fields searched

Electronic data base consulted during the international search (name of data base and, where practicable, search terms used)

CPRSABS, CNTXT, WPI, EPODOC: head, EEG, perfusion, twig, blood, sleep, insomnia, detect+, sensor, infrared, wireless, wifi, net, laser, brain, forehead, supraorbital, blood stream, transducer, silica gel, web of things

**C. DOCUMENTS CONSIDERED TO BE RELEVANT**

| Category* | Citation of document, with indication, where appropriate, of the relevant passages | Relevant to claim No. |
|---|---|---|
| X | CN 201389013 Y (WU, Yibing et al.) 27 January 2010 (27.01.2010) description, page 3, the last paragraph to page 4, the last paragraph, claims 1-5 and figure 1 | 8-12 |
| Y | CN 201389013 Y (WU, Yibing et al.) 27 January 2010 (27.01.2010) description, page 3, the last paragraph to page 4, the last paragraph, claims 1-5 and figure 1 | 22 |
| Y | CN 203208022 U (ACSKY CO., LTD.) 25 September 2013 (25.09.2013) description, paragraphs [0014]-[0034] and figure 1 | 13, 14 |
| X | CN 101599110 A (PEKING UNIVERSITY PEOPLE'S HOSPITAL) 09 December 2009 (19.12.2009) description, page 7, the last paragraph to page 8, the second paragraph | 15 |
| X | US 2008/0208480 A1 (KURIYAMA HIROYUKI et al.) 28 August 2008 (28.08.208) description, paragraphs [0049]-[0054] and figures 1-3 | 17-21 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 September 2014 | 22 October 2014 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>　　　　MA, Nan<br><br>Telephone No. (86-10) 82245608 |

Form PCT/ISA/210 (second sheet) (July 2009)

EP 2 918 222 A1

<table>
<tr><td colspan="2">INTERNATIONAL SEARCH REPORT</td><td>International application No.<br>PCT/CN2014/082449</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| Y | US 2008/0208480 A1 (KURIYAMA HIROYUKI et al.) 28 August 2008 (28.08.208) description, paragraphs [0049]-[0054] and figures 1-3 | 22, 23 |
| Y | WO 2011/076886 A2 (DELTA, DANSK ELEKTRONIK, LYS OG AKUSTIK) 30 June 2011 (30.06.2011) description, page 19, lines 3-9 and figure 1 | 13, 14 |
| Y | CN 201767952 U (WU, Yibing) 23 March 2011 (2303.2011) description, paragraph [0014] and figure 1 | 14 |
| Y | CN 2627984 Y (WU, Yibing) 28 July 2004 (28.07.2004) description, page 2, the third paragraph and figure 1 | 23 |
| A | CN 102274022 A (ZHEJIANG UNIVERSITY) 14 December 2011 (14.12.2011) description, paragraphs [0011]-[0022] and [0035]-[0043] and figure 1 | 16 |
| A | CN 1550210 A (SANYO ELECTRIC CO.) 01 December 2004 (01.12.2004) the whole | 16 |
| A | CN 2707186 Y (AVIATION MEDICINE INSTITUTE, AIR FORCE PLA) 06 July 2005 (06.07.2005) the whole document | 17-23 |
| A | US 2006/0100530 A1 (ALLEZ PHYSIONIX LIMITED et al.) 11 May 2006 (11.05.2006) the | 8-23 |

Form PCT/ISA /210 (continuation of second sheet ) (July 2009)

## INTERNATIONAL SEARCH REPORT
### Information on patent family members

| International application No. |
|---|
| PCT/CN2014/082449 |

| Patent Documents referred in the Report | Publication Date | Patent Family | Publication Date |
|---|---|---|---|
| CN 201389013 Y | 27 January 2010 | None | |
| CN 203208022 U | 25 September 2013 | None | |
| CN 101599110 A | 09 December 2009 | None | |
| US 2008/0208480 A1 | 28 August 2008 | JP 5090013 B2 | |
| | | JP 2008206575 A | |
| | | CN 101248984 B | |
| | | CN 101248984 A | |
| WO 2011/076886 A2 | 30 June 2011 | EP 2515744 A2 | |
| | | CA 2785354 A1 | |
| | | JP 2013515528 A | |
| | | CN 102811657 A | |
| | | AU 2010334812 A1 | |
| | | US 2013060098 A1 | |
| CN 201767952 U | 23 March 2011 | None | |
| CN 2627984 Y | 28 July 2004 | None | |
| CN 102274022 A | 14 December 2011 | CN 102274022 B | |
| CN 1550210 A | 01 December 2004 | US 2004230398 A1 | |
| | | JP 4059867 B | |
| | | JP 2004358235 A | |
| CN 2707186 Y | 06 July 2005 | None | |
| US 2006/0100530 A1 | 11 May 2006 | CN 101316549 A | |
| | | JP 2009509630 A | |
| | | WO 2007040645 A1 | |
| | | CA 2622544 A1 | |
| | | EP 1937141 A1 | |
| | | CN 101316549 B | |

Form PCT/ISA /210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102783936 A